(19) Europäisches Patentamt
European Patent Office
Office européen des brevets

(11) EP 1 149 092 B1

# (12) EUROPEAN PATENT SPECIFICATION

(45) Date of publication and mention of the grant of the patent:
**29.10.2003 Bulletin 2003/44**

(21) Application number: **99957341.3**

(22) Date of filing: **07.12.1999**

(51) Int Cl.$^7$: **C07D 403/00**

(86) International application number:
**PCT/EP99/09578**

(87) International publication number:
**WO 00/035906 (22.06.2000 Gazette 2000/25)**

## (54) 4- AND 5-ALKYNYLOXINDOLES AND 4- AND 5-ALKENYLOXINDOLES

4- UND 5-ALKYNYLOXINDOLE SOWIE 4- UND 5-ALKENYLOXINDOLE

4- ET 5-ALKYNYLOXINDOLES ET 4- ET 5-ALKENYLOXINDOLES

(84) Designated Contracting States:
**AT BE CH CY DE DK ES FI FR GB GR IE IT LI LU MC NL PT SE**

(30) Priority: **17.12.1998 US 112589 P**
**29.06.1999 US 141482 P**

(43) Date of publication of application:
**31.10.2001 Bulletin 2001/44**

(73) Proprietor: **F. HOFFMANN-LA ROCHE AG**
**4070 Basel (CH)**

(72) Inventors:
- **LUK, Kin-Chun**
  **North Caldwell, NJ 07006-4622 (US)**
- **MAHANEY, Paige, E.**
  **Scotch Plains, NJ 07076 (US)**
- **MISCHKE, Steven Gregory**
  **Florham Park NJ 07932 (US)**

(74) Representative: **Wächter, Dieter Ernst, Dr. et al**
**F.Hoffmann-La Roche AG**
**Patent Department (PLP),**
**124 Grenzacherstrasse**
**4070 Basel (CH)**

(56) References cited:
**WO-A-96/22976**
**WO-A-96/32380**
**WO-A-96/40116**
**WO-A-97/45409**
**WO-A-98/50356**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 149 092 B1

## Description

The present invention is directed to novel 4- and 5-alkynyloxindoles as well as 4- and 5-alkenyloxindoles which inhibit or modulate protein kinases, in particular JNK protein kinases. These compounds and their pharmaceutically acceptable salts, and prodrugs of said compounds, are useful as anti-inflammatory agents, particularly useful in the treatment of rheumatoid arthritis. The invention is also directed to pharmaceutical compositions containing such compounds, and to methods for the treatment and/or control of inflammation, particularly in the treatment or control of rheumatoid arthritis. This invention is further directed to intermediates useful in the preparation of the foregoing compounds.

Protein kinases are a class of proteins that regulate a variety of cellular functions. This is accomplished by the phosphorylation of specific amino acids on protein substrates resulting in conformational alteration of the substrate protein. The conformational change modulates the activity of the substrate or its ability to interact with other binding partners. The enzyme activity of the protein kinase refers to the rate at which the kinase adds phosphate groups to a substrate. It can be measured, for example, by determining the amount of a substrate that is converted to a product as a function of time. Phosphorylation of a substrate occurs at the active-site of a protein kinase.

The JNK (Jun N-terminal kinase) protein kinases (also know as "stress-activated protein kinases" or "SAPK") are members of the mitogen-activated protein (MAP) kinases. *See, e.g.,* S. Gupta et al., EMBO J., vol. 15 no. 11 (1996) pp. 2760-2770; and Yang et al., Nature, vol. 289 (23 October 1997) pp. 865-870. At least ten JNK isoforms are currently known. *See*, Gupta, *id*. As its name indicates, one of the substrates for JNK is c-Jun. JNK phosphorylates the $NH_2$-terminal activation domain of c-Jun on Ser63 and Ser73, causing increased c-Jun transcriptional activity. *See* Gupta, *id*. In turn, c-Jun is an AP-1 transcription factor that mediates immediate-early gene expression. *See, e.g.,* A. Minden et al., Biochimica et Biophysica Acta 1333 (1997) F85-F104; and P. Agel et. al., Biochimica et Biophysica Acta, vol. 1072 (1991) pp.129-157.

The JNK protein kinase is markedly activated in response to treatment of cells with pro-inflammatory cytokines or exposure to environmental stress. JNK thus mediates the effect of extracellular stimuli on c-Jun. *See* Gupta, *supra;* and Minden, *supra*. Accordingly, JNK is a physiological regulator of AP-1 transcriptional activity. Thus, inhibition of JNK activity will inhibit AP-1-dependent transcription of inflammatory and immune mediators which are implicated in pathological proliferative conditions, for example inflammatory diseases and neuro-degenerative diseases, in particular, rheumatoid arthritis. *See, eg.* Swantek et al., Molecular and Cellular Biology, vol. 17 (1997) pp. 6274-6282; Maroney et al., J. Neuroscience, vol. 18 (1 Jan. 1998) pp. 104-111 ; and Minden, *supra*, at F92.

The rat homologue of JNK is also called SAPK (stress-activated protein kinase). SAPK isoforms share significant (>90%) sequence identity with the corresponding JNK isoforms [*compare* Kyriakis et al., Nature, Vol 369 (12 May 1994) pp. 156-160 and Gupta et al., *supra*]. Both JNK and SAPK are capable of phosphorylation of the c-Jun substrate and thus have very similar enzyme activity. JNK and SAPK are part of a protein kinase cascade that is activated by various extracellular stimuli. *See e.g.* Minden *supra*; and Kyriakis et al., BioEssays Vol 18 (1996) pp. 567-577. JNK and SAPK each can be activated by phosphorylation on specific threonine and tyrosine residues by dual specificity MAP kinase kinases such as MKK4, SEK-1, or MKK7. *See* Kyriakis et al., *supra;* and Tournier et al., Proceedings of the National Academy of Sciences USA Vol. 94 (July 1997), pp. 7337-7342. The dual specificity MAP kinase kinases can be activated by phosphorylation on serine and/or threonine residues by MAP kinase kinase kinases such as MEKK-1. Thus, measurement of JNK or SAPK enzyme activity may be enhanced by activation by the upstream or preceding kinases. Moreover, measurement of SAPK inhibition is closely correlated with JNK inhibition.

Inhibitors of protein kinase catalytic activity are known in the art. *See* WO 98/24432 (indoline compounds that inhibit *FLK* protein kinase); WO 97/45409 (substituted tetralylmethylene-oxindole analogues that inhibit tyrosine kinase). In particular, small molecule inhibitors typically block the binding of substrates by tightly interacting with the protein kinase ATP binding site (or "active site"). *See* WO 98/24432. It is desirable to identify small-molecule compounds that may be readily synthesized and are effective in inhibiting the catalytic activity of protein kinases, in particular of the JNK protein kinases.

Indolinone (also known as oxindole) compounds asserted to be useful in regulating abnormal cell proliferation through tyrosine kinase inhibition are disclosed for example in WO 96/40116, WO 98/07695, WO 95/01349, WO 96/32380, WO 96/22976, WO 96/16964 and WO 98/50356 (2-indolinone derivatives as modulators of protein kinase activity); Mohammadi et. al, Science, Vol. 276, 9 May 1997, pp. 955-960. Oxindole derivatives have also been described for various other therapeutic uses: 5,206,261 (improvement of cerebral function); WO 92/07830 (peptide antagonists); EP 580 502 A1 (antioxidants).

There continues to be a need for easily synthesized, small molecule compounds effective in inhibiting JNK protein kinase and thus useful in the treatment or control of pathological proliferative conditions, for example inflammatory diseases and neuro-degenerative diseases, in particular, rheumatoid arthritis. It is thus an object of this invention to provide such compounds and compositions containing such compounds.

In one embodiment, the present invention is directed to 4-alkynyloxindoles and 4-alkenyloxindoles of the

formula

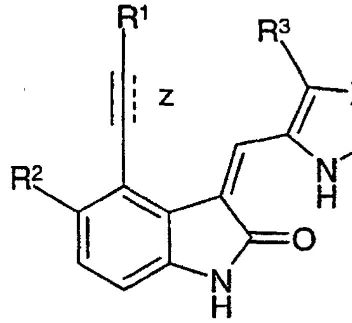

I

and the pharmaceutically acceptable salts thereof,
wherein:

$R^1$ is lower alkyl that is substituted by aryl, aryloxy, heteroaryl, heteroaryloxy, substituted aryl, substituted aryloxy, substituted heteroaryl, and/or substituted heteroaryloxy, and optionally also may be substituted by $R^{13}$, perfluoroalkyl, cycloalkyl (or cycloalkyl substituted by lower alkyl and/or $R^{13}$), or heterocycle (or heterocycle substituted by lower alkyl and/or $R^{13}$),
and wherein the substitutents on the substituted aryl, substituted aryloxy, substituted heteroaryl, and substituted heteroaryloxy are one or more of

$R^{13}$, lower alkyl (optionally substituted by $R^{13}$), cycloalkyl (optionally substituted by $R^{13}$), heterocycle (optionally substituted by $R^{13}$); aryl (optionally substituted by $R^{13}$, perfluoroalkyl, lower alkyl, lower alkyl substituted by $R^{13}$, cycloalkyl, cycloalkyl substituted by $R^{13}$, heterocycle (optionally substituted by $R^{13}$); or heteroaryl (optionally substituted by $R^{13}$, perfluoroalkyl, lower alkyl, lower alkyl substituted by $R^{13}$, cycloalkyl, cycloalkyl substituted by $R^{13}$, or heterocycle or heterocycle substituted by $R^{13}$); or
aryl (optionally substituted by halogen, $-OR^4$, $-COR^4$, $-COOR^4$, $-CONR^6R^7$, $C_{1-6}$ alkyl, $C_{3-8}$ cycloalkyl, heterocycle, $C_{1-6}$ alkyl which is substituted by $-OR^4$ , $-NR^6R^7$, $COOR^4$, $CONR^6R^7$, $C_{3-8}$ cycloalkyl which is substituted by $-OR^4$, $COOR^4$, $CONR^6R^7$, and/or $-NR^6R^7$, and heterocycle which is substituted by $-OR^4$, $COOR^4$, $CONR^6R^7$, and/or $-NR^6R^7$), or
heteroaryl (optionally substituted by halogen, $-OR^4$, $-COR^4$, $-COOR^4$, $-CONR^6R^7$, $C_{1-6}$ alkyl, $C_{3-8}$ cycloalkyl, heterocycle, $C_{1-6}$ alkyl which is substituted by $-OR^4$, $COOR^4$, $CONR^6R^7$, and/or $-NR^6R^7$, $C_{3-8}$ cycloalkyl which is substituted by $-OR^4$, $COOR^4$, $CONR^6R^7$, and/or $-NR^6R^7$, and/or heterocycle which is substituted by $-OR^4$, $COOR^4$, $CONR^6R^7$, and/or $-NR^6R^7$);

$R^2$ is hydrogen, $-OR^4$, $-OCOR^4$, $-COR^4$, $-COOR^4$, $-CONR^6R^7$, $-NR^6R^7$, halogen, $-NO_2$, -CN, $-SO_2R^4$, $-SO_2NR^6R^7$, perfluoroalkyl, lower alkyl or lower alkyl substituted by $-OR^8$ or $-NR^6R^7$;

$R^3$ is hydrogen, $-OR^4$, $-COR^4$, $-COOR^4$, $-CONR^6R^7$, halogen, -CN, $-NR^6R^7$, perfluoroalkyl, lower alkyl or lower alkyl substituted by $-OR^8$ or $-NR^6R^7$;

$R^4$ is hydrogen, lower alkyl (optionally substituted by (a), cycloalkyl and /or heterocycle), cycloalkyl (optionally substituted by (a), lower alkyl and/or heterocycle), heterocycle (optionally substituted by (a), lower alkyl and/or cycloalkyl), aryl (optionally substituted by (a), cycloalkyl, heterocycle and/or halogen), heteroaryl (optionally substituted by (a), cycloalkyl, heterocycle, and/or halogen,
where (a) is $-OR^5$, $-COOR^8$, $-COR^8$, $-CONR^8R^9$, $-NR^6R^7$, -CN, $-NO_2$, $-SO_2R^8$, and/or $-SO_2NR^8R^9$;

$R^5$ is hydrogen, $-COR^8$, $-CONR^8R^9$ or lower alkyl (optionally substituted by $OR^9$, $-NR^9R^{10}$, $-N(COR^9)R^{10}$, $-COR^9$, $-CONR^9R^{10}$, $-SR^9$ and/or $-COOR^9$;

$R^6$ and $R^7$ are each hydrogen, $-COR^8$, $-COOR^8$, $-CONR^8R^9$, $-SO_2R^8$ $SO_2NR^8R^9$, lower alkyl, lower alkyl substituted by (b), cycloalkyl (optionally substituted by (b), lower alkyl, and/or heterocycle), heterocycle, heterocycle substituted by (b), lower alkyl and/or cycloalkyl), aryl, aryl substituted by (b), lower alkyl, cycloalkyl and/or heterocycle), heteroaryl, heteroaryl substituted by (b), lower alkyl, cycloalkyl and/or heterocycle);

or $R^6$ and $R^7$ are each cycloalkyl (optionally substituted by (b), lower alkyl and/or heterocycle; heterocycle (optionally substituted by (b), lower alkyl and/or cycloalkyl; aryl (optionally substituted by (b), lower alkyl, cycloalkyl and/or heterocycle; or heteroaryl (optionally substituted by (b), lower alkyl, cycloalkyl and/or heterocycle;
where (b) is $OR^5$, $-NR^8R^9$, $-COOR^8$, $-COR^8$, $-CONR^8R^9$, -CN, $-NO_2$, $-SO_2R^8$, $-SO_2NR^8R^9$;
alternatively, $-NR^6R^7$ can form a ring having 3 to 7 atoms, said ring optionally including one or more additional hetero atoms and being optionally substituted by one or more of lower alkyl, $-OR^5$, $-COR^8$, $-COOR^8$, $-CONR^8R^9$, and $-NR^5R^9$;

$R^8$ is hydrogen, lower alkyl (optionally substituted by cycloalkyl, heterocycle, aryl, heteroaryl, $-OR^9$, $-NR^9R^{10}$, and/or $-N(COR^9)R^{10}$),
aryl (optionally substituted by (c), lower alkyl, cycloalkyl and/or heterocycle), heteroaryl (optionally substituted by (c), lower alkyl, cycloalkyl and/or heterocycle), cycloalkyl (optionally substituted by (c), lower alkyl and/or heterocycle), heterocycle (optionally substituted by (c), lower alkyl and/or cycloalkyl);
where (c) is $-OR^9$, $-COOR^9$, $-COR^9$, $-CONR^{10}R^9$, $-NR^{10}R^9$
-CN, $-NO_2$, $-SO_2R^9$, $-SO_2NR^{10}R^9$;

$R^9$ and $R^{10}$ are each independently hydrogen or lower alkyl;

$R^{13}$ is halogen, $-OR^4$, $-OCOR^4$, $-COR^4$ $-COOR^4$, $-CONR^6R^7$, $-NO_2$, $-NR^6R^7$, -CN, $-SO_2R^4$, or $-SO_2NR^6R^7$;

X is =N- or -CH-; and
the dotted bond represented by z is optional;

wherein
"aryl" refers to an aromatic group having 5 to 10 atoms and consisting of 1 or 2 rings;
"aryloxy" refers to an aryl radical that includes at least one oxygen and which is attached to rest of molecule via the oxygen atom;
"heteroaryl" refers to an aromatic group having 5 to 10 atoms, one or 2 rings, and containing one or more hetero atoms;
"heteroaryloxy" refers to a heteroaryl radical that includes at least one oxygen and which is attached to rest of molecule via the oxygen atom;
"hetero atom" refers to an atom selected from N, O and S;
"heterocycle" refers to a 3- to 10-membered non-aromatic, partially or completely saturated hydrocarbon group which contains one or two rings and at least one hetero atom.

In another embodiment, the invention is directed to 5-alkynyloxindoles and 5-alkenyloxindoles having the formula:

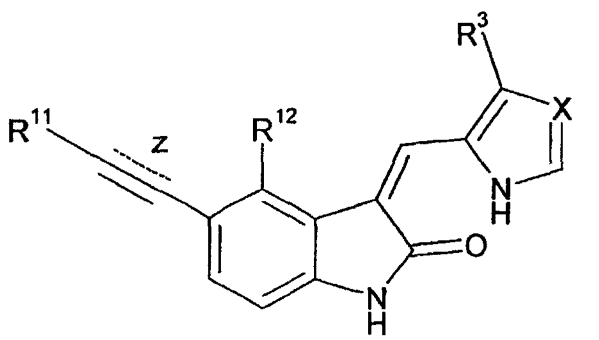

II

and the pharmaceutically acceptable salts thereof,
wherein:

$R^{11}$ is hydrogen, $-COR^4$, $-COOR^4$, $-CONR^6R^7$,
lower alkyl (optionally substituted by $-OR^5$, $-NR^6R^7$, halogen, $-NO_2$, $-SO_2R^4$, $-SO_2NR^6R^7$, -CN, $-COR^4$, $-COOR^4$, $-CONR^6R^7$, cycloalkyl, heterocycle, aryl, and/or heteroaryl),
cycloalkyl (optionally substituted by $-OR^5$, $-NR^6R^7$, halogen, $-NO_2$, $-SO_2R^4$, $-SO_2NR^6R^7$, -CN, $-COR^4$, $-COOR^4$, $-CONR^6R^7$, lower alkyl, heterocycle, aryl, and/or heteroaryl)

heterocycle (optionally substituted by $-OR^5$, $-NR^6R^7$, halogen, $-NO_2$, $-SO_2R^4$, $-SO_2NR^6R^7$, -CN, $-COR^4$, $-COOR^4$, $-CONR^6R^7$, lower alkyl, cycloalkyl, aryl, and/or heteroaryl),

aryl (optionally substituted by $-OR^5$, $-NR^6R^7$, halogen, $-NO_2$, $-SO_2R^4$, $-SO_2NR^6R^7$, -CN, $-COR^4$, $-COOR^4$, $-CONR^6R^7$, lower alkyl, and/or perfluoroalkyl) or

heteroaryl (optionally substituted by $-OR^5$, $-NR^6R^7$, halogen, $-NO_2$, $-SO_2R^4$, $-SO_2NR^6R^7$, -CN, $-COR^4$, $-COOR^4$, $-CONR^6R^7$, lower alkyl, and/or perfluoroalkyl);

$R^{12}$ is hydrogen, $-OR^4$, $-OCOR^4$, $-COR^4$, $-COOR^4$, $-CONR^6R^7$, $-NR^6R^7$, halogen, $-NO_2$, -CN, $-SO_2R^4$, $-SO_2NR^6R7$, perfluoroalkyl,

lower alkyl (optionally substituted by $OR^4$,$-NR^6R^7$, cycloalkyl, heterocycle, $-COR^4$, $-COOR^4$, $-CONR^6R^7$, -CN, $-NO_2$, $-SO_2R^4$, $-SO_2NR^6R^7$ and/or halogen),

cycloalkyl (optionally substituted by $-OR^4$, $-NR^6R^7$, lower alkyl, heterocycle, $-COR^4$, $-COOR^4$, $-CONR^6R^7$, -CN, $-NO_2$, $-SO_2R^4$, $-SO_2NR^6R^7$ and/or halogen), or

heterocycle (optionally substituted by $-OR^4$, $-NR^6R^7$, lower alkyl, cycloalkyl, $-COR^4$, $-COOR^4$, $-CONR^6R^7$, -CN, $-NO_2$, $-SO_2R^4$, $-SO_2NR^6R^7$ and/or halogen), and

$R^3$ through $R^7$, X and z are as defined for formula I above.

The present invention is further directed to pharmaceutical compositions comprising a pharmaceutically effective amount of any one or more of the above-described compounds and a pharmaceutically acceptable carrier or excipient.

The present invention is also directed to the use of a compound of claim 1 or 13 or prodrugs and pharmaceutically active metabolites of such compound in the preparation of a medicament for the treatment or control of inflammatory diseases, particularly rheumatoid arthritis.

The present invention is also directed to intermediates useful in the preparation of the above-described 4- and 5-alkynyloxindoles and 4- and 5-alkenyloxindoles.

As used herein, the following terms shall have the following definitions.

"Aryl" means an aromatic group having 5 to 10 atoms and consisting of 1 or 2 rings.

"Aryloxy" means an aryl radical that includes at least one oxygen and which is attached to rest of molecule via the oxygen atom.

"Cycloalkyl" means a non-aromatic, partially or completely saturated cyclic aliphatic hydrocarbon group containing 3 to 8 atoms. Examples of cycloalkyl groups include cyclopropyl, cyclopentyl and cyclohexyl.

"Effective Amount" means an amount of at least one compound of formula I and/or II, or a pharmaceutically acceptable salt, prodrug or metabolite thereof, that inhibits the development or proliferation of (1) an inflammatory disease or response and/or (2) a neuro-degenerative disease or response, such as for example, and not as a limitation, rheumatoid arthritis.

"Halogen" means fluorine, chlorine, bromine or iodine.

"Heteroaryl" groups are aromatic groups having 5 to 10 atoms, one or 2 rings, and containing one or more hetero atoms. Examples of heteroaryl groups are 2-, 3- or 4-pyridyl, tetrazolyl, oxadiazolyl, pyrazinyl and quinolyl.

"Heteroaryloxy" means a heteroaryl radical that includes at least one oxygen and which is attached to rest of molecule via the oxygen atom.

"Hetero atom" means an atom selected from N, O and S.

"Heterocycle" means a 3- to 10-membered non-aromatic, partially or completely saturated hydrocarbon group, such as tetrahydroquinolyl, which contains one or two rings and at least one hetero atom.

"$IC_{50}$" refers to the concentration of a particular 4- or 5-alkynyloxindole or 4-or 5-alkenyloxindole required to inhibit 50% of cJun phosphorylation, which is a measure of inhibition of SAPK activity. $IC_{50}$ can be measured, *inter alia,* using the assay described herein in Example 102.

"Lower Alkyl" denotes a straight-chain or branched saturated aliphatic hydrocarbon having 1 to 6, preferably 1 to 4, carbon atoms. Typical lower alkyl groups include methyl, ethyl, propyl, isopropyl, butyl, t-butyl, 2-butyl, pentyl, hexyl and the like.

"Pharmaceutically acceptable salt" refers to conventional acid-addition salts or base-addition salts which retain the biological effectiveness and properties of the compounds of formula I or II and are formed from suitable nontoxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Sample base-addition salts include those derived from sodium, potassium, ammonium, and quaternary ammonium hydroxide, such as for example

tetramethylammonium hydroxide.

"Pharmaceutically acceptable," such as pharmaceutically acceptable carrier, excipient, prodrug, etc., means pharmacologically acceptable and substantially non-toxic to the subject to which the particular compound is administered.

"Pharmaceutically active metabolite" means a metabolic product of a compound of formula I or II which is pharmaceutically acceptable and effective.

"Prodrug" refers to a compound that may be converted under physiological conditions or by solvolysis to any of the compounds of formula I or II or to a pharmaceutically acceptable salt of a compound of formula I or II. A prodrug may be inactive when administered to a subject but is converted *in vivo* to an active compound of formula I or II.

"Substituted," as in substituted alkyl means that the substitution can occur at one or more positions, that one or more substituents may be selected, and, unless otherwise indicated, that the substituents are independently selected from the specified options.

In one embodiment, the invention is concerned with compounds of formulae I and It, wherein $R^4$ is hydrogen, lower alkyl (optionally substituted by (a), cycloalkyl and /or heterocycle), cycloalkyl (optionally substituted by (a), lower alkyl and/or heterocycle), or heterocycle (optionally substituted by (a), lower alkyl and/or cycloalkyl), where (a) is $-OR^5$, $-COOR^8$, $-COR^8$, $-CONR^8R^9$, $-NR^6R^7$, -CN, $-NO_2$, $-SO_2R^8$, and/or $-SO_2NR^8R^9$; and $R^5$ is hydrogen, $-COR^8$, $-CONR^8R^9$ or lower alkyl (optionally substituted by $-OR^9$, $-NR^9R^{10}$, $-N(COR^9)R^{10}$, $-COR^9$, $-CONR^9R^{10}$ and/or $-COOR^9$ ; and $R^1$ through $R^{10}$, X and z are as above.

In a preferred embodiment, the invention is directed to compounds of the formula I wherein $R^1$ is

lower alkyl that is substituted by aryl or substituted aryl, and optionally also substituted by halogen, $-OR^4$, $-COR^4$, $-COOR^4$, $-CONR^6R^7$, cycloalkyl, heterocycle, $-COOR^4$, $CONR^6R^7$, cycloalkyl which is substituted by $OR^4$, $-NR^6R^7$, $COOR^4$, $CONR^6R^7$, and/or heterocycle which is substituted by $OR^4$ and $-NR^6R^7$, $COOR^4$, $CONR^6R^7$; and wherein the substituents on the substituted aryl are selected from halogen, $-OR^4$, $-COR^4$, $-COOR^4$, $-CONR^6R^7$, $-NO_2$, $NR^6R^7$, $-SO_2R^4$, $-SO_2NR^6R^7$, -CN, perfluoroalkyl, lower alkyl, cycloalkyl, heterocycle, lower alkyl which is substituted by $-OR^4$ and $-NR^6R^7$, $COOR^4$, $CONR^6R^7$, cycloalkyl which is substituted by $OR^4$ and $-NR^6R^7$, $COOR^4$, $CONR^6R^7$, or heterocycle which is substituted by $OR^4$ and $-NR^6R^7$, $COOR^4$, $CONR^6R^7$;

lower alkyl that is substituted by heteroaryl or substituted heteroaryl, and optionally also substituted by halogen, $-OR^4$, $-COR^4$, $-COOR^4$, $-CONR^6R^7$, cycloalkyl, heterocycle, cycloalkyl which is substituted by $OR^4$, $COOR^4$, $CONR^6R^7$, and/or $-NR^6R^7$, and/or heterocycle which is substituted by $-OR^4$, $COOR^4$, $CONR^6R^7$, and/or $-NR^6R^7$; and wherein the substituents on the substituted heteroaryl are selected from halogen, $-OR^4$, $-COR^4$, $-COOR^4$, $NR^6R^7$, $-SO_2R^4$, $-SO_2NR^6R^7$, $-NO_2$, -CN, $-CONR^6R^7$, lower alkyl, cycloalkyl, heterocycle, lower alkyl which is substituted by $-OR^4$ $-NR^6R^7$, $COOR^4$, $CONR^6R^7$, cycloalkyl which is substituted by $-OR^4$ ,

$-NR^6R^7$, $COOR^4$, $CONR^6R^7$, and/or heterocycle which is substituted by $-OR^4$, $-NR^6R^7$, $COOR^4$ and/or $CONR^6R^7$),

aryl (optionally substituted by halogen, $-OR^4$, $-COR^4$, $-COOR^4$, $-CONR^6R^7$, lower alkyl, cycloalkyl, heterocycle, lower alkyl which is substituted by $-OR^4$ , $-NR^6R^7$, $COOR^4$, $CONR^6R^7$, cycloalkyl which is substituted by $-OR^4$, $COOR^4$, $CONR^6R^7$, and/or $-NR^6R^7$, and heterocycle which is substituted by $-OR^4$, $COOR^4$, $CONR^6R^7$, and/or $-NR^6R^7$), or

heteroaryl (optionally substituted by halogen, $-OR^4$, $-COR^4$, $-COOR^4$, $-CONR^6R^7$, lower alkyl, cycloalkyl, heterocycle, lower alkyl which is substituted by $-OR^4$, $COOR^4$, $CONR^6R^7$, and/or $-NR^6R^7$, cycloalkyl which is substituted by $-OR^4$, $COOR^4$, $CONR^6R^7$, and/or $-NR^6R^7$, and/or heterocycle which is substituted by $-OR^4$, $COOR^4$, $CONR^6R^7$, and/or $-NR^6R^7$).

In another preferred embodiment, the invention is concerned with compounds of formulae I and II, wherein

X is CH and $R^3$ is lower alkoxy; further, wherein

$R^1$ is lower alkyl substituted by phenyl which is substituted by one to three substituents from the group hydroxy, lower alkoxy, di-(lower alkyl)-amino, di-(lower alkyl)amino-lower alkoxy, morpholino-lower alkyl, carboxy-lower alkoxy and lower alkanoylamino; or $R^1$ is lower alkyl substituted as before and additionally by hydroxy; or wherein $R^1$ is lower alkyl substituted by pyridyl, pyrrolyl, N-lower alkyl-pyyrolyl, thienyl, lower-alkoxy substitituted thienyl, furyl, 1,3-benzodioxolyl, or lower-alkoxy substituted 1,3-benzodioxolyl; or wherein

$R^1$ is lower alkyl substituted as before and additionally by hydroxy; or wherein $R^1$ is pyridyl.

Also preferred are compounds of formula I wherein the optional bond z is present.

Preferred compounds of formula II are those $R^3$ is hydrogen, $-OR^4$, $-NR^6R^7$, and/or lower alkyl (optionally substituted by $-OR^8$ and/or $-NR^6R^7$);

$R^4$ is hydrogen, lower alkyl (optionally substituted by one or more $-OR^5$, $-COOR^8$, $-COR^8$, $-CONR^8R^9$), cycloalkyl (optionally substituted by one or more $-OR^5$, $-COOR^8$, $-COR^8$ and $-CONR^8R^9$), or heterocycle (optionally substituted by one or more $-OR^5$, $-COOR^8$, $-COR^8$ and $-CONR^8R^9$);

$R^5$ is hydrogen, $-COR^8$, $-CONR^8R^9$, or lower alkyl;

$R^6$ and $R^7$ are each independently hydrogen, $-COR^8$, $-COOR^8$, $-CONR^8R^9$, or lower alkyl (optionally substituted by one or more of $-OR^9$ , $-NR^8R^9$, $COOR^8$, and $CONR^8R^9$), or alternatively, $-NR^6R^7$ optionally form a ring having 3 to 7 atoms, said ring optionally including one or more additional hetero atoms and being optionally substituted by one or more of lower alkyl, $-OR^5$, $-COR^8$, $-COOR^8$, $-CONR^8R^9$, and $-NR^5R^9$;

$R^8$ is hydrogen or lower alkyl (optionally substituted by one or more of aryl, heteroaryl, $-OR^9$, $COOR^9$, $CONR^9R^{10}$, and $-NR^9R^{10}$);

$R^{11}$ is aryl (optionally substituted by $-OR^5$ and/or $-NR^6R^7$);

$R^{12}$ is hydrogen, $-COR^4$, $-COOR^4$, $-CONR^6R^7$,
lower alkyl (optionally substituted by one or more of $-OR^4$ ,$-NR^6R^7$, cycloalkyl, heterocycle, $-COR^4$, $-COOR^4$, $-CONR^6R^7$, -CN, $-NO_2$, $-SO_2R^4$, $-SO_2$ $NR^6R^7$ and halogen),
cycloalkyl (optionally substituted by one or more of $-OR^4$, $-NR^6R^7$, lower alkyl, heterocycle, $-COR^4$, $-COOR^4$, $-CONR^6R^7$, -CN, $-NO_2$, $-SO_2R^4$, $-SO_2$ $NR^6R^7$ and halogen), or
heterocycle (optionally substituted by one or more of $-OR^4$, $-NR^6R^7$, lower alkyl, cycloalkyl, $-COR^4$, $-COOR^4$, $-CONR^6R^7$, -CN, $-NO_2$, $-SO_2R^4$, $-SO_2$ $NR^6R^7$ and halogen);

and the optional bond z is present.

The invention further relates to novel intermediates useful in the preparation of compounds of formula I:

(Z)-1,3-Dihydro-3-[(1H-pyrrol-2-yl)methylene]-5-(trimethylsilyl)ethynyl-2H-indol-2-one,
(Z)-5-Bromo-1,3-dihydro-3-[(3-methoxy-1H-pyrrol-2-yl)methylene]-2H-indol-2-one,
(Z)-1,3-Dihydro-3-[(3-methoxy-1H-pyrrol-2-yl)methylene]-5-(trimethylsilyl)ethynyl-2H-indol-2-one,
(Z)-5-Bromo-1,3-dihydro-3-[(4-methyl-1H-imidazol-5-yl)methylene]-2H-indol-2-one,
(Z)-1,3-Dihydro-3-[(4-methyl-1H-imidazol-5-yl)methylene]-5-(trimethylsilyl)ethynyl-2H-indol-2-one.

The compounds disclosed herein and covered by the above formulae may exhibit tautomerism. It is intended that the invention encompasses any tautomeric form of these compounds, or mixtures of such forms, and is not limited to any one tautomeric form utilized within the formulae drawn above.

## General Synthesis Schemes

The compounds of formulas I and II may be prepared by processes known in the art. Suitable processes for synthesizing these compounds are provided in the examples. Generally, these compounds may be prepared according to the following synthesis schemes.

Compounds of Formula I: Scheme I

General Step 1

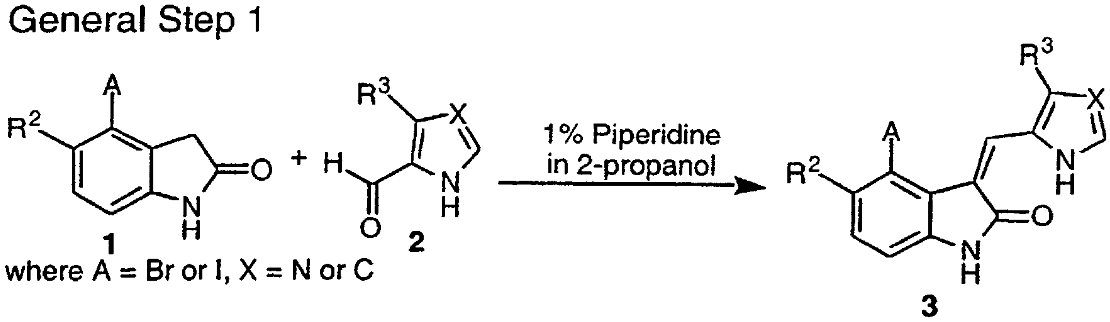


where A = Br or I, X = N or C

General Step 2a

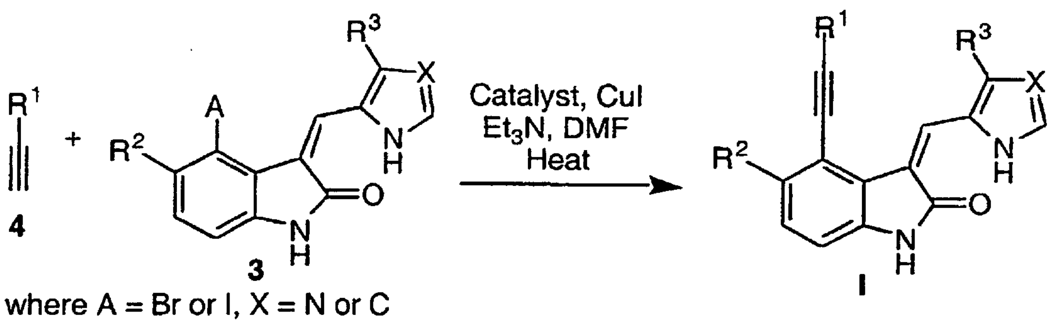


where A = Br or I, X = N or C

General Step 2b

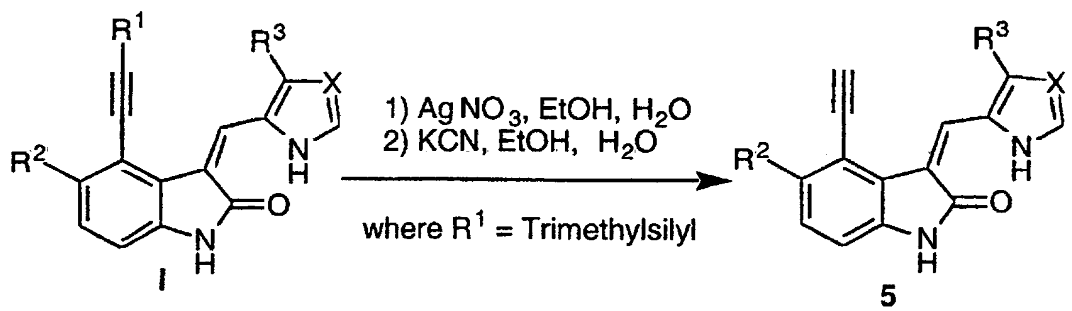


General Step 2C

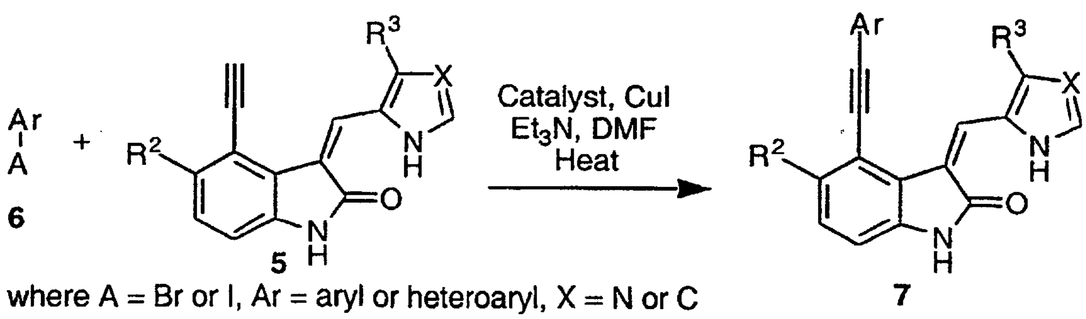


where A = Br or I, Ar = aryl or heteroaryl, X = N or C

Compounds 1 and 2 are either available from commercial sources or are synthesized by methods known in the art. Compounds 1 and 2 are reacted in piperidine to yield compound 3. When $R^1$ of the compound to be synthesized is other than Ar, compound 3 is then reacted with compound 4, which is also either available from commercial sources or is synthesized by methods known in the art, to yield compound I. *See,* General Step 2a. When $R^1$ of the product to

be synthesized is Ar, then compound I wherein $R^1$ is trimethylsilyl is further reacted with $AgNO_3$ and KCN in accordance with General Step 2b to yield compound 5. In accordance with General Step 2c, compound 5 is then reacted with compound 6, which is either available from commercial sources or is synthesized by methods known in the art, to yield compound 7.

Compounds of Formula II: Scheme II

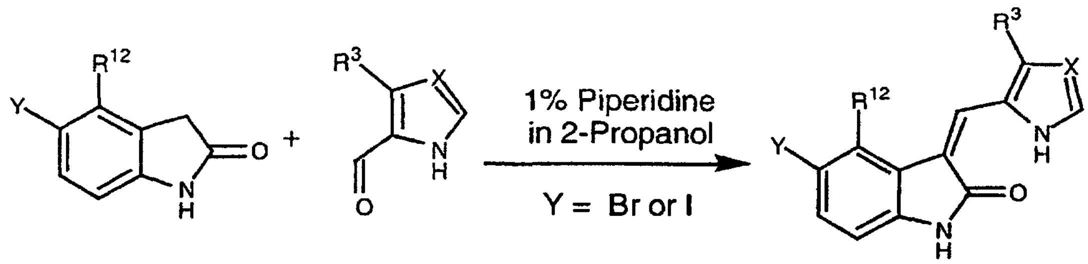


**8** **9** **10**

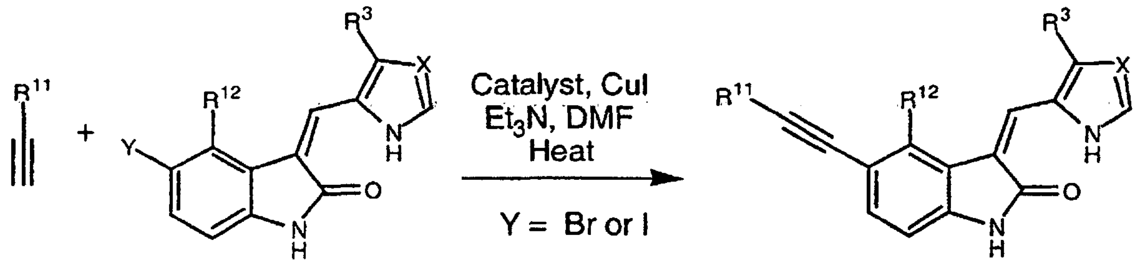


**11** **10** **II**

Compounds of formula 8 and 9 are available from commercial sources. These compounds are reacted in piperidine in an appropriate solvent to yield a compound of formula 10. Compounds of formula 10 are then reacted with a compound of formula 11, which is also commercially available, to yield a compound of formula II.

In an alternative embodiment, the present invention is directed to pharmaceutical compositions comprising at least one compound of formula I or II or a prodrug thereof, or a pharmaceutically acceptable salt of a compound of formula I or II or a prodrug of such compound.

These pharmaceutical compositions can be administered orally, for example, in the form of tablets, coated tablets, dragees, hard or soft gelatin capsules, solutions, emulsions or suspensions. They can also be administered rectally, for example, in the form of suppositories, or parenterally, for example, in the form of injection solutions.

The pharmaceutical compositions of the present invention comprising compounds of formula I or II, prodrugs of such compounds, or the salts thereof, may be manufactured in a manner that is know in the art, *e.g.* by means of conventional mixing, encapsulating, dissolving, granulating, emulsifying, entrapping, dragee-making, or lyophilizing processes. These pharmaceutical preparations can be formulated with therapeutically inert, inorganic or organic carriers. Lactose, maize starch or derivatives thereof, talc, steric acid or its salts can be used as such carriers for tablets, coated tablets, dragees and hard gelatin capsules. Suitable carriers for soft gelatin capsules are vegetable oils, waxes, fats, semi-solid or liquid poll. Depending on the nature of the active substance, no carriers are generally required in the case of soft gelatin capsules. Suitable carriers for the manufacture of solutions and syrups are water, polyols, saccharose, invert sugar and glucose. Suitable carriers for injection are water, alcohols, polyols, glycerin, vegetable oils, phospholipids and surfactants. Suitable carriers for suppositories are natural or hardened oils, waxes, fats and semi-liquid polyols.

The pharmaceutical preparations can also contain preserving agents, solubilizing agents, stabilizing agents, wetting agents, emulsifying agents, sweetening agents, coloring agents, flavoring agents, salts for varying the osmotic pressure, buffers, coating agents or antioxidants. They can also contain other therapeutically valuable substances, including additional active ingredients other than those of formula I or II.

As mentioned above, the compounds of formula I or II, prodrugs thereof, and their salts, and compositions containing these compounds are useful in the treatment or control of inflammatory diseases and neuro-degenerative diseases, in particular, in the treatment or control of rheumatoid arthritis.

A therapeutically effective amount of a compound in accordance with this invention means an amount of compound that is effective to prevent, alleviate or ameliorate symptoms of disease of the subject being treated. Determination of a therapeutically effective amount is within the skill in the art.

The therapeutically effective amount or dosage of a compound of formula I or II can vary within wide limits and will be adjusted to the individual requirements in each particular case. In general, in the case of oral or parenteral administration to adult humans weighing approximately 70 Kg, a daily dosage of about 10 mg to about 10,000 mg. preferably from about 200 mg to about 1,000 mg, should be appropriate, although the upper limit may be exceeded when indicated. The daily dosage can be administered as a single dose or in divided doses, or for parenteral administration, it may be given as continuous infusion.

The compounds of the present invention may be synthesized according to known techniques, such as for example General Scheme I provided above. The following examples illustrate preferred methods for synthesizing the compounds and formulations of the present invention.

Example 1: General Synthesis Methods and Starting Materials

**Method A: Preparation of 1-alkyl or 1-aryl-2-propyn-1-ols via Grignard addition to aldehydes**

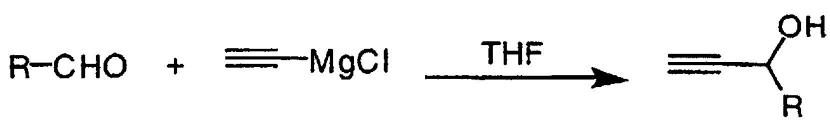


A solution of the appropriate aldehyde (4.0 mmol) in 30 mL dry tetrahydrofuran, under argon, was cooled to 0 °C with an ice bath. Ethynylmagnesium chloride (5 mmol, 0.5 M solution in THF) was added dropwise, and the solution was stirred at 0 °C or room temperature for 1 to 3 h. The reaction was quenched by the addition of a saturated ammonium chloride solution in water (15 mL), and the tetrahydrofuran was evaporated *in vacuo*. The residue was then extracted with ethyl acetate (3x30 mL), and the combined organic extracts were dried over magnesium sulfate, and concentrated *in vacuo* to yield the desired propargyl alcohol which was used in the coupling reaction without further purification.

**Method B: Preparation of 1-alkyl or 1-aryl-2-propyn-1-ols via Grignard addition to aldehydes**

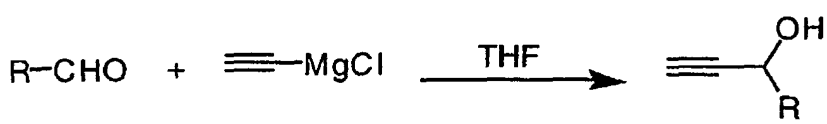


A solution of the appropriate aldehyde (4.0 mmol) in 30 mL dry tetrahydrofuran, under argon, was cooled to 0 °C with an ice bath. Ethynylmagnesium chloride (10 mmol, 0.5 M solution in THF) was added dropwise, and the solution was stirred at 0 °C or room temperature for 1 to 3 h. The reaction was quenched by the addition of a saturated ammonium chloride solution in water (15 mL), and the tetrahydrofuran was evaporated *in vacuo*. The residue was then extracted with ethyl acetate (3x30 mL), and the combined organic extracts were dried over magnesium sulfate, and concentrated *in vacuo* to yield the desired propargyl alcohol which was used in the coupling reaction without further purification.

**Method C: Preparation of 4-alkynyloxindoles via Palladium(0)-mediated coupling**

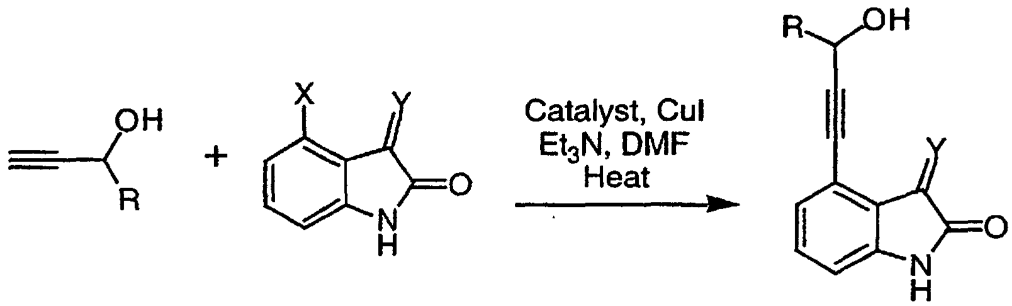


A solution of the appropriate 4-iodooxindole (4 mmol), and the appropriate alkyne (4.4 mmol) in 3 mL dimethylformamide and 3 mL triethylamine was degassed by bubbling argon through the solution for 15 minutes. At this time, copper (I) iodide (16 mg, 0.1 mmol) and palladium (0) catalyst *(see* Examples) (0.04 mmol) were added, and the reaction was heated, under argon, at a temperature between 60 to 90 °C, for 6 to 96 hours. After cooling, water (20 mL) was added and the precipitate was filtered off and dried. The product was purified via either flash column chromatography ($SiO_2$, 230-400 mesh with ethyl acetate/hexane as solvent) or with reverse phase HPLC (using either acetonitrile / water or acetonitrile / water / trifluoroacetic acid as solvent).

**Method D: Preparation of 4-alkynyloxindoles via Palladium(0)-mediated coupling**

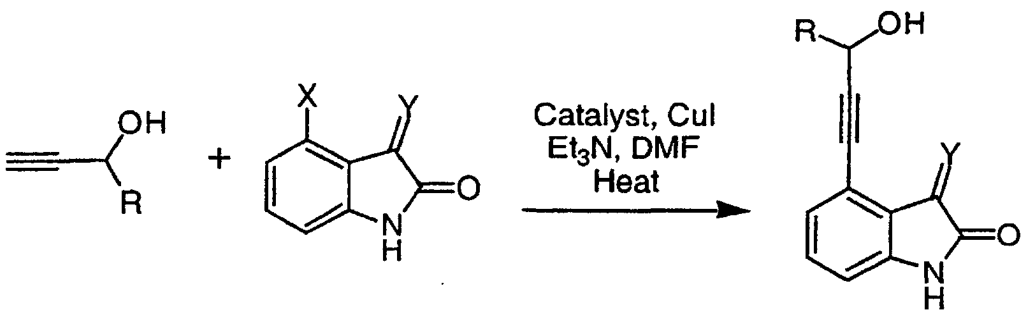


A solution of the appropriate 4-bromooxindole (4 mmol), and the appropriate alkyne (4.4 mmol) in 3 mL dimethylformamide and 3 mL triethylamine was degassed by bubbling argon through the solution for 15 minutes. At this time, copper (I) iodide (16 mg, 0.1 mmol) and catalyst (0.04 mmol) were added, and the reaction was heated, under argon, at between 60 to 90 °C for 6 to 96 hours. After cooling, water (20 mL) was added and the precipitate was filtered off and dried. The product was purified via either flash column chromatography ($SiO_2$, 230-400 mesh with ethyl acetate/hexane as solvent) or with reverse phase HPLC (using either acetonitrile / water or acetonitrile / water / trifluoroacetic acid as solvent).

**Method E: Preparation of methyl esters from carboxylic acids**

$$R{-}CO_2H + CH_2N_2 \xrightarrow{\text{Ether}} R{-}CO_2CH_3$$

To a solution of the appropriate carboxylic acid (15.3 mmol) in diethyl ether (30 mL) was added a solution of diazomethane (20 mmol, 0.47 M in ether). The reaction was stirred at room temperature for 1 hour at which time a few drops of

acetic acid was added. The solution was washed with saturated sodium bicarbonate (3x25 mL) and the solvent was evaporated to yield the desired methyl ester which was used without further purification.

**Method F: Preparation of carboxylic acids from the methyl esters**

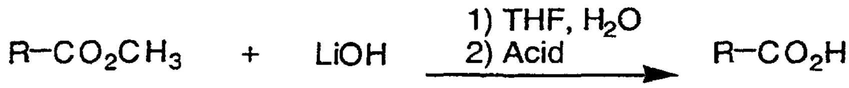


The appropriate methyl ester (0.14 mmol) was dissolved in a mixture of 2 mL tetrahydrofuran and 2 mL water. Lithium hydroxide (2.8 mmol, 20 equiv.) was added, and the reaction was stirred at room temperature from 1 to 96 hours. The tetrahydrofuran was then evaporated and 10 mL water was added. The aqueous layer was then extracted with ethyl acetate (2x10 mL) and the aqueous layer was then acidified to pH = 2 with 1 N hydrochloric acid. The aqueous layer was then extracted with ethyl acetate (4x20 mL), and the combined organic extracts were washed with a saturated solution of sodium chloride and were then dried over magnesium sulfate. The ethyl acetate was then evaporated and the product was recrystallized from ethanol.

**Method H: Mitsunobu Coupling of N-(2-hydroxyethyl)morpholine to phenols**

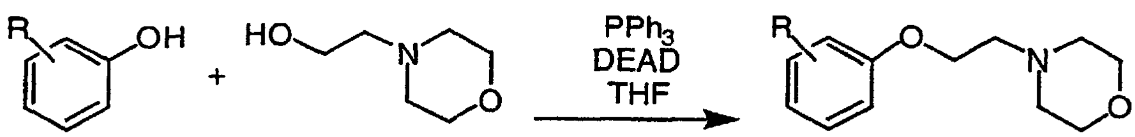


To a solution of the appropriate phenol (3.3 mmol), N-(2-hydroxyethyl)morpholine (4.9 mmol), and triphenylphosphine (5.0 mmol) in tetrahydrofuran (30 mL), under argon, was added via an addition funnel a solution of diethyl azodicarboxylate (5.0 mmol, 0.863 g) in 15 mL tetrahydrofuran. The reaction was stirred at room temperature for 14 hours at which time water (15 mL) was added and the tetrahydrofuran was evaporated. The aqueous layer was extracted with ethyl acetate (4x30 mL), and the combined organic extracts were washed with a saturated solution of sodium chloride, dried over magnesium sulfate and the solvent evaporated. The product was purified via flash column chromatography ($SiO_2$, 230-400 mesh) with ethyl acetate/hexane.

**Method J: Preparation of 4-alkynyloxindoles via Palladium(0)-mediated coupling**

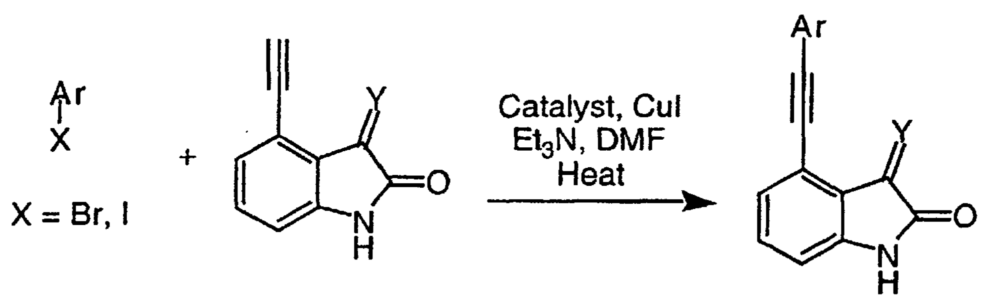


A solution of the appropriate 4-ethynyl-oxindole (4 mmol), and the appropriate aryl halide (4.4 mmol) in 3 mL dimethylformamide and 3 mL triethyl amine was degassed by bubbling argon through the solution for 15 minutes. At this time, copper (I) iodide (16 mg, 0.1 mmol) and palladium (0) catalyst (0.04 mmol) were added, and the reaction was heated, under argon, at between 60 to 90 °C for 12 to 96 hours. After cooling, water (20 mL) was added and the precipitate was filtered off and dried. The product was purified via either flash column chromatography ($SiO_2$, 230-400 mesh with

ethyl acetate/hexane as solvent) or with reverse phase HPLC (using either acetonitrile / water or acetonitrile / water / trifluoroacetic acid as solvent).

**Method K: Hydrolysis of trimethylsilyl alkyne to alkyne**

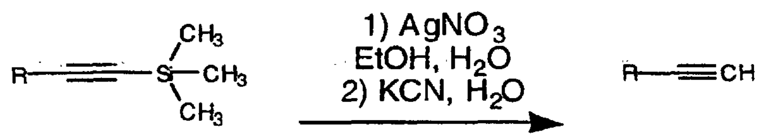


To a solution of the appropriate trimethylsilyl alkyne (4 mmol) in EtOH (80 mL), with addition of THF until complete dissolution if necessary, was added dropwise a solution of $AgNO_3$ (1.46 g, 8.59 mmol) in EtOH (5 mL) and water (15 mL). The mixture was stirred at room temperature for 1 h, then treated with a solution of KCN (2.71 g 41.6 mmol) in water (10 mL). After stirring for an additional 20 min, the reaction mixture was diluted with water (100 mL) and extracted with EtOAc (3X 100 mL). Combined EtOAc layers was dried ($MgSO_4$) and concentrated to dryness under reduced pressure to yield the above-identified product.

**Method L:**

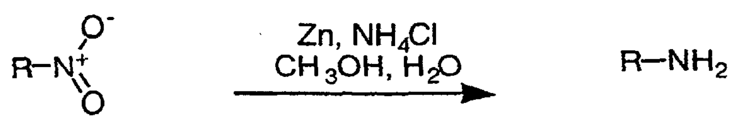


To a solution of nitro compound in 10% water in methanol was added Zn dust and $NH_4Cl$. The mixture was heated at reflux for 6 h then filtered through Celite® (Fisher Scientific). Filtrate was concentrated in vacuo. The product was purified via either flash column chromatography ($SiO_2$, 230-400 mesh with ethyl acetate/hexane as solvent) or with reverse phase HPLC (using either acetonitrile /water or acetonitrile / water / trifluoroacetic acid as solvent).

**Method M:**

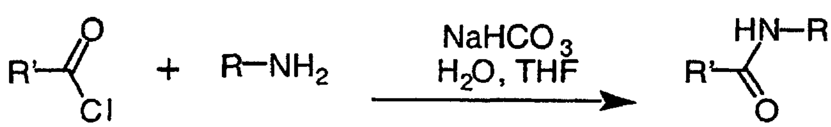


To a mixture of amino compound in THF and saturated aqueous $NaHCO_3$ was added a THF solution of the acid chloride dropwise. The mixture was stirred for 3 h to 10 days at room temperature then diluted with ethyl acetate. The phases were separated and the organic solution was washed with water then dried ($MgSO_4$). The product was purified via either flash column chromatography ($SiO_2$, 230-400 mesh with ethyl acetate/hexane as solvent) or with reverse phase HPLC (using either acetonitrile / water or acetonitrile / water / trifluoroacetic acid as solvent).

**Method N: Preparation of 3-arylmethylene-substituted oxindoles via coupling with aldehyde**

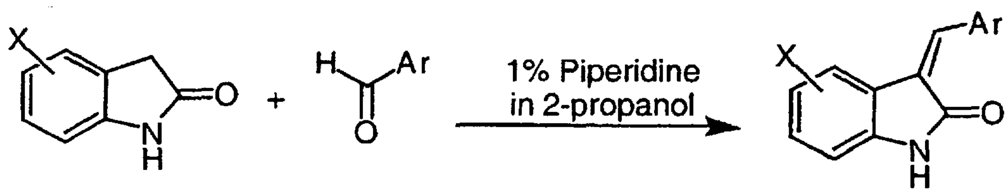


A solution or suspension of the appropriate oxindole (1 mmol), and excess aldehyde (1 to 2 mmol) in 2 mL of 1% piperidine in 2-propanol was heated at between 60 to 90 °C for 1 to 48 hours. Hot water (2 mL) was added. On cooling, the crystallized product was filtered off, washed with aqueous 2-propanol, and dried.

**Starting Material 1: (Z)-4-Bromo-1,3-dihydro-3-[(3-methoxy-1H-pyrrol-2-yl)methylene]-2H-indol-2-one**

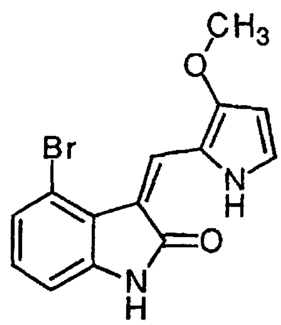

A mixture of 4-bromo-1,3-dihydro-2H-indol-2-one (100 mg, 0.47 mmol) (prepared according to T. Kosuge et al., *Chem. Pharm. Bull.* 33(4):1414-1418 (1985)), and excess 3-methoxy-2-pyrrolecarboxyaldehyde (70.8 mg, 0.57 mmol) (prepared according to F. Bellamy, *J. Chem. Research (S)* (1979) 18-19; *J. Chem. Research (M)* (1979) 0106-0116) in 1% piperidine in 2-propanol (1 mL) was heated at 85 °C for 2 h. Hot water (1 mL) was added. On cooling, the crystallized product was filtered off, washed with aqueous 2-propanol and dried. (Yield 0.13 g, 83%).

**Starting Material 2: (Z)-1,3-Dihydro-4-iodo-3-[(3-methoxy-1H-pyrrol-2-yl)methylene]-2H-indol-2-one**

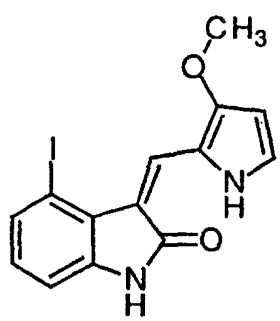

A mixture of 1,3-dihydro-4-iodo-2H-indol-2-one (prepared according to T. Fukuyama et al., *J. A. Chem. Soc.* 118:7426-7427 (1996)) (0.51 g, 1.97 mmol), and excess 3-methoxy-2-pyrrolecarboxyaldehyde (0.30 g, 2.36 mmol) *(see* Bellamy, *supra)* in 1% piperidine in 2-propanol (10 mL) was heated at 85 °C for 4 h. Hot water (10 mL) was added. On cooling, the crystallized product was filtered off, washed with aqueous 2-propanol and dried. (Yield 0.46 g, 64%).

**Starting Material 3: (Z)-4-bromo-1,3-dihydro-3-[(3-methoxy-1H-pyrrol-2-yl)methylene]-5-nitro-2H-indol-2-one**

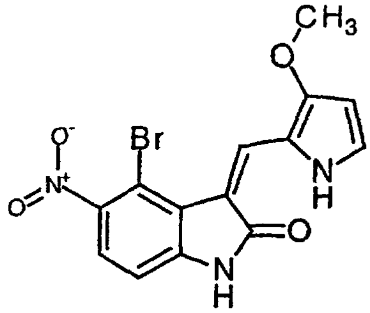

A mixture of 4-bromo-1,3-dihydro-5-nitro-2H-indol-2-one (from Example 4 *infra*) (0.113 g, 0.44 mmol), and excess 3-methoxy-2-pyrrolecarboxyaldehyde (66.3 mg, 0.53 mmol) *(see* Bellamy, *supra*) in 1% piperidine in 2-propanol (2 mL) was heated at 85 °C for 3 h. Hot water (2 mL) was added. On cooling, the crystallized product was filtered off, washed with aqueous 2-propanol and dried. (Yield 0.136 g, 85%).

**Starting Material 4: (Z)-1,3-Dihydro-3-[(3-methoxy-1H-pyrrol-2-yl)methylene]-4-(2-trimethylsilyl-ethynyl)-2H-indol-2-one**

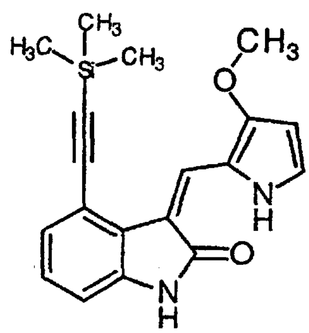

Trimethylsilyl acetylene (0.94 g, 9.63 mmol) (Aldrich) was coupled with (Z)-4-bromo-1,3-dihydro-3-[(3-methoxy-1H-pyrrol-2-yl)methylene]-2H-indol-2-one (2.05 g, 6.42 mmol) (Starting Material 1) using $(Ph_3P)_2PdCl_2$ (0.23 g) (Aldrich) and CuI (61 mg) (Aldrich) as catalyst in DMF (15 mL) and $Et_3N$ (15 mL) as solvent at 80 °C for 2 days in accordance with method D above. (Yield 1.3 g, 60%).

**Starting Material 5: (Z)-1,3-Dihydro-4-ethynyl-3-[(3-methoxy-1H-pyrrol-2-yl)methylene]-2H-indol-2-one**

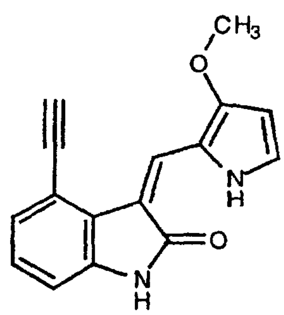

A solution of (Z)-1,3-dihydro-3-[(3-methoxy-1H-pyrrol-2-yl)methylene]-4-(2-trimethylsilyl-ethynyl)-2H-indol-2-one (1.3 g, 3.86 mmol) (Starting Material 4) in EtOH (80 mL) was treated with $AgNO_3$ (1.46 g, 8.59 mmol) in ethanol (5 mL) and water (15 mL) at room temperature for 1 h followed by KCN (2.71 g, 41.6 mmol) in water (10 mL) according to method K above. (Yield 1.02 g, 100%).

**Starting Material 6: 5-Bromo-1,3-dihydro-2H-indol-2-one**

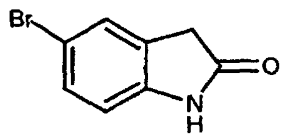

1,3-Dihydro-2H-indol-2-one (5.25 g, 39.43 mmol) (Aldrich) was treated with a 1:1 solution of glacial acetic acid and distilled water (246 mL). The resulting reaction mixture was cooled to 0 °C and then slowly treated with N-bromosuccinimide (14.03 g, 78.85 mmol) (J.T. Baker). After the complete addition of N-bromosuccinimide, the cooling bath was removed, and the reaction mixture was stirred at 23 °C for 1 h. Upon stirring at 23 °C, the reaction mixture became viscous and a white solid precipitated. The reaction mixture was poured into 500 mL distilled water and filtered to provide a crude white solid. Recrystallization from methanol provided pure 5-bromo-1,3-dihydro-2H-indol-2-one as a light pink solid. (Yield 5.28 g, 63%; mp 219 - 220 °C).

**Starting Material 7: (Z)-5-Bromo-1,3-dihydro-3-[(1H-pyrrol-2-yl)methylene]-2H-indol-2-one**

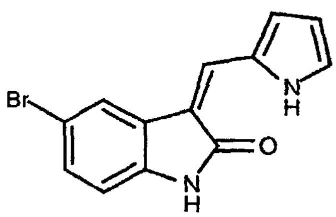

A mixture of 5-bromo-1,3-dihydro-2H-indol-2-one (3.10 g, 14.62 mmol) (Starting Material 6) and pyrrole-2-carboxaldehyde (1.46 g, 15.35 mmol) (Aldrich) in 2-propanol (73 mL) was treated with 10-12 drops of piperidne. The reaction mixture was heated at reflux for 20 h and then allowed to cool to 23 °C, at which time, the reaction mixture was filtered. The resulting solid was washed well with hexanes, followed by petroleum ether, and then allowed to air dry to provide pure (Z)-5-bromo-1,3-dihydro-3-[(1H-pyrrol-2-yl)methylene]-2H-indol-2-one as a yellow solid which was used without further purification. (Yield 4.01 g, 95%; mp 267 - 268 °C).

**Starting Material 8: (Z)-1,3-Dihydro-5-iodo-3-[(1H-pyrrol-2-yl)methylene]-2H-indol-2-one**

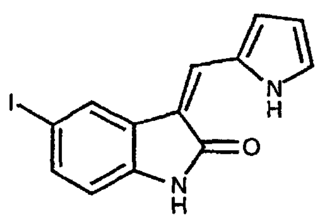

To a solution of [[1-[[(1,1-dimethylethyl)oxy]carbonyl]-1H-pyrrol-2-yl]methyl]triphenylphosphonium iodide (2.3 g, 4.0 mmol) (prepared according to the procedure of: V. H. Rawal et. al., *J. Org. Chem.* **1987,** *52(1),* 19-28) in 36 mL DMF at 0 °C under argon, was added slowly NaH (0.13 g, 5.4 mmol). The mixture was stirred at 0 °C for 45 min. The

solution was then allowed to warm to room temperature and 5-iodoisatin (1.0 g, 3.66 mmol) was added. The solution was heated at reflux for 15 h, at which time acetone (1 mL) was added and the solvent mixture was evaporated. The residue was then purified via flash column chromatography (25% EtOAc/hex) to yield (Z)-1,3-dihydro-5-iodo-3-[(1H-pyrrol-2-yl)methylene]-2H-indol-2-one. (Yield 1.05 g, 83%).

**Starting Material 9: (Z)-4-Bromo-1,3-dihydro-3-[(1H-pyrrol-2-yl)methylene]-2H-indol-2-one**

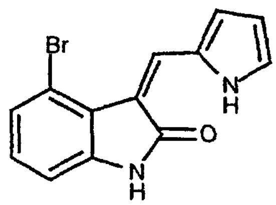

A mixture of 4-bromo-1,3-dihydro-2H-indol-2-one (0.2 g, 0.94 mmol) (*see* T. Kosuge et. al., *Chem. Pharm. Bull.* 33(4):1414-1418 (1985)), and excess pyrrole-2-carboxaldehyde (0.11 g, 1.13 mmol) (Aldrich) in 1% piperidine in 2-propanol (2 mL) was heated at 85 °C for 2 h. Hot water (2 mL) was added. On cooling, the crystallized product was filtered off, washed with aqueous 2-propanol and dried. (Yield 0.26 g, 96%)

**Example 3: Synthesis of 1,3-Dihydro-4-iodo-5-nitro-2H-indol-2-one (B)**

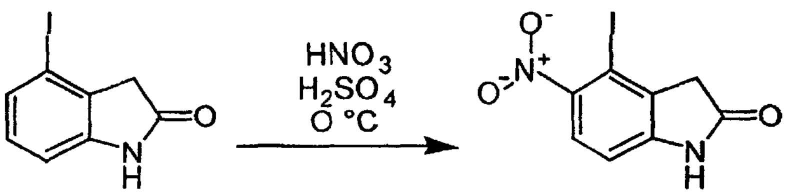


A mixture of concentrated sulfuric acid (0.73 mL) and concentrated nitric acid (0.14 mL) was added slowly to a solution of 1,3-dihydro-4-iodo-2H-indol-2-one (0.5 g, 1.93 mmol) (*see* Fukuyama, *supra*) in concentrated sulfuric acid (6 mL) at -5 °C, with stirring. The mixture was stirred for an additional 15 min at -5 °C, then poured onto ice. After standing for 1 h, solid was collected by filtration and washed with water, and dried in a vacuum oven to give 1,3-dihydro-4-iodo-5-nitro-2H-indol-2-one. (Yield 0.46 g, 78%).

**Example 4: Synthesis of 4-Bromo-1,3-dihydro-5-nitro-2H-indol-2-one (C)**

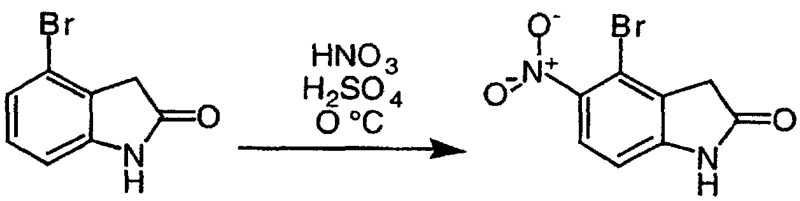


A mixture of concentrated sulfuric acid (3.6 mL) and concentrated nitric acid (0.7 mL) was added slowly to a solution of 4-bromo-1,3-dihydro -2H-indol-2-one (2 g, 9.48 mmol) (prepared according to T. Kosuge et al., *Chem. Pharm. Bull.* 33(4):1414-1418 (1985)) in concentrated sulfuric acid (20 mL) at -5 °C, with stirring. The mixture was stirred for an additional 1 h at -5 °C, then poured in ice. After standing for 1 h, precipitate formed was collected by filtration and washed with water, and dried in a vacuum oven to give 4-bromo-1,3-dihydro-5-nitro-2H-indol-2-one. (Yield 2.33 g, 96%).

**Example 5: Synthesis of (Z)-1,3-Dihydro-4-(phenylethynyl)-3-[(3-methoxy-1H-pyrrol-2-yl)methylene]-2H-indol-2-one (D)**

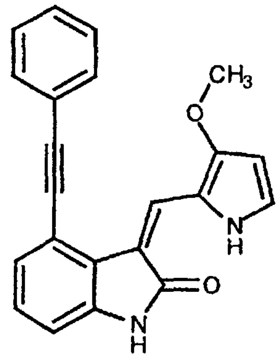

Using general Method D above, phenyl acetylene (32 mg, 0.31 mmol) (Aldrich) was coupled with (Z)-4-bromo-1,3-dihydro-3-[(3-methoxy-1H-pyrrol-2-yl)methylene]-2H-indol-2-one (50 mg, 0.16 mmol) (Starting Material 1) using DPPFPdCl$_2$ (6.5 mg) (Aldrich) and CuI (1.5 mg) (Aldrich) as catalyst in DMF (2 mL) and $Et_3N$ (3 mL) as solvent at 85 °C for 18 h, to yield (Z)-1,3-dihydro-4-(phenylethynyl)-3-[(3-methoxy-1H-pyrrol-2-yl)methylene]-2H-indol-2-one. (Yield 20 mg, 37%)

**Example 6: Synthesis of (Z)-1,3-Dihydro-4-(phenylethynyl)-3-[(1H-pyrrol-2-yl)methylene]-2H-indol-2-one (E)**

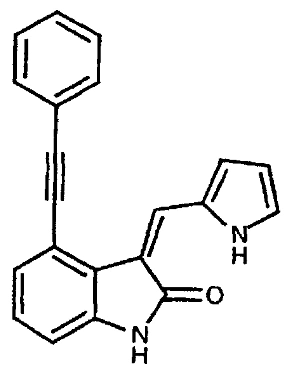

Using general Method D above, phenyl acetylene (32 mg, 0.31 mmol) (Aldrich) was coupled with (Z)-4-bromo-1,3-dihydro-3-[(1H-pyrrol-2-yl)methylene]-2H-indol-2-one (Starting Material 9) (46.3 mg, 0.16 mmol) using $(Ph_3P)_4Pd$ (8 mg) (Aldrich) and CuI (1.5 mg) (Aldrich) as catalyst in DMF (2 mL) and $Et_3N$ (3 mL) as solvent at 85 °C for 18 h, yielding (Z)-1,3-dihydro-4-(phenylethynyl)-3-[(1H-pyrrol-2-yl)methylene]-2H-indol-2-one. (Yield 41 mg, 83%)

**Example 7: Synthesis of (Z)-5-[3-[2,3-dihydro-2-oxo-3-(1H-pyrrol-2-ylmethylene)-1H-indol-4-yl]-2-propynyl]-6 (5H)-phenanthridinone (F)**

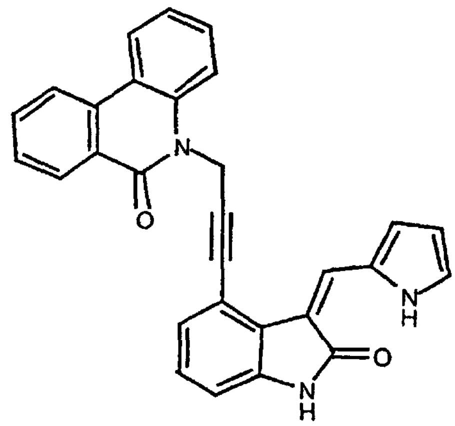

A solution of (Z)-1,3-dihydro-4-iodo-3-[(1H-pyrrol-2-yl)methylene]-2H-indol-2-one (from Example 71, *infra*) (50 mg, 0.15 mmol), and 5-(2-propynyl)-6(5H)-phenanthridinone (45 mg, 0.19 mmol) (prepared according to Walser et al., *J. Med. Chem.* 34(3), 1209-1221 (1991)) in 1 mL tetrahydrofuran and 1 mL triethylamine was degassed by bubbling argon through the solution for 10 minutes. At this time, copper (I) iodide (11 mg, 0.06 mmol) and tetrakis (triphenylphosphine) palladium(0) (3 mg, 0.03 mmol) were added, and the reaction was stirred at room temperature for 72 hours. Water (10 mL) was then added and the precipitate was filtered off and dried. The product was purified via flash column chromatography ($SiO_2$, 230-400 mesh) with ethyl acetate/hexane, to yield a yellow powder which was recrystallized from ethyl acetate/hexane, to give (Z)-5-[3-[2,3-dihydro-2-oxo-3-(1H-pyrrol-2-ylmethylene)-7H-indol-4-yl]-2-propynyl]-6(5H)-phenanthridinone. (Yield 25 mg, 38%).

**Example 8: Synthesis of (Z)-1,3-Dihydro-4-[(4-methoxyphenyl)ethynyl]-3-[(3-methoxy-1H-pyrrol-2-yl) methylene]-2H-indol-2-one (G)**

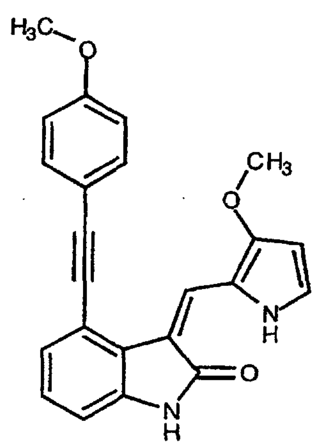

**Step A: 4-Methoxyphenyl acetylene**

Using Method D above, 4-bromoanisole (Aldrich) was coupled with trimethylsilylacetylene (Aldrich) using $(Ph_3P)_2PdCl_2$ and CuI as catalyst in DMF and $Et_3N$ as solvent and was heated at reflux for 1 day. The resulting trimethylsilyl derivative was hydrolized with aqueous potassium hydroxide to give 4-methoxyphenyl acetylene.

**Step B:** Using general Method D above, 4-methoxyphenyl acetylene (0.49 g, 3.68 mmol) (from Step A above)

was coupled with (Z)-4-bromo-1,3-dihydro-3-[(3-methoxy-1H-pyrrol-2-yl)methylene]-2H-indol-2-one (Starting Material 1) (0.47 g, 1.47 mmol) using $DPPFPdCl_2$ (0.12 g) (Aldrich) and CuI (28 mg) (Aldrich) as catalyst in DMF (10 mL) and $Et_3N$ (15 mL) as solvent and was heated at reflux for 1 day, yielding (Z)-1,3-dihydro-4-[(4-methoxyphenyl)ethynyl]-3-[(3-methoxy-1H-pyrrol-2-yl)methylene]-2H-indol-2-one. (Yield 0.31 g, 57%).

**Example 9: Synthesis of *rac*-(Z)-1,3-Dihydro-4-[3-hydroxy-3-(4-methoxyphenyl)-1-propynyl]-3-[(3-methoxy-1H-pyrrol-2-yl)methylene]-2H-indol-2-one (H)**

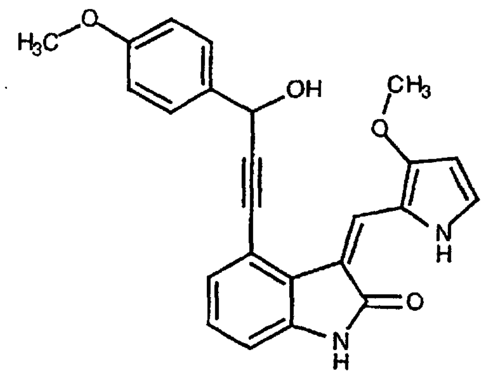

Using Method D above, 3-hydroxy-3-(4-methoxyphenyl)-1-propyne (115 mg, 0.70 mmol) (prepared by the addition of ethylmagnesium chloride (Aldrich) to 4-methoxybenzaldehyde (Aldrich) according to Method A above), was coupled with (Z)-4-bromo-1,3-dihydro-3-[(3-methoxy-1H-pyrrol-2-yl)methylene]-2H-indol-2-one (Starting Material 1) (174 mg, 0.55 mmol) using $(Ph_3P)_2PdCl_2$ (18 mg) (Aldrich) and CuI (10 mg) (Aldrich) as catalyst in DMF (2.5 mL) and $Et_3N$ (2.5 mL) as solvent at 70 °C for 15 h, yielding *rac*-(Z)-1,3-dihydro-4-[3-hydroxy-3-(4-methoxyphenyl)-1-propynyl]-3-[(3-methoxy-1H-pyrrol-2-yl)methylene]-2H-indol-2-one. (Yield 32 mg, 32%).

**Example 10: Synthesis of *rac*-(Z)-1,3-Dihydro-4-[3-hydroxy-3-(3-hydroxyphenyl)-1-propynyl]-3-[(3-methoxy-1H-pyrrol-2-yl)methylene]-2H-indol-2-one (I)**

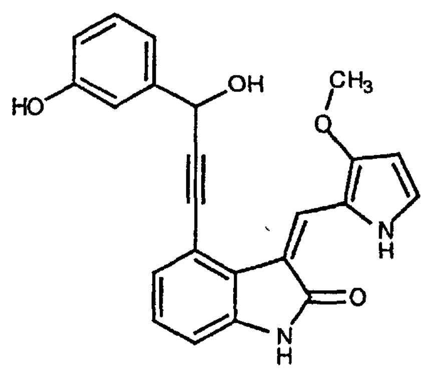

Using Method D above, 3-hydroxy-3-(3-hydroxyphenyl)-1-propyne (140 mg, 0.94 mmol) (prepared by the addition of ethynylmagnesium chloride (Aldrich) to 3-hydroxybenzaldehyde (Aldrich) through Method B above) was coupled to (Z)-4-bromo-1,3-dihydro-3-[(3-methoxy-1H-pyrrol-2-yl)methylene]-2H-indol-2-one (Starting Material 1) (200 mg, 0.63 mmol) using $(Ph_3P)_2PdCl_2$ (68 mg) (Aldrich) and CuI (32 mg) (Aldrich) as catalyst in DMF (4 mL) and $Et_3N$ (4 mL) as solvent at 80 °C for 15 h, yielding *rac*-(Z)-1,3-dihydro-4-[3-hydroxy-3-(3-hydroxyphenyl)-1-propynyl]-3-[(3-methoxy-1H-pyrrol-2-yl)methylene]-2H-indol-2-one. (Yield 82 mg, 31%).

**Example 11: Synthesis of *rac*-(Z)-1,3-Dihydro-4-[3-hydroxy-3-(3-methoxyphenyl)-1-propynyl]-3-[(3-methoxy-1H-pyrrol-2-yl)methylene]-2H-indol-2-one (J)**

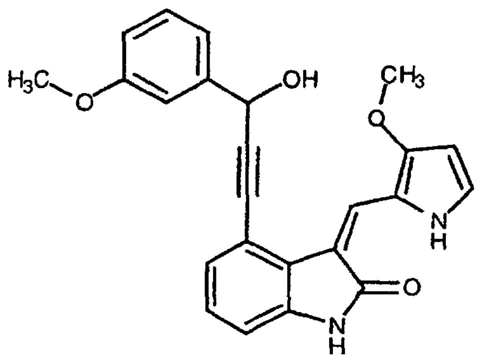

Using Method D above, 3-hydroxy-3-(3-methoxyphenyl)-1-propyne (151 mg, 1.0 mmol) (prepared by the addition of ethynylmagnesium chloride (Aldrich) to 3-methoxybenzaldehyde (Aldrich) according to Method A above) was coupled to (Z)-4-bromo-1,3-dihydro-3-[(3-methoxy-1H-pyrrol-2-yl)methylene]-2H-indol-2-one (Starting Material 1) (220 mg, 0.69 mmol) using $(Ph_3P)_2PdCl_2$ (100 mg) (Aldrich) and CuI (55 mg) (Aldrich) as catalyst in DMF (5 mL) and $Et_3N$ (5 mL) as solvent at 70 °C for 18 h, yielding *rac*-(Z)-1,3-dihydro-4-[3-hydroxy-3-(3-methoxyphenyl)-1-propynyl]-3-[(3-methoxy-1H-pyrrol-2-yl)methylene]-2H-indol-2-one. (Yield 66 mg, 24%).

**Example 12: Synthesis of *rac*-(Z)-4-[3-[2,3-Dihydro-3-[(3-methoxy-1H-pyrrol-2-yl)methylene]-2-oxo-1H-indol-4-yl]-1-hydroxy-2-propynyl]benzoic acid methyl ester (K)**

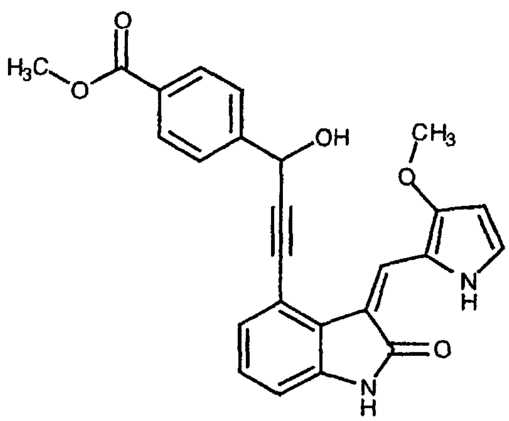

Using Method D above, 4-(1-hydroxy-2-propynyl)-benzoic acid methyl ester (137 mg, 0.72 mmol) (prepared by the addition of ethynylmagnesium chloride (Aldrich) to 4-carboxybenzaldehyde (Aldrich) using Method B above to give the acid which was converted to its methyl ester by Method E above), was coupled to (Z)-4-bromo-1,3-dihydro-3-[(3-methoxy-1H-pyrrol-2-yl)methylene]-2H-indol-2-one (Starting Material 1) (221 mg, 0.69 mmol) using $(Ph_3P)_2PdCl_2$ (37 mg) (Aldrich) and CuI (18 mg) (Aldrich) as catalyst in DMF (4 mL) and $Et_3N$ (4 mL) as solvent at 70 °C for 18 h, yielding *rac*-(Z)-4-[3-[2,3-dihydro-3-[(3-methoxy-1H-pyrrol-2-yl)methylene]-2-oxo-1H-indol-4-yl]-1-hydroxy-2-propynyl]benzoic acid methyl ester. (Yield 52 mg, 18%).

**Example 13: Synthesis of *rac*-(Z)-4-[3-[2,3-Dihydro-3-[(3-methoxy-1H-pyrrol-2-yl)methylene]-2-oxo-1H-indol-4-yl]-1-hydroxy-2-propynyl]benzoic acid (L)**

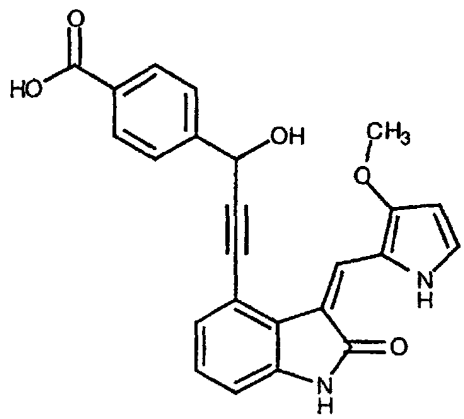

Using Method F above, 4-[1-hydroxy-3-[3-(3-methoxy-1H-pyrrol-2-ylmethylene)-2-oxo-2,3-dihydro-1H-indol-4-yl]-prop-2-ynyl]-benzoic acid methyl ester (30 mg, 0.07 mmol) (from Example 12 above) was hydrolyzed with $LiOH \cdot H_2O$ (13 mg, 2.7 mmol) in THF (1 mL) and water (1 mL) at room temperature for 18 h, to yield *rac*-(Z)-4-[3-[2,3-dihydro-3-[(3-methoxy-1H-pyrrol-2-yl)methylene]-2-oxo-1H-indol-4-yl]-1-hydroxy-2-propynyl]benzoic acid. (Yield 21 mg, 72%).

**Example 14: Synthesis of *rac*-(Z)-1,3-Dihydro-4-[3-hydroxy-3-(2-methoxyphenyl)-1-propynyl]-3-[(3-methoxy-1H-pyrrol-2-yl)methylene]-2H-indol-2-one (M)**

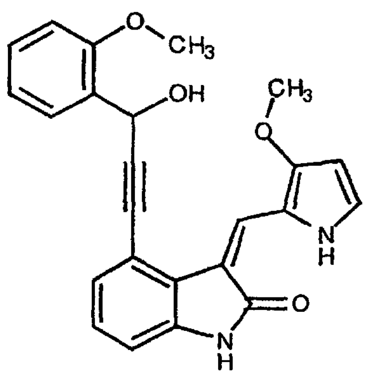

Using Method D above, 3-hydroxy-3-(2-methoxyphenyl)-1-propyne (150 mg, 0.92 mmol) (prepared by the addition of ethynylmagnesium chloride (Aldrich) to 2-methoxybenzaldehyde (Aldrich) according to Method A above), was coupled to (Z)-4-bromo-1,3-dihydro-3-[(3-methoxy-1H-pyrrol-2-yl)methylene]-2H-indol-2-one (Starting Material 1) (200 mg, 0.63 mmol) using $(Ph_3P)_2PdCl_2$ (70 mg) (Aldrich) and CuI (40 mg) (Aldrich) as catalyst in DMF (3 mL) and $Et_3N$ (3 mL) as solvent at 70 °C for 18 h, yielding *rac*-(Z)-1,3-dihydro-4-[3-hydroxy-3-(2-methoxyphenyl)-1-propynyl]-3-[(3-methoxy-1H-pyrrol-2-yl)methylene]-2H-indol-2-one. (Yield 71 mg, 28%).

**Example 15: Synthesis of *rac*-(Z)-4-[3-(1,3-benzodioxol-5-yl)-3-hydroxy-1-propynyl]-1,3-dihydro-3-[(3-methoxy-1H-pyrrol-2-yl)methylene]-2H-indol-2-one (N)**

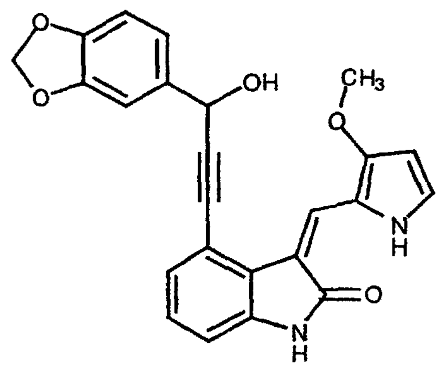

Using Method D above, 3-(1,3-benzodioxol-5-yl)-3-hydroxy-1-propyne (110 mg, 0.62 mmol) (prepared by the addition of ethynylmagnesium chloride (Aldrich) to piperonal (Aldrich) according to Method A above) was coupled to (Z)-4-bromo-1,3-dihydro-3-[(3-methoxy-1H-pyrrol-2-yl)methylene]-2H-indol-2-one (100 mg, 0.34 mmol) (Starting Material 1) using $(Ph_3P)_2PdCl_2$ (30 mg) (Aldrich) and CuI (16 mg) (Aldrich) as catalyst in DMF (3 mL) and $Et_3N$ (3 mL) as solvent at 70 °C for 16 h, yielding *rac*-(Z)-4-[3-(1,3-benzodioxol-5-yl)-3-hydroxy-1-propynyl]-1,3-dihydro-3-[(3-methoxy-1H-pyrrol-2-yl)methylene]-2H-indol-2-one. (Yield 46 mg, 33%).

**Example 16: Synthesis of *rac*-(Z)-1,3-Dihydro-4-[3-hydroxy-3-(4-hydroxy-3-methoxyphenyl)-1-propynyl]-3-[(3-methoxy-1H-pyrrol-2-yl)methylene]-2H-indol-2-one (O)**

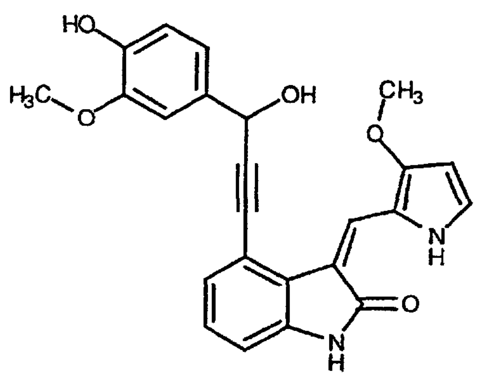

Using Method D above, 3-hydroxy-3-(4-hydroxy-3-methoxyphenyl)-1-propyne (197 mg, 1.1 mmol) (prepared by the addition of ethynylmagnesium chloride (Aldrich) to vanillin (Aldrich) according to Method B above) was coupled to (Z)-4-bromo-1,3-dihydro-3-[(3-methoxy-1H-pyrrol-2-yl)methylene]-2H-indol-2-one (Starting Material 1) (116 mg, 0.36 mmol) using $(Ph_3P)_2PdCl_2$ (33 mg) (Aldrich) and CuI (18 mg) (Aldrich) as catalyst in DMF (3 mL) and $Et_3N$ (3 mL) as solvent at 70 °C for 16 h, yielding *rac*-(Z)-1,3-dihydro-4-[3-hydroxy-3-(4-hydroxy-3-methoxyphenyl)-1-propynyl]-3-[(3-methoxy-1H-pyrrol-2-yl)methylene]-2H-indol-2-one. (Yield 31 mg, 21%).

**Example 17: Synthesis of 3-Hydroxy-3-(4-hydroxyphenyl)-1-propyne (P)**

To a solution of trimethylsilylacetylene (1.00 g, 10 mmol) (Aldrich) in dry THF (100 mL) under argon at -78 °C was added n-butyllithium (4.4 mL, 11 mmol, 2.5 M solution in hexanes) (Aldrich) dropwise. The reaction was stirred for 30 min at -78 °C, after which time 4-hydroxybenzaldehyde (0.50 g, 4 mmol) (Aldrich) was added and the reaction was allowed to slowly warm to room temperature. A saturated solution of ammonium chloride (5 mL) was then added

and the reaction was stirred at room temperature for 2 h. The solution was then diluted by the addition of 30 mL water, and THF was removed *in vacuo*. The product was extracted with ethyl acetate (3 x 50 mL) and the combined organic layers were dried over magnesium sulfate and concentrated in vacuo to give clean 3-hydroxy-3-(4-hydroxyphenyl)-1-propyne which was used directly, without further purification. (Yield 501 mg, 84%).

**Example 18: Synthesis of *rac*-(Z)-1,3-Dihydro-4-[3-hydroxy-3-(4-hydroxyphenyl)-1-propynyl]-3-[(3-methoxy-1H-pyrrol-2-yl)methylene]-2H-indol-2-one (Q)**

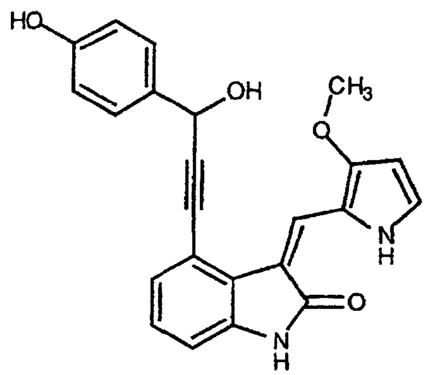

Using Method D above, 3-hydroxy-3-(4-hydroxyphenyl)-1-propyne (120 mg, 0.84 mmol) (from Example 17 above) was coupled to (Z)-4-bromo-1,3-dihydro-3-[(3-methoxy-1H-pyrrol-2-yl)methylene]-2H-indol-2-one (110 mg, 0.34 mmol) (Starting Material 1) using $(Ph_3P)_2PdCl_2$ (30 mg) (Aldrich) and CuI (15 mg) (Aldrich) as catalyst in DMF (3 mL) and $Et_3N$ (3 mL) as solvent at 70 °C for 16 h, yielding *rac*-(Z)-1,3-dihydro-4-[3-hydroxy-3-(4-hydroxyphenyl)-1-propynyl]-3-[(3-methoxy-1 H-pyrrol-2-yl)methylene]-2H-indol-2-one. (Yield 62 mg, 47%).

**Example 19: Synthesis of *rac*-(Z)-1,3-Dihydro-4-[3-(4-dimethylaminophenyl)-3-hydroxy-1-propynyl]-3-[(3-methoxy-1H-pyrrol-2-yl)methylene]-2H-indol-2-one (R)**

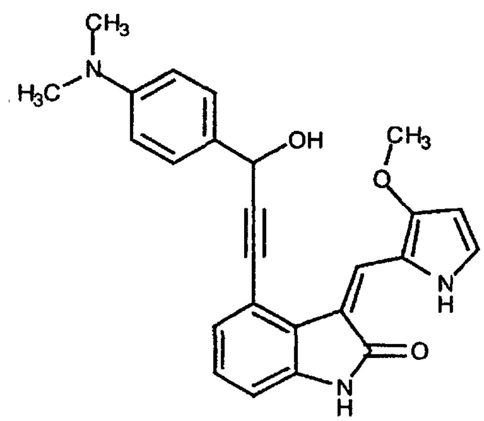

Using Method D above, 3-(4-dimethylaminophenyl)-3-hydroxy-1-propyne (160 mg, 0.91 mmol) (prepared by the addition of ethynylmagnesium chloride (Aldrich) to 4-dimethylaminobenzaldehyde (Aldrich) according to Method A above) was coupled to (Z)-4-bromo-1,3-dihydro-3-[(3-methoxy-1H-pyrrol-2-yl)methylene]-2H-indol-2-one (108 mg, 0.34 mmol) (Starting Material 1) using $(Ph_3P)_2PdCl_2$ (30 mg) (Aldrich) and CuI (16 mg) (Aldrich) as catalyst in DMF (3 mL) and $Et_3N$ (3 mL) as solvent at 70 °C for 19 h, yielding *rac*-(Z)-1,3-dihydro-4-[3-(4-dimethylaminophenyl)-3-hydroxy-1-propynyl]-3-[(3-methoxy-1H-pyrrol-2-yl)methylene]-2H-indol-2-one. (Yield 190 mg, 77%).

**Example 20: Synthesis of *rac*-(Z)-1,3-Dihydro-4-[3-hydroxy-3-(4-phenoxyphenyl)-1-propynyl]-3-[(3-methoxy-1H-pyrrol-2-yl)methylene]-2H-indol-2-one (S)**

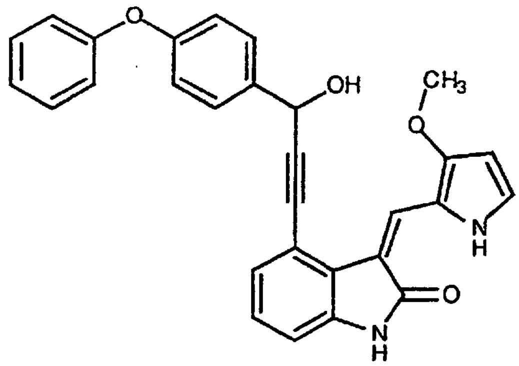

Using Method C above, 3-hydroxy-3-(4-phenoxyphenyl)-1-propyne (200 mg, 0.89 mmol) (prepared by the addition of ethynylmagnesium chloride (Aldrich) to 4-phenoxybenzaldehyde (Aldrich) according to Method A above) was coupled to (Z)-1,3-dihydro-4-iodo-3-[(3-methoxy-1H-pyrrol-2-yl)methylene]-2H-indol-2-one (Starting Material 2) (146 mg, 0.40 mmol) using $(Ph_3P)_2PdCl_2$ (30 mg) (Aldrich) and CuI (16 mg) (Aldrich) as catalyst in DMF (3 mL) and $Et_3N$ (3 mL) as solvent at 70 °C for 16 h, yielding *rac*-(Z)-1,3-dihydro-4-[3-hydroxy-3-(4-phenoxyphenyl)-1-propynyl]-3-[(3-methoxy-1H-pyrrol-2-yl)methylene]-2H-indol-2-one. (Yield 100 mg, 54%).

**Example 21: Synthesis of *rac*-(Z)-1,3-Dihydro-4-[3-hydroxy-3-phenyl-1-butynyl]-3-[(3-methoxy-1H-pyrrol-2-yl) methylene]-2H-indol-2-one (T)**

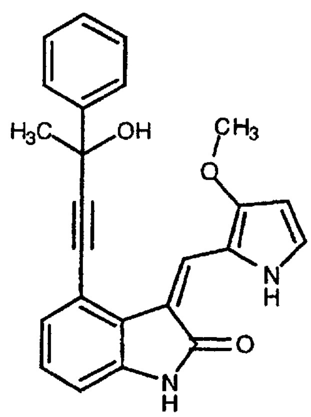

Using Method C above, 2-phenyl-3-butyn-2-ol (70 mg, 0.48 mmol) (Aldrich) was coupled to (Z)-1,3-dihydro-4-iodo-3-[(3-methoxy-1H-pyrrol-2-yl)methylene]-2H-indol-2-one (146 mg, 0.4 mmol) (Starting Material 2) using $(Ph_3P)_2PdCl_2$ (20 mg) (Aldrich) and CuI (10 mg) (Aldrich) as catalyst in DMF (2 mL) and $Et_3N$ (2 mL) as solvent at 70 °C for 15 h, yielding *rac*-(Z)-1,3-dihydro-4-[3-hydroxy-3-phenyl-1-butynyl]-3-[(3-methoxy-1H-pyrrol-2-yl)methylene]-2H-indol-2-one. (Yield 85 mg, 55%).

**Example 22: Synthesis of 3-[4-(3-Dimethylaminopropoxy)-phenyl]-3-hydroxy-1-propyne (U)**

To a solution of 4-(3-dimethylaminopropoxy)benzaldehyde (0.83 g, 4 mmol) (Aldrich) in dry tetrahydrofuran (30 mL) under argon at room temperature was added ethynylmagnesium chloride (5 mmmol, 10 mL, 0.5M solution in tetrahydrofuran) (Aldrich) dropwise. The resulting solution was stirred for 1.5 h at which time 100 mL water was added, and the THF was removed *in vacuo.* The product was extracted with ethyl acetate (3 x 50 mL) and the combined organic

layers were dried over magnesium sulfate and concentrated *in vacuo* to give clean 3-[4-(3-dimethylaminopropoxy)-phenyl]-3-hydroxy-1-propyne which was used directly, without further purification. (Yield 831 mg, 89%)

**Example 23: Synthesis of *rac*-(Z)-1,3-Dihydro-4-[3-[4-(3-dimethylaminopropoxy)-phenyl]-3-hydroxy-1-propynyl]-3-[(3-methoxy-1H-pyrrol-2-yl)methylene]-2H-indol-2-one (V)**

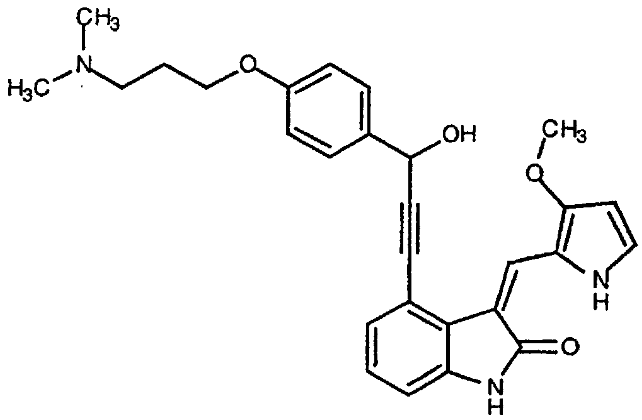

Using Method C above, 3-[4-(3-dimethylaminopropoxy)-phenyl]-3-hydroxy-1-propyne (201 mg, 0.86 mmol) (from Example 22 above) was coupled to (Z)-1,3-dihydro-4-iodo-3-[(3-methoxy-1H-pyrrol-2-yl)methylene)-2H-indol-2-one (Starting Material 2) (146 mg, 0.4 mmol) using $(Ph_3P)_2PdCl_2$ (34 mg) (Aldrich) and CuI (15 mg) (Aldrich) as catalyst in DMF (3 mL) and $Et_3N$ (3 mL) as solvent at 70 °C for 16 h, to yield *rac*-(Z)-1,3-dihydro-4-[3-[4-(3-dimethylaminopropoxy)-phenyl]-3-hydroxy-1-propynyl]-3-[(3-methoxy-1H-pyrrol-2-yl)methylene]-2H-indol-2-one. (Yield 151 mg, 80%).

**Example 24: Synthesis of 3-Hydroxy-3-(3-pyridinyl)-1-propyne (W)**

3-Hydroxy-3-(3-pyridinyl)-1-propyne was prepared according to Method A above using 3-pyridine carboxaldehyde (0.428 g, 4 mmol) (Aldrich) in THF (20 mL) and ethynylmagnesium chloride (5 mmol, 10 mL, 0.5M solution in tetrahydrofuran) (Aldrich). (Yield 440 mg, 83%).

**Example 25: Synthesis of *rac*-(Z)-1,3-Dihydro-4-[3-hydroxy-3-(3-pyridinyl)-1-propynyl]-3-[(3-methoxy-1H-pyrrol-2-yl)methylene]-2H-indol-2-one (X)**

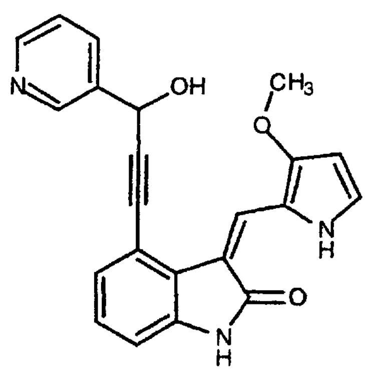

Using Method D above, 3-hydroxy-3-(3-pyridinyl)-1-propyne (150 mg, 1.13 mmol) (from Example 24 above) was coupled to (Z)-4-bromo-1,3-dihydro-3-[(3-methoxy-1H-pyrrol-2-yl)methylene]-2H-indol-2-one (135 mg, 0.42 mmol) (Starting Material 1) using $(Ph_3P)_2PdCl_2$ (32 mg) (Aldrich) and CuI (17 mg) (Aldrich) as catalyst in DMF (3 mL) and $Et_3N$ (3 mL) as solvent at 70 °C for 17 h, to yield *rac*-(Z)-1,3-dihydro-4-[3-hydroxy-3-(3-pyridinyl)-1-propynyl]-3-[(3-methoxy-1H-pyrrol-2-yl)methylene]-2H-indol-2-one. (Yield 40 mg, 22%).

**Example 26: Synthesis of (Z)-1,3-Dihydro-3-[(3-methoxy-1H-pyrrol-2-yl)methylene]-4-(3-phenoxy-1-propynyl)-2H-indol-2-one (Y)**

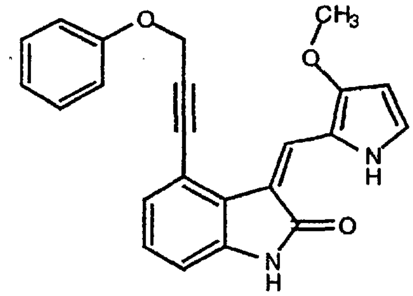

Using Method C above, phenyl propargyl ether (65 mg, 0.49 mmol) (Lancaster) was coupled to (Z)-1,3-dihydro-4-iodo-3-[(3-methoxy-1H-pyrrol-2-yl)methylene]-2H-indol-2-one (146 mg, 0.4 mmol) (Starting Material 2) using $(Ph_3P)_2PdCl_2$ (20 mg) (Aldrich) and CuI (10 mg) (Aldrich) as catalyst in DMF (2 mL) and $Et_3N$ (2 mL) as solvent at 70 °C for 18 h, yielding (Z)-1,3-dihydro-3-[(3-methoxy-1H-pyrrol-2-yl)methylene]-4-(3-phenoxy-1-propynyl)-2H-indol-2-one. (Yield 92 mg, 62%).

**Example 27: Synthesis of 3-Hydroxy-3-(1-methyl-pyrrol-2-yl)-1-propyne (Z)**

3-Hydroxy-3-(1-methyl-pyrrol-2-yl)-1-propyne was prepared according to Method A above using 1-methyl-2-pyrrolecarboxaldehyde (0.450 g, 4 mmol) (Aldrich) in THF (20 mL) and ethynylmagnesium chloride (5 mmol, 10 mL, 0.5M solution in tetrahydrofuran) (Aldrich). (Yield 422 mg, 76%).

**Example 28: Synthesis of *rac*-(Z)-1,3-Dihydro-4-[3-hydroxy-3-(1-methyl-pyrrol-2-yl)-1-propynyl]-3-[(3-methoxy-1H-pyrrol-2-yl)methylene]-2H-indol-2-one (AA)**

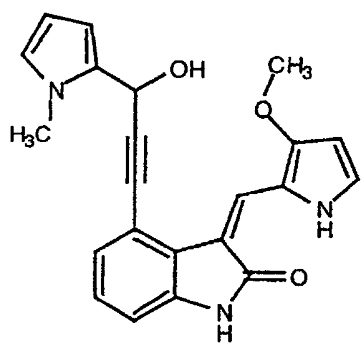

Using Method D above, 3-hydroxy-3-(1-methyl-pyrrol-2-yl)-1-propyne (132 mg, 0.98 mmol) (from Example 27 above) was coupled to (Z)-4-bromo-1,3-dihydro-3-[(3-methoxy-1H-pyrrol-2-yl)methylene]-2H-indol-2-one (112 mg, 0.35 mmol) (Starting Material 2) using $(Ph_3P)_2PdCl_2$ (31 mg)(Aldrich) and CuI (17 mg)(Aldrich) as catalyst in DMF (3 mL) and $Et_3N$ (3 mL) as solvent at 70 °C for 28 h, yielding *rac*-(Z)-1,3-dihydro-4-[3-hydroxy-3-(1-methyl-pyrrol-2-yl)-1-propynyl]-3-[(3-methoxy-1H-pyrrol-2-yl)methylene]-2H-indol-2-one. (Yield 114 mg, 87%).

**Example 29: Synthesis of *rac*-(Z)-1,3-Dihydro-4-[3-hydroxy-3-(thiophen-3-yl)-1-propynyl]-3-[(3-methoxy-1H-pyrrol-2-yl)methylene]-2H-indol-2-one (BB)**

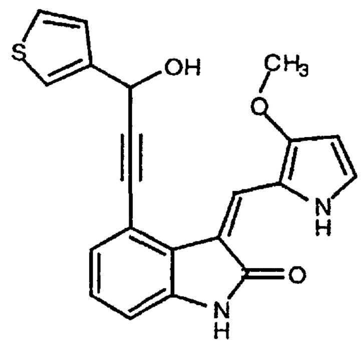

Using Method D above, 3-hydroxy-3-(thiophen-3-yl)-1-propyne (131 mg, 0.95 mmol) (prepared by the addition of ethynylmagnesium chloride (Aldrich) to 3-thiophenecarboxaldehyde (Aldrich) according to Method A above) was coupled to (Z)-4-bromo-1,3-dihydro-3-[(3-methoxy-1H-pyrrol-2-yl)methylene]-2H-indol-2-one (107 mg, 0.34 mmol) (Starting Material 1) using $(Ph_3P)_2PdCl_2$ (32 mg) (Aldrich) and CuI (17 mg) (Aldrich)as catalyst in DMF (3 mL) and $Et_3N$ (3 mL) as solvent at 70 °C for 18 h, yielding *rac*-(Z)-1,3-dihydro-4-[3-hydroxy-3-(thiophen-3-yl)-1-propynyl]-3-[(3-methoxy-1H-pyrrol-2-yl)methylene]-2H-indol-2-one. (Yield 93 mg, 72%).

**Example 30: Synthesis of 3-Hydroxy-3-(1H-pyrrol-2-yl)-1-propyne (CC)**

3-Hydroxy-3-(1H-pyrrol-2-yl)-1-propyne was prepared by Method B above using 2-pyrrolecarboxaldehyde (0.389 g, 4 mmol) (Aldrich) in THF (30 mL) and ethynylmagnesium chloride (20 mmol, 40 mL, 0.5M solution in tetrahydrofuran) (Aldrich). (Yield 345 mg, 82%).

**Example 31: Synthesis of *rac*-(Z)-1,3-Dihydro-4-[3-hydroxy-3-(1H-pyrrol-2-yl)-1-propynyl]-3-[(3-methoxy-1H-pyrrol-2-yl)methylene]-2H-indol-2-one (DD)**

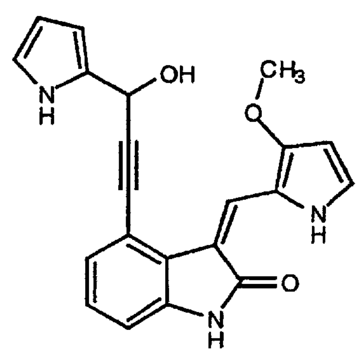

Using Method D above, 3-hydroxy-3-(1H-pyrrol-2-yl)-1-propyne (212 mg, 1.75 mmol) (from Example 30 above) was coupled to (Z)-4-bromo-1,3-dihydro-3-[(3-methoxy-1H-pyrrol-2-yl)methylene]-2H-indol-2-one (125 mg, 0.39 mmol) (Starting Material 1) using $(Ph_3P)_2PdCl_2$ (42 mg) (Aldrich) and CuI (20 mg) (Aldrich) as catalyst in DMF (4 mL) and $Et_3N$ (4 mL) as solvent at 70 °C for 18 h, yielding *rac*-(Z)-1,3-dihydro-4-[3-hydroxy-3-(1H-pyrrol-2-yl)-1-propynyl]-3-[(3-methoxy-1H-pyrrol-2-yl)methylene]-2H-indol-2-one. (Yield 68 mg, 49%).

**Example 32: Synthesis of *rac*-(Z)-1,3-Dihydro-4-[3-(2,3-dimethoxyphenyl)-3-hydroxy-1-propynyl]-3-[(3-methoxy-1H-pyrrol-2-yl)methylene]-2H-indol-2-one (EE)**

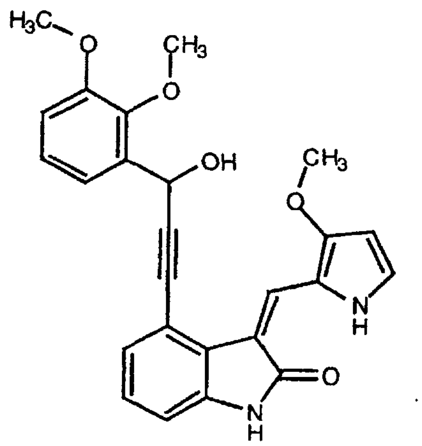

Using Method D above, 3-(2,3-dimethoxyphenyl)-3-hydroxy-1-propyne (132 mg, 0.69 mmol) (prepared by the addition of ethynylmagnesium chloride (Aldrich) to 2,3-dimethoxybenzaldehyde (Aldrich) according to Method A above) was coupled to (Z)-4-bromo-1,3-dihydro-3-[(3-methoxy-1H-pyrrol-2-yl)methylene]-2H-indol-2-one (Starting Material 1) (98 mg, 0.31 mmol) using $(Ph_3P)_2PdCl_2$ (38 mg) (Aldrich) and CuI (17 mg) (Aldrich) as catalyst in DMF (3 mL) and $Et_3N$ (3 mL) as solvent at 70 °C for 17 h, yielding *rac*-(Z)-1,3-dihydro-4-[3-(2,3-dimethoxyphenyl)-3-hydroxy-1-propynyl]-3-[(3-methoxy-1H-pyrrol-2-yl)methylene]-2H-indol-2-one. (Yield 98 mg, 71%).

**Example 33: Synthesis of *rac*-(Z)-1,3-Dihydro-4-[3-(3,4-dimethoxyphenyl)-3-hydroxy-1-propynyl]-3-[(3-methoxy-1H-pyrrol-2-yl)methylene]-2H-indol-2-one (FF)**

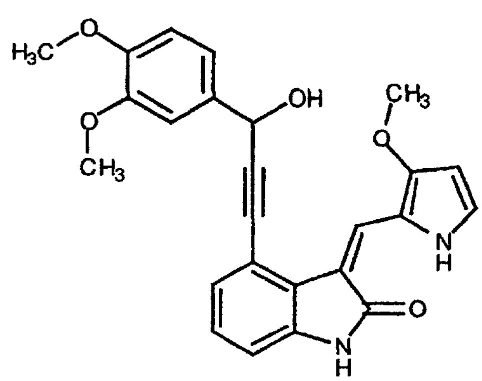

Using Method C above, 3-(3,4-dimethoxyphenyl)-3-hydroxy-1-propyne (150 mg, 0.78 mmol) (prepared by the addition of ethynylmagnesium chloride (Aldrich) to 3,4-dimethoxybenzaldehyde (Aldrich) according to Method A above) was coupled to (Z)-1,3-dihydro-4-iodo-3-[(3-methoxy-1H-pyrrol-2-yl)methylene]-2H-indol-2-one (Starting Material 2) (146 mg, 0.4 mmol) using $(Ph_3P)_2PdCl_2$ (40 mg) (Aldrich) and CuI (22 mg) (Aldrich) as catalyst in DMF (3 mL) and $Et_3N$ (3 mL) as solvent at 70 °C for 18 h, yielding *rac*-(Z)-1,3-dihydro-4-[3-(3,4-dimethoxyphenyl)-3-hydroxy-1-propynyl]-3-[(3-methoxy-1H-pyrrol-2-yl)methylene]-2H-indol-2-one. (Yield 121 mg, 70%).

**Example 34: Synthesis of 3-Hydroxy-3-(3-hydroxy-4-methoxyphenyl)-1-propyne (GG)**

3-Hydroxy-3-(3-hydroxy-4-methoxyphenyl)-1-propyne was prepared according to Method B above from 3-hydroxy-4-methoxybenzaldehyde (0.304 g, 2 mmol) (Aldrich) in THF (20 mL) and ethynylmagnesium chloride (5 mmol,

10 mL, 0.5M solution in tetrahydrofuran) (Aldrich). (Yield 273 mg, 77%).

**Example 35: Synthesis of *rac*-(Z)-1,3-Dihydro-4-[3-hydroxy-3-(3-hydroxy-4-methoxyphenyl)-1-propynyl]-3-[(3-methoxy-1H-pyrrol-2-yl)methylene]-2H-indol-2-one (HH)**

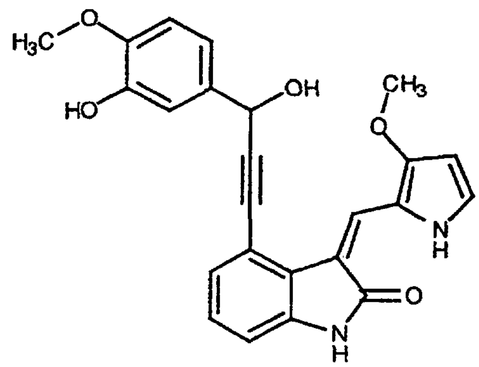

Using Method D above, 3-hydroxy-3-(3-hydroxy-4-methoxyphenyl)-1-propyne (105 mg, 0.59 mmol) (from Example 34 above) was coupled to (Z)-4-bromo-1,3-dihydro-3-[(3-methoxy-1H-pyrrol-2-yl)methylene]-2H-indol-2-one (122 mg, 0.38 mmol) (Starting Material 1) using $(Ph_3P)_2PdCl_2$ (34 mg) (Aldrich) and CuI (15 mg) (Aldrich) as catalyst in DMF (3 mL) and $Et_3N$ (3 mL) as solvent at 70 °C for 18 h, yielding *rac*-(Z)-1,3-dihydro-4-[3-hydroxy-3-(3-hydroxy-4-methoxyphenyl)-1-propynyl]-3-[(3-methoxy-1H-pyrrol-2-yl)methylene]-2H-indol-2-one. (Yield 101 mg, 64%).

**Example 36: Synthesis of 3-Hydroxy-3-(2-pyridinyl)-1-propyne (II)**

3-Hydroxy-3-(2-pyridinyl)-1-propyne was prepared according to Method A above from 2-pyridine carboxaldehyde (1.0 g, 9.3 mmol) (Aldrich) in THF (50 mL) and ethynylmagnesium chloride (10 mmol, 20 mL, 0.5M solution in tetrahydrofuran) (Aldrich). (Yield 956 mg, 77%).

**Example 37: Synthesis of *rac*-(Z)-1,3-Dihydro-4-[3-hydroxy-3-(2-pyridinyl)-1-propynyl]-3-[(3-methoxy-1H-pyrrol-2-yl)methylene]-2H-indol-2-one (JJ)**

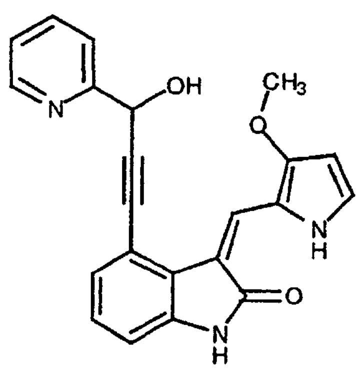

Using Method C above, 3-hydroxy-3-(2-pyridinyl)-1-propyne (133 mg, 1 mmol) (from Example 36 above) was coupled to (Z)-1,3-dihydro-4-iodo-3-[(3-methoxy-1H-pyrrol-2-yl)methylene]-2H-indol-2-one (147 mg, 0.4 mmol) using (Starting Material 2) $(Ph_3P)_2PdCl_2$ (40 mg) (Aldrich) and CuI (20 mg) (Aldrich) as catalyst in DMF (3 mL) and $Et_3N$ (3 mL) as solvent at 70 °C for 19 h, yielding *rac*-(Z)-1,3-dihydro-4-[3-hydroxy-3-(2-pyridinyl)-1-propynyl]-3-[(3-methoxy-1H-pyrrol-2-yl)methylene]-2H-indol-2-one. (Yield 84 mg, 56%).

**Example 38: Synthesis of *rac*-(Z)-1,3-Dihydro-4-[3-hydroxy-3-(2-thiophenyl)-1-propynyl]-3-[(3-methoxy-1H-pyrrol-2-yl)methylene]-2H-indol-2-one (KK)**

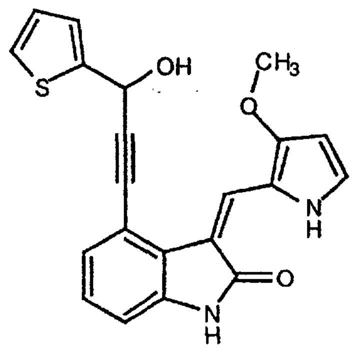

Using Method D above, 3-hydroxy-3-(2-thiophenyl)-1-propyne (102 mg, 0.74 mmol) (prepared by the addition of ethynylmagnesium chloride (Aldrich) to 2-thiophenecarboxaldehyde (Aldrich) according to Method A above) was coupled to (Z)-4-bromo-1,3-dihydro-3-[(3-methoxy-1H-pyrrol-2-yl)methylene]-2H-indol-2-one (126 mg, 0.39 mmol) (Starting Material 1) using $(Ph_3P)_2PdCl_2$ (35 mg) (Aldrich) and CuI (17 mg) (Aldrich) as catalyst in DMF (3 mL) and $Et_3N$ (3 mL) as solvent at 70 °C for 18 h, yielding *rac*-(Z)-1,3-dihydro-4-[3-hydroxy-3-(2-thiophenyl)-1-propynyl]-3-[(3-methoxy-1H-pyrrol-2-yl)methylene]-2H-indol-2-one. (Yield 105 mg, 71%).

**Example 39: Synthesis of 3-Hydroxy-3-[3-methoxy-4-[2-(4-morpholinyl)-ethoxy]-phenyl]-1-propyne (LL)**

3-Hydroxy-3-[3-methoxy-4-[2-(4-morpholinyl)-ethoxy]-phenyl]-1-propyne was prepared according to Method A above from 3-methoxy-4-(2-morpholin-4-yl-ethoxy)-benzaldehyde (0.60 g, 2.26 mmol) (*see* below) in THF (25 mL) and ethynylmagnesium chloride (5 mmol, 10 mL, 0.5M solution in tetrahydrofuran) (Aldrich). (Yield 502 mg, 76%).

**3-Methoxy-4-(2-morpholin-4-yl-ethoxy)-benzaldehyde** was prepared from N-(2-hydroxyethyl)morpholine (Aldrich) and vanillin (Aldrich) by method H above.

**Example 40: Synthesis of *rac*-(Z)-1,3-Dihydro-4-[3-hydroxy-3-[3-methoxy-4-[2-(4-morpholinyl)-ethoxy]-phenyl]-1-propynyl]-3-[(3-methoxy-1H-pyrrol-2-yl)methylene]-2H-indol-2-one (MM)**

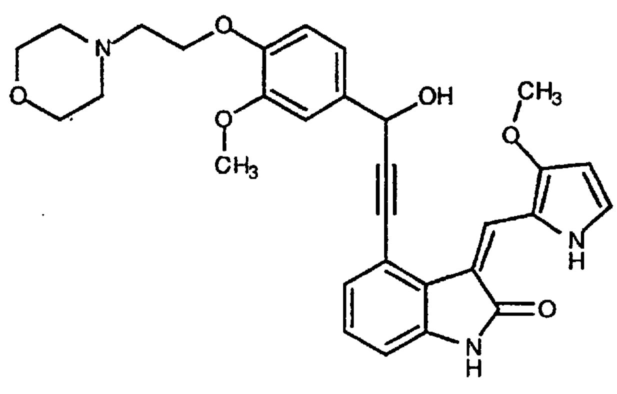

Using Method C above, 3-hydroxy-3-[3-methoxy-4-[2-(4-morpholinyl)-ethoxy]-phenyl]-1-propyne (610 mg, 2.09 mmol) (from Example 39 above) was coupled to (Z)-1,3-dihydro-4-iodo-3-[(3-methoxy-1H-pyrrol-2-yl)methylene]-2H-indol-2-one (200 mg, 0.55 mmol) (Starting Material 2) using $(Ph_3P)_2PdCl_2$ (50 mg) (Aldrich) and CuI (25 mg) (Aldrich) as catalyst in DMF (3 mL) and $Et_3N$ (3 mL) as solvent at 70 °C for 22 h, yielding *rac*-(Z)-1,3-dihydro-4-[3-hydroxy-3-[3-methoxy-4-[2-(4-morpholinyl)-ethoxy]-phenyl]-1-propynyl]-3-[(3-methoxy-1H-pyrrol-2-yl)methylene]-2H-indol-2-one. (Yield 154 mg, 53%).

**Example 41: Synthesis of *rac*-(Z)-1,3-Dihydro-4-[3-hydroxy-3-[3-methoxy-4-[2-(4-morpholinyl)-ethoxy]-phenyl]-1-propynyl]-3-[(3-methoxy-1H-pyrrol-2-yl)methylene]-2H-indol-2-one hydrochloride salt (NN)**

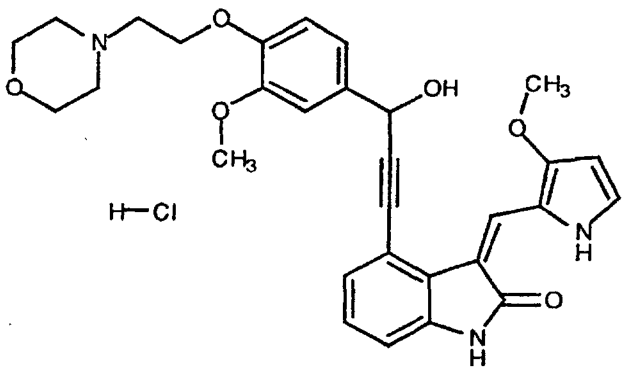

The hydrochloride salt of *rac*-(Z)-1,3-dihydro-4-[3-hydroxy-3-[3-methoxy-4-[2-(4-morpholinyl)-ethoxy]-phenyl]-1-propynyl]-3-[(3-methoxy-1H-pyrrol-2-yl) methylene]-2H-indol-2-one (Compound MM from Example 40) was prepared by dissolving Compound MM in ethyl acetate and bubbling hydrogen chloride gas through the solution. The resulting red precipitate was then filtered off and dried.

**Example 42: Synthesis of *rac*-(Z)-1,3-Dihydro-4-[3-hydroxy-3-(3-methoxy-2-thiophenyl)-1-propynyl]-3-[(3-methoxy-1H-pyrrol-2-yl)methylene]-2H-indol-2-one (OO)**

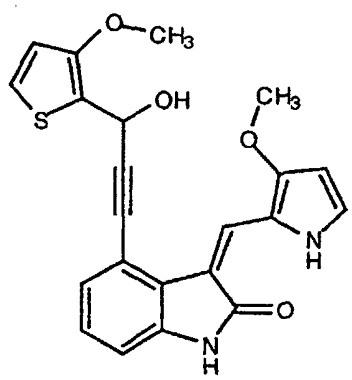

**Step A: 3-Hydroxy-3-(3-methoxy-2-thiophenyl)-1-propyne.**

3-Methoxy-2-thiophenecarboxadlehyde was prepared by adding *n*-butyllithium (10.56 mmol, 2.5M solution in hexane)(Aldrich), without cooling, to a solution of 3-methoxythiophene (1g, 8.8 mmol) (Aldrich) in dry diethyl ether (5 mL) over a period of 5 min. The mixture was gently heated at reflux for 2 h at which time the organolithium compound was transferred, via cannula, to a solution of DMF (23 mmol) in diethyl ether (5 mL) which was cooled in an ice bath. The reaction was stirred at room temperature for 14 h, at which time 1N HCl (10 mL) was added and the layers were separated. The aqueous layer was extracted with diethyl ether (3x25 mL), and the combined organic extracts were dried over magnesium sulfate, and concentrated to yield 3-methoxy-2-thiophenecarboxaldehyde as a pale, yellow solid. The 3-methoxy-2-thiophenecarboxaldehyde was then added to ethynylmagnesium chloride (Aldrich) according to Method A above to yield 3-hydroxy-3-(3-methoxy-2-thiophenyl)-1-propyne. (Yield 151 mg, 0.9 mmol).

**Step B:** Using Method C above, 3-hydroxy-3-(3-methoxy-2-thiophenyl)-1-propyne (from Step A above) was coupled to (Z)-1,3-dihydro-4-iodo-3-[(3-methoxy-1H-pyrrol-2-yl)methylene]-2H-indol-2-one (150 mg, 0.41 mmol) (Starting Material 2) using $(Ph_3P)_2PdCl_2$ (20 mg) (Aldrich) and CuI (10 mg) (Aldrich) as catalyst in DMF (2 mL) and $Et_3N$ (2 mL) as solvent at 70 °C for 20 h, yielding *rac*-(Z)-1,3-dihydro-4-[3-hydroxy-3-(3-methoxy-2-thiophenyl)-1-propynyl]-3-[(3-methoxy-1H-pyrrol-2-yl)methylene]-2H-indol-2-one. (Yield 98 mg, 59%: mp = 213 - 216 °C).

**Example 43: Synthesis of 3-Hydroxy-3-(2,4,5-trimethoxyphenyl)-1-propyne**

3-Hydroxy-3-(2,4,5-trimethoxyphenyl)-1-propyne was prepared according to Method A above from 2,4,5-trimethoxybenzaldehyde (0.784 g, 4 mmol) (Aldrich) in THF (20 mL) and ethynylmagnesium chloride (5 mmol, 10 mL, 0.5M solution in tetrahydrofuran) (Aldrich). (Yield 548 mg, 71%).

**Example 44: Synthesis of *rac*-(Z)-1,3-Dihydro-4-[3-hydroxy-3-(2,4,5-trimethoxyphenyl)-1-propynyl]-3-[(3-methoxy-1H-pyrrol-2-yl)methylene]-2H-indol-2-one (PP)**

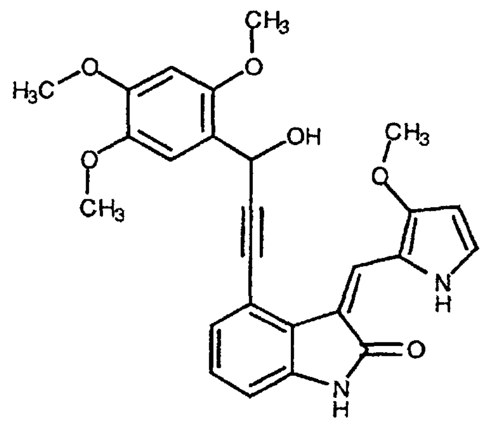

Using Method C above, 3-hydroxy-3-(2,4,5-trimethoxyphenyl)-1-propyne (150 mg, 0.67 mmol) (from Example 43 above) was coupled to (Z)-1,3-dihydro-4-iodo-3-[(3-methoxy-1H-pyrrol-2-yl)methylene]-2H-indol-2-one (150 mg, 0.41 mmol) (Starting Material 2) using $(Ph_3P)_2PdCl_2$ (35 mg) (Aldrich) and CuI (16 mg) (Aldrich) as catalyst in DMF (3 mL) and $Et_3N$ (3 mL) as solvent at 70 °C for 16 h, yielding *rac*-(Z)-1,3-dihydro-4-[3-hydroxy-3-(2,4,5-trimethoxyphenyl)-1-propynyl]-3-[(3-methoxy-1H-pyrrol-2-yl)methylene]-2H-indol-2-one. (Yield 101 mg, 53%).

**Example 45: Synthesis of (4-Formyl-2-methoxy-phenoxy)-acetic acid methyl ester**

(4-Formyl-2-methoxy-phenoxy)-acetic acid methyl ester was prepared by dissolving vanillin (4.1 mmol) (Aldrich) in dry THF (10 mL) and dry DMF (1 mL). Sodium hydride (109 mg, 4.5 mmol) was then added to the solution slowly and the resulting mixture was stirred at room temperature for 1 h at which time methyl bromoacetate (5 mmol) (Aldrich) was added dropwise. The reaction was stirred at room temperature for 14 h at which time water (10 mL) was added and the THF was evaporated *in vacuo.* The aqueous layer was then extracted with ethyl acetate (3x15 mL), and the combined organic layers were dried over magnesium sulfate and concentrated. The resulting (4-formyl-2-methoxy-phenoxy)-acetic acid methyl ester was purified via flash column chromatography ($SiO_2$, 230-400 mesh) with ethyl acetate/hexane.

**Example 46: Synthesis of [4-(1-Hydroxy-2-propynyl)-2-methoxy-phenoxy]-acetic acid methyl ester**

[4-(1-Hydroxy-2-propynyl)-2-methoxy-phenoxy]-acetic acid methyl ester was prepared according to Method A above (except the Grignard reagent was added at -78 °C) from (4-formyl-2-methoxy-phenoxy)-acetic acid methyl ester (0.645 g, 2.9 mmol) (from Example 45) in THF (30 mL) and ethynylmagnesium chloride (3.45 mmol, 7 mL, 0.5M solution in tetrahydrofuran) (Aldrich). (Yield 525 mg, 72%).

**Example 47: Synthesis of *rac*-(Z)-[4-[3-[2,3-Dihydro-3-[(3-methoxy-1H-pyrrol-2-yl)methylene]-2-oxo-1H-indol-4-yl]-1-hydroxy-2-propynyl]-2-methoxyphenoxy]acetic acid methyl ester (QQ)**

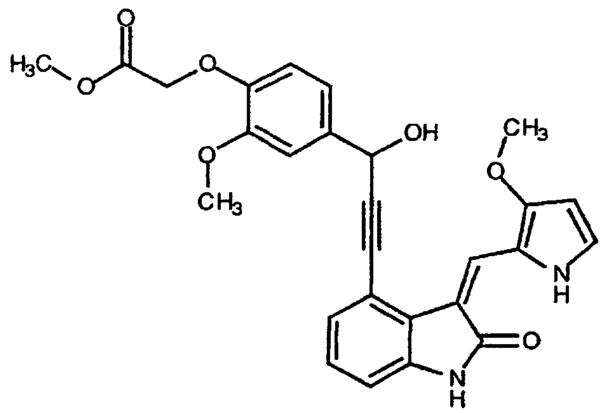

Using Method C above, [4-(1-hydroxy-2-propynyl)-2-methoxy-phenoxy]-acetic acid methyl ester (158 mg, 0.63 mmol) (from Example 46) was coupled to (Z)-1,3-dihydro-4-iodo-3-[(3-methoxy-1H-pyrrol-2-yl)methylene]-2H-indol-2-one (150 mg, 0.41 mmol) (Starting Material 2) using $(Ph_3P)_2PdCl_2$ (20 mg) (Aldrich) and CuI (10 mg) (Aldrich) as catalyst in DMF (2 mL) and $Et_3N$ (2 mL) as solvent at 70 °C for 16 h, yielding *rac*-(Z)-[4-[3-[2,3-dihydro-3-[(3-methoxy-1H-pyrrol-2-yl)methylene]-2-oxo-1H-indol-4-yl]-1-hydroxy-2-propynyl]-2-methoxyphenoxy]acetic acid methyl ester. (Yield 91 mg, 46%).

**Example 48: Synthesis of *rac*-(Z)-[4-[3-[2,3-Dihydro-3-[(3-methoxy-1H-pyrrol-2-yl)methylene]-2-oxo-1H-indol-4-yl]-1-hydroxy-2-propynyl]-2-methoxyphenoxy]acetic acid (RR)**

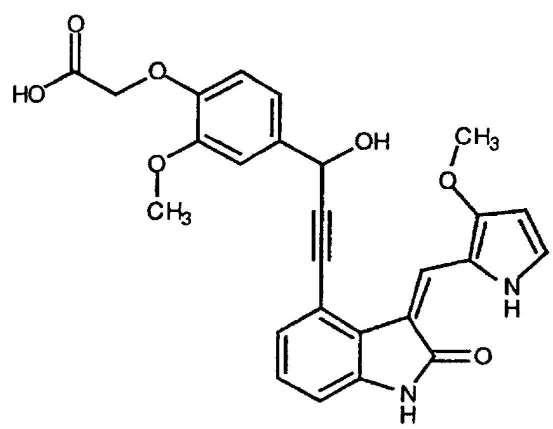

*rac*-(Z)-[4-[3-[2,3-Dihydro-3-[(3-methoxy-1H-pyrrol-2-yl)methylene]-2-oxo-1H-indol-4-yl]-1-hydroxy-2-propynyl]-2-methoxyphenoxy]acetic acid methyl ester(28 mg, 0.057 mmol) (from Example 47) was hydrolyzed with $LiOH \cdot H_2O$ (55 mg, 1.15 mmol) in THF (0.5 mL) and $H_2O$ (0.5 mL) at room temperature for 20 h according to Method F above to yield *rac*-(Z)-[4-[3-[2,3-dihydro-3-[(3-methoxy-1H-pyrrol-2-yl)methylene]-2-oxo-1H-indol-4-yl]-1-hydroxy-2-propynyl]-2-methoxyphenoxy]acetic acid. (Yield 22 mg, 81%).

**Example 49: Synthesis of 3-Hydroxy-3-(4-methoxy-1,3-benzodioxol-6-yl)-1-propyne**

3-Hydroxy-3-(4-methoxy-1,3-benzodioxol-6-yl)-1-propyne was prepared according to Method A above from 3-methoxy-4,5-methylenedioxybenzaldehyde (0.721 g, 4 mmol) (Lancaster) in THF (20 mL) and ethynylmagnesium chloride (5 mmol, 10 mL, 0.5M solution in tetrahydrofuran) (Aldrich). (Yield 629 mg, 76%).

**Example 50: Synthesis of *rac*-(Z)-4-[3-hydroxy-3-(4-methoxy-1,3-benzodioxol-6-yl)-1-propynyl]-1,3-dihydro-3-[(3-methoxy-1H-pyrrol-2-yl)methylene]-2H-indol-2-one (SS)**

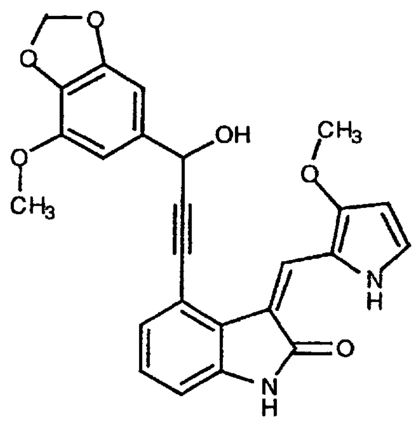

Using Method C above, 3-hydroxy-3-(4-methoxy-1,3-benzodioxol-6-yl)-1-propyne (153 mg, 0.74 mmol) (from Example 49 above) was coupled to (Z)-1,3-dihydro-4-iodo-3-[(3-methoxy-1H-pyrrol-2-yl)methylene]-2H-indol-2-one (147 mg, 0.4 mmol) (Starting Material 2) using $(Ph_3P)_2PdCl_2$ (40 mg) (Aldrich) and CuI (20 mg) (Aldrich) as catalyst in DMF (3 mL) and $Et_3N$ (3 mL) as solvent at 70 °C for 16 h, yielding *rac*-(Z)-4-[3-hydroxy-3-(4-methoxy-1,3-benzodioxol-6-yl)-1-propynyl]-1,3-dihydro-3-[(3-methoxy-1H-pyrrol-2-yl)methylene]-2H-indol-2-one. (Yield 98 mg, 55%).

**Example 51: Synthesis of 3-Hydroxy-3-[4-[2-(4-morpholinyl)-ethoxy]-phenyl]-1-propyne**

3-Hydroxy-3-[4-[2-(4-morpholinyl)-ethoxy]-phenyl]-1-propyne was prepared according to Method A above from 4-[2-(4-morpholinyl)-ethoxy]-benzaldehyde (0.630 g, 2.68 mmol) (*see* below) in THF (20 mL) and ethynylmagnesium chloride (3.0 mmol, 6 mL, 0.5M solution in tetrahydrofuran) (Aldrich). (Yield 492 mg, 70%).

**4-[2-(4-Morpholinyl)-ethoxy]-benzaldehyde** was prepared from N-(2-hydroxyethyl)morpholine (Aldrich) and vanillin (Aldrich) by method H above.

**Example 52: Synthesis of *rac*-(Z)-1,3-Dihydro-4-[3-hydroxy-3-[4-[2-(4-morpholinyl)-ethoxy]-phenyl]-1-propynyl]-3-[(3-methoxy-1H-pyrrol-2-yl)methylene]-2H-indol-2-one (TT)**

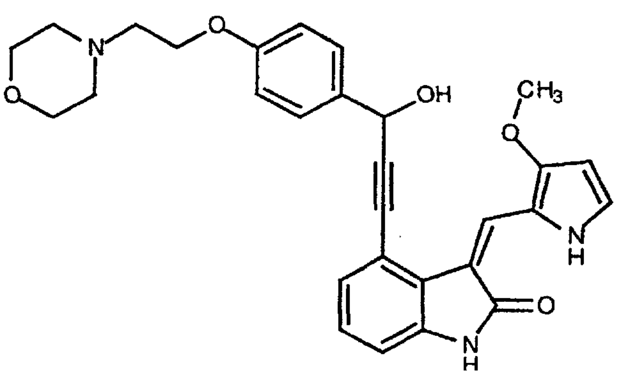

Using Method C above, 3-hydroxy-3-[4-[2-(4-morpholinyl)-ethoxy]-phenyl]-1-propyne (220 mg, 0.84 mmol) (from Example 51) was coupled to (Z)-1,3-dihydro-4-iodo-3-[(3-methoxy-1H-pyrrol-2-yl)methylene]-2H-indol-2-one (147 mg, 0.4 mmol) (Starting Material 2) using $(Ph_3P)_2PdCl_2$ (38 mg) (Aldrich) and CuI (17 mg) (Aldrich) as catalyst in DMF (3 mL) and $Et_3N$ (3 mL) as solvent at 70 °C for 20 h, yielding *rac*-(Z)-1,3-dihydro-4-[3-hydroxy-3-[4-[2-(4-morpholinyl)-ethoxy]-phenyl]-1-propynyl]-3-[(3-methoxy-1H-pyrrol-2-yl)methylene]-2H-indol-2-one. (Yield 164 mg, 82%).

**Example 53: Synthesis of 3-(4-Chloro-2-methylsulfanylmethoxy-phenyl)-3-hydroxy-1-propyne**

**Step A:** 4-Chlorosalicylic acid (34.51 g, 0.2 mol) (Aldrich) was suspended in a solution of methanol (100 mL) and concentrated sulfuric acid (8 mL). The mixture was heated at reflux for 17 h. After cooling to room temperature, the mixture was concentrated under reduced pressure. The resulting residue was dissolved in ether (400 mL) and washed successively with water (400 mL), saturated aqueous sodium bicarbonate (400 mL), and saturated aqueous sodium chloride (400 mL). The ether solution was then dried ($MgSO_4$), filtered, and concentrated. The resulting yellow oil was distilled to give **methyl 4-chlorosalicylate.** (Yield 32.89 g, 88%; b.p. 86 - 90 °C, 0.15 mm Hg).

**Step B:** Sodium hydride (11.80 g, 50% in oil) was washed with petroleum ether twice to remove oil. Hexamethylphosphorimide (HMPA) (100 mL) (Aldrich) was added to this washed sodium hydride under an argon atmosphere. The resulting mixture was stirred magnetically and cooled in an ice/water bath. Methyl 4-chlorosalicylate (37.32 g) (from Step A above) in HMPA (50 mL) was added dropwise and the mixture stirred for an additional 10 min. A solution of chloromethyl methyl sulfide (20 mL) (Aldrich) in HMPA (100 mL) was added and stirring at room temperature was continued for 24 h. The reaction mixture was then partitioned between toluene (3 X 1 L) and water (3 X 1 L). The toluene layers were combined, dried ($MgSO_4$), and concentrated under reduced pressure. The resulting oil was recrystallized from dichloromethane - hexane mixture to give the desired **methyl thiomethyl ether.** (Yield 33.92 g, 68.75%; mp 64 - 65.5 °C).

**Step C:** A solution of the methyl thiomethyl ether (10.0 g, 40.5 mmol) from Step B above in THF (50 mL) was added dropwise to a suspension of lithium aluminum hydride (LAH) (Aldrich) in THF (50 mL) under argon with magnetic stirring over 30 min. The suspension was then heated at reflux for 3 h. After cooling, the mixture was poured into 2N aqueous HCl (200 mL) and extracted with ether (2 X 200 mL). The ether layers were washed with saturated aqueous sodium chloride solution (200 mL), then combined, dried ($MgSO_4$) and concentrated. Residue was filtered through silica gel (100 g) and product eluted with dichloromethane (Fisher Scientific). The product was further purified by vacuum distillation to give the benzyl alcohol product as a colorless oil. (Yield 7.24 g, 82%), bp 156 - 160 °C, 0.07 mm Hg).

**Step D:** The benzyl alcohol (5.66 g, 25.9 mmol) oil of Step C above, in dichloromethane (70 mL), was mixed with pyridinium dichromate (20 g) (Aldrich) and stirred at 4 °C for 20 h. The mixture was then diluted with dichloromethane (35 mL) and hexane (35 mL) and filtered through silica gel (50 g) and eluted with dichloromethane. The first 500 mL of eluate were concentrated under reduced pressure and the residue recrystallized from hexane to give **4-chloro-2-methylsulfanylmethoxybenzaldehyde** as white needles. (Yield 4.99 g, 89%; mp 67 - 68 °C).

**Step E:** The desired **3-(4-Chloro-2-methylsulfanylmethoxy-phenyl)-3-hydroxy-1-propyne** was then prepared according to Method A above from the 4-chloro-2-methylsulfanylmethoxybenzaldehyde (0.870 g, 4 mmol) (from step D above) in THF (20 mL) and ethynylmagnesium chloride (5 mmol, 10 mL, 0.5M solution in tetrahydrofuran) (Aldrich). (Yield 802 mg, 83%).

**Example 54: Synthesis of *rac*-(Z)-4-[3-(4-Chloro-2-methylsulfanylmethoxyphenyl)-3-hydroxy-1-propynyl]-1,3-dihydro-3-[(3-methoxy-1H-pyrrol-2-yl)methylene]-2H-indol-2-one (UU)**

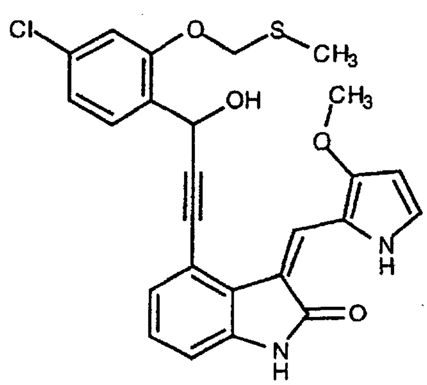

Using Method D above, 3-(4-chloro-2-methylsulfanylmethoxy-phenyl)-3-hydroxy-1 -propyne (158 mg, 0.65 mmol) (from Example 53 above) was coupled to (Z)-4-bromo-1,3-dihydro-3-[(3-methoxy-1H-pyrrol-2-yl)methylene]-2H-indol-2-one (152 mg, 0.48 mmol) (Starting Material 1) using $(Ph_3P)_2PdCl_2$ (40 mg) (Aldrich) and CuI (22 mg) (Aldrich) as catalyst in DMF (3 mL) and $Et_3N$ (3 mL) as solvent at 70 °C for 17 h, yielding *rac*-(Z)-4-[3-(4-chloro-2-methylsulfanylmethoxy-phenyl)-3-hydroxy-1-propynyl]-1,3-dihydro-3-[(3-methoxy-1H-pyrrol-2-yl)methylene]-2H-indol-2-one. (Yield 132 mg, 57%).

**Example 55: Synthesis of *rac*-(Z)-1,3-Dihydro-4-[3-(2-furanyl)-3-hydroxy-1-propynyl]-3-[(3-methoxy-1H-pyrrol-2-yl)methylene]-2H-indol-2-one (VV)**

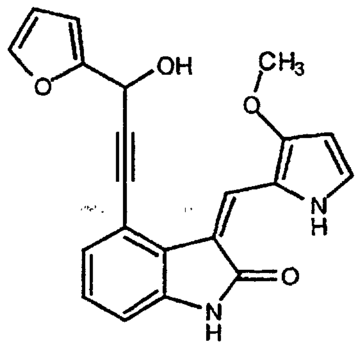

Using Method C above, 3-(2-furanyl)-3-hydroxy-1-propyne (148 mg, 1.24 mmol) (prepared by the addition of ethynylmagnesium chloride (Aldrich) to 2-furfural (Aldrich) according to Method A above) was coupled to (Z)-1,3-dihydro-4-iodo-3-[(3-methoxy-1H-pyrrol-2-yl)methylene]-2H-indol-2-one (152 mg, 0.42 mmol) (Starting Material 2) using $(Ph_3P)_2PdCl_2$ (34 mg) and CuI (16 mg) as catalyst in DMF (3 mL) and $Et_3N$ (3 mL) as solvent at 70 °C for 20 h, to yield *rac*-(Z)-1,3-dihydro-4-[3-(2-furanyl)-3-hydroxy-1-propynyl]-3-[(3-methoxy-1H-pyrrol-2-yl)methylene]-2H-indol-2-one. (Yield 88 mg, 58%).

**Example 56: Synthesis of *rac*-(Z)-4-[3-(3-Chlorophenyl)-3-hydroxy-1-propynyl]-1,3-dihydro-3-[(3-methoxy-1H-pyrrol-2-yl)methylene]-2H-indol-2-one (WW)**

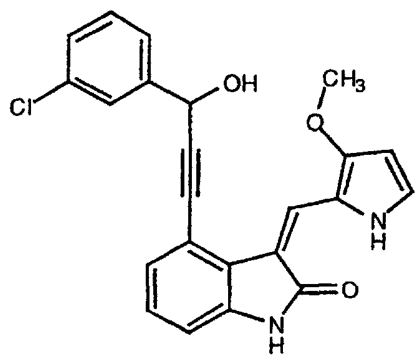

Using Method C above, 3-(3-chlorophenyl)-3-hydroxy-1-propyne (150 mg, 0.9 mmol) (prepared by the addition of ethynylmagnesium chloride to 3-chlorobenzaldehyde according to Method A above) was coupled to (Z)-1,3-dihydro-4-iodo-3-[(3-methoxy-1H-pyrrol-2-yl)methylene]-2H-indol-2-one (148 mg, 0.4 mmol) (Starting Material 2) using $(Ph_3P)_2PdCl_2$ (35 mg) and CuI (16 mg) as catalyst in DMF (3 mL) and $Et_3N$ (3 mL) as solvent at 70 °C for 17 h, yielding *rac*-(Z)-4-[3-(3-chlorophenyl)-3-hydroxy-1-propynyl]-1,3-dihydro-3-[(3-methoxy-1H-pyrrol-2-yl)methylene]-2H-indol-2-one. (Yield 132 mg, 81%).

**Example 57: Synthesis of [4-(1-Hydroxy-2-propnyl)-phenoxy]-acetic acid 1,1-dimethyl ethyl ester**

**Step A:** Sodium hydride (109 mg, 4.5 mmol) was added slowly to a solution of 4-hydroxybenzaldehyde (4.1 mmol) (Aldrich) in dry THF (10 mL) and dry DMF (1 mL), and the resulting mixture was stirred at room temperature for 1 h at which time 1,1-dimethylethyl bromoacetate (5 mmol) (Aldrich) was added dropwise. The reaction was stirred at room temperature for 14 h. Water (10 mL) was then added and the THF was evaporated *in vacuo*. The aqueous layer

was extracted with ethyl acetate (3x15 mL), and the combined organic layers were dried over magnesium sulfate and concentrated. The resulting product, **(4-formyl-phenoxy)- acetic acid 1,1-dimethyl ethyl ester,** was purified via flash column chromatography ($SiO_2$, 230-400 mesh) with ethyl acetate/hexane.

<u>**Step B:**</u> **[4-(1-Hydroxy-2-propynyl)-phenoxy]-acetic acid 1,1-dimethyl ethyl ester** was then prepared according to Method A above (except the Grignard reagent was added at 0°C) from **(4-formyl-phenoxy)-acetic acid 1,1-dimethyl ethyl ester** (1.0 g, 4.2 mmol) (from Step A above) in THF (20 mL) and ethynylmagnesium chloride (5.1 mmol, 10.2 mL, 0.5M solution in tetrahydrofuran) (Aldrich). (Yield 817 mg, 74%).

<u>**Example 58:**</u> **Synthesis of *rac*-(Z)-[4-[3-[2,3-dihydro-3-[(3-methoxy-1H-pyrrol-2-yl)methylene]-2-oxo-1H-indol-4-yl]-1-hydroxy-2-propynyl]phenoxy]acetic acid 1,1-dimethylethyl ester (XX)**

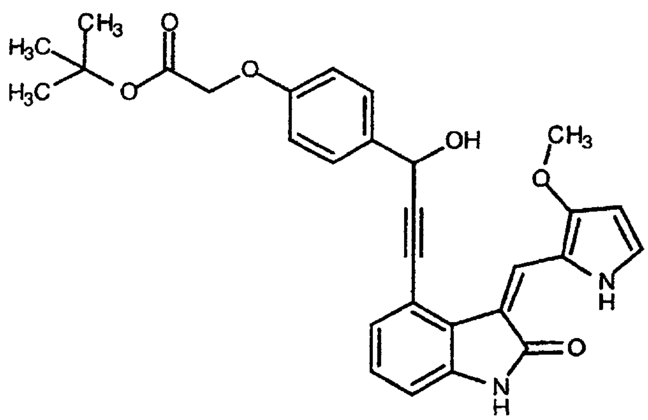

Using Method C above, [4-(1-hydroxy-2-propynyl)-phenoxy]-acetic acid 1,1-dimethyl ehtyl ester (129 mg, 0.49 mmol) (from Example 57 above) was coupled to (Z)-1,3-dihydro-4-iodo-3-[(3-methoxy-1H-pyrrol-2-yl)methylene]-2H-indol-2-one (150 mg, 0.41 mmol) (Starting Material 2) using $(Ph_3P)_2PdCl_2$ (20 mg) (Aldrich) and CuI (10 mg) (Aldrich) as catalyst in DMF (2 mL) and $Et_3N$ (2 mL) as solvent at 70 °C for 20 h, yielding *rac*-(Z)-[4-[3-[2,3-dihydro-3-[(3-methoxy-1H-pyrrol-2-yl)methylene]-2-oxo-1H-indol-4-yl]-1-hydroxy-2-propynyl]phenoxy]acetic acid 1,1-dimethylethyl ester. (Yield 106 mg, 52%: m.p. 173 - 175 °C).

<u>**Example 59**</u>**: Synthesis of *rac*-(Z)-[4-[3-[2,3-dihydro-3-[(3-methoxy-1H-pyrrol-2-yl)methylene]-2-oxo-1H-indol-4-yl]-1-hydroxy-2-propynyl]phenoxy]acetic acid(YY)**

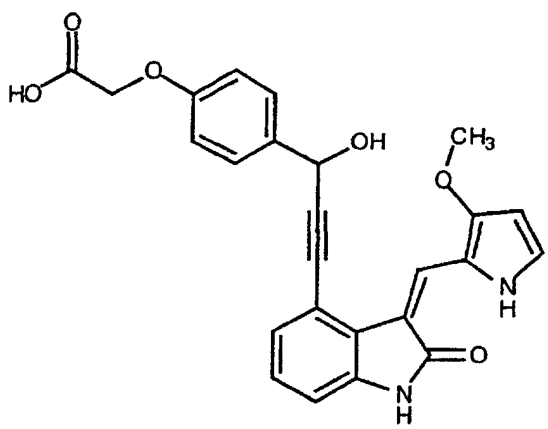

Using Method F above, *rac*-(Z)-[4-[3-[2,3-dihydro-3-[(3-methoxy-1H-pyrrol-2-yl)methylene]-2-oxo-1H-indol-4-yl]-1-hydroxy-2-propynyl]phenoxy]acetic acid 1,1-dimethylethyl ester (from Example 58 above) (30 mg, 0.061 mmol) was hydrolyzed with LiOH•$H_2O$ (58 mg, 1.22 mmol) in THF (0.5 mL) and $H_2O$ (0.5 mL) for 12 h, yielding *rac*-(Z)

-[4-[3-[2,3-dihydro-3-[(3-methoxy-1H-pyrrol-2-yl)methylene]-2-oxo-1H-indol-4-yl]-1-hydroxy-2-propynyl]phenoxy]acetic acid. (Yield 24 mg, 89%).

**Example 60: Synthesis of *rac*-(Z)-1,3-Dihydro-4-[3-hydroxy-3-(3-nitrophenyl)-1-propynyl]-3-[(3-methoxy-1H-pyrrol-2-yl)methylene]-2H-indol-2-one (ZZ)**

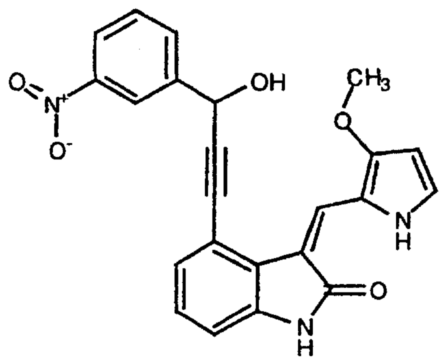

Using Method C above, 1-(3-nitro-phenyl)-2-propyn-1-ol (126 mg, 0.71 mmol) (prepared by the addition of ethynylmagnesium chloride (Aldrich) to 3-nitrobenzaldehyde (Aldrich) according to Method A above) was coupled to (Z)-1,3-dihydro-4-iodo-3-[(3-methoxy-1H-pyrrol-2-yl)methylene]-2H-indol-2-one (134 mg, 0.37 mmol) (Starting Material 2) using $(Ph_3P)_2PdCl_2$ (35 mg) (Aldrich) and CuI (20 mg) (Aldrich) as catalyst in DMF (3 mL) and $Et_3N$ (3 mL) as solvent at 70 °C for 20 h, yielding *rac*-(Z)-1,3-dihydro-4-[3-hydroxy-3-(3-nitrophenyl)-1-propynyl]-3-[(3-methoxy-1H-pyrrol-2-yl)methylene]-2H-indol-2-one. (Yield 84 mg, 52%).

**Example 61: Synthesis of *rac*-(Z)-4-[3-(3-Aminophenyl)-3-hydroxy-1-propynyl]-1,3-dihydro-3-[(3-methoxy-1H-pyrrol-2-yl)methylene]-2H-indol-2-one (AAA)**

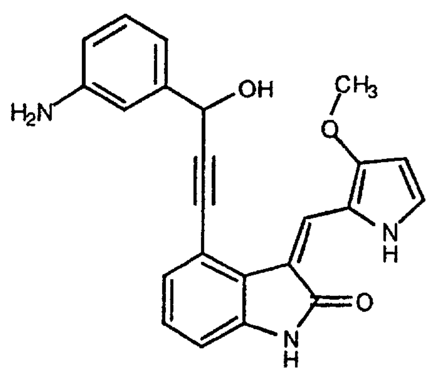

*rac*-(Z)-1,3-Dihydro-4-[3-hydroxy-3-(3-nitrophenyl)-1-propynyl]-3-[(3-methoxy-1H-pyrrol-2-yl)methylene]-2H-indol-2-one (25 mg, 0.068 mmol) (from Example 60) was added to 10% $H_2O$ in methanol (2 mL) and to this mixture was added zinc dust (35 mg, 0.53 mmol) and ammonium chloride (10 mg, 0.19 mmol). The reaction was heated at reflux for 3 h, at which time the reaction was cooled and the solid was filtered off. The solids were washed extensively with ethyl acetate, and the ethyl acetate and methanol were evaporated *in vacuo*. The resulting precipitate was filtered off, dried and recrystallized from ethyl acetate/hexane to give *rac*-(Z)-4-[3-(3-aminophenyl)-3-hydroxy-1-propynyl]-1,3-dihydro-3-[(3-methoxy-1H-pyrrol-2-yl)methylene]-2H-indol-2-one. (Yield 19 mg, 73%).

**Example 62: Synthesis of 3-(4-Acetamidophenyl)-3-hydroxy-1-propyne**

**Step A: 1-(4-Nitro-phenyl)-2-propyn-1-ol** (4.89 mmol) (prepared by the addition of ethynylmagnesium chloride (Aldrich) to 4-nitrobenzaldehyde (Aldrich) according to method A above) was added to 10% $H_2O$ in methanol (150 mL) and to this mixture was added zinc dust (44.01 mmol) and ammonium chloride (10.67 mmol). The reaction was heated at reflux for 3 h, at which time the reaction was cooled and the solid was filtered off. The solids were washed extensively with ethyl acetate, and the ethyl acetate and methanol were evaporated *in vacuo.* The resulting precipitate was filtered off, dried and recrystallized from ethyl acetate/hexane to yield **1-(4-amino-phenyl)-2-propyn-1-ol.**

__Step B__: 1-(4-Amino-phenyl)-2-propyn-1-ol (3.0 mmol) (from Step A above) was dissolved in dry THF (20 mL) and DMF (1 mL). Acetic anhydride (4.2 mmol) was added dropwise followed by triethylamine (3.0 mmol). The reaction was stirred at room temperature for 2 h, after which time water (30 mL) was added, and the THF was evaporated *in vacuo.* The aqueous layer was extracted with ethyl acetate (3x50 mL), and the combined organic layers were dried over magnesium sulfate and concentrated. The resulting product, **3-(4-acetamidophenyl)-3-hydroxy-1-propyne,** was purified via flash column chromatography ($SiO_2$, 230-400 mesh) with ethyl acetate/hexane.

**__Example 63__: Synthesis of *rac*-(Z)-4-[3-(4-Acetamidophenyl)-3-hydroxy-1-propynyl]-1,3-dihydro-3-[(3-methoxy-1H-pyrrol-2-yl)methylene]-2H-indol-2-one (BBB)**

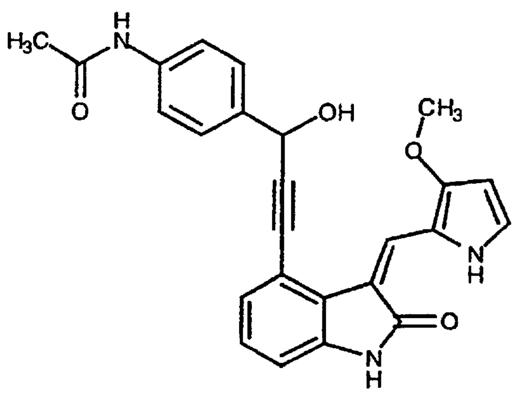

Using Method C above, 3-(4-acetamidophenyl)-3-hydroxy-1-propyne (111 mg, 0.59 mmol) (from Example 62 above) was coupled to (Z)-1,3-dihydro-4-iodo-3-[(3-methoxy-1H-pyrrol-2-yl)methylene]-2H-indol-2-one (110 mg, 0.3 mmol) (Starting Material 2) using $(Ph_3P)_2PdCl_2$ (25 mg) (Aldrich) and CuI (10 mg) (Aldrich) as catalyst in DMF (3 mL) and $Et_3N$ (3 mL) as solvent at 70 °C for 16 h, yielding *rac*-(Z)-4-[3-(4-acetamidophenyl)-3-hydroxy-1-propynyl]-1,3-dihydro-3-[(3-methoxy-1H-pyrrol-2-yl)methylene]-2H-indol-2-one. (Yield 57 mg, 44%).

**__Example 64__: Synthesis of (Z)-1,3-Dihydro-3-[(3-methoxy-1H-pyrrol-2-yl)methylene]-4-[(3-pyridinyl)ethynyl]-2H-indol-2-one (CCC)**

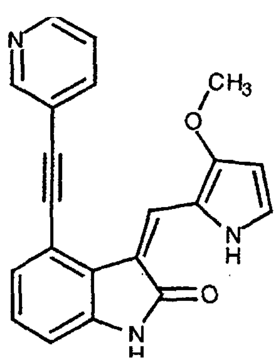

Using Method D above, 3-ethynyl pyridine (60.6 mg, 0.59 mmol) (*see* below) was coupled with (Z)-4-bromo-1,3-dihydro-3-[(3-methoxy-1H-pyrrol-2-yl)methylene]-2H-indol-2-one (75 mg, 0.23 mmol) (Starting Material 1) using $(Ph_3P)_4Pd$ (13.3 mg) (Aldrich) and CuI (3 mg) (Aldrich) as catalyst in DMF (4 mL) and $Et_3N$ (4 mL) as solvent at 100

°C for 18 h, to yield (Z)-1,3-dihydro-3-[(3-methoxy-1H-pyrrol-2-yl)methylene]-4-[(3-pyridinyl)ethynyl]-2H-indol-2-one. (Yield 52 mg, 66%).

**3-Ethynyl pyridine** was prepared by coupling 2-methyl-3-butyne-2-ol to 3-bromopyridine using $(Ph_3P)_2PdCl_2$ (Aldrich) and CuI (Aldrich) as catalyst in DMF and $Et_3N$ as solvent according to method D above.

**<u>Example 65:</u> Synthesis of (Z)-1,3-Dihydro-3-[(3-methoxy-1H-pyrrol-2-yl)methylene]-4-[(2-pyridinyl)ethynyl]-2H-indol-2-one (DDD)**

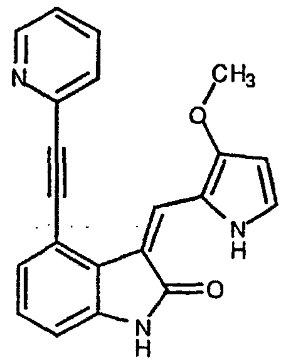

Using Method J above, 2-bromopyridine (44.9 mg, 0.28 mmol) (Aldrich) was coupled with (Z)-1,3-dihydro-4-ethynyl-3-[(3-methoxy-1H-pyrrol-2-yl)methylene]-2H-indol-2-one (Starting Material 5) (50 mg, 0.19 mmol) using $DPPFPdCl_2$ (7.7 mg) (Aldrich) and CuI (2 mg) (Aldrich) as catalyst in DMF (3 mL) and $Et_3N$ (3 mL) as solvent and heating at reflux for 2 days, yielding (Z)-1,3-dihydro-3-[(3-methoxy-1H-pyrrol-2-yl)methylene]-4-[(2-pyridinyl)ethynyl]-2H-indol-2-one. (Yield 30 mg, 47%).

**<u>Example 66</u>: Synthesis of (Z)-1,3-Dihydro-3-[(3-methoxy-1 H-pyrrol-2-yl)methylene]-4-[(4-pyridinyl)ethynyl]-2H-indol-2-one (EEE)**

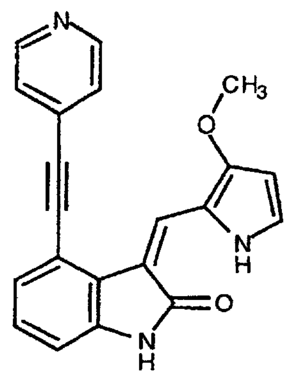

Using Method J above, 4-bromopyridine hydrochloride (110 mg, 0.57 mmol) (Aldrich) was coupled with (Z)-1,3-dihydro-4-ethynyl-3-[(3-methoxy-1H-pyrrol-2-yl)methylene]-2H-indol-2-one (Starting Material 5) (100 mg, 0.38 mmol) using $DPPFPdCl_2$ (15.4 mg) (Aldrich) and CuI (4 mg) (Aldrich) as catalyst in DMF (5 mL) and $Et_3N$ (5 mL) as solvent and heating at reflux for 1 day, yielding (Z)-1,3-dihydro-3-[(3-methoxy-1H-pyrrol-2-yl)methylene]-4-[(4-pyridinyl)ethynyl]-2H-indol-2-one. (Yield 70 mg, 54%).

**<u>Example 67</u>: Synthesis of *rac*-(Z)-1,3-Dihydro-4-(3-hydroxy-3-phenyl-1-propynyl)-3-[(3-methoxy-1H-pyrrol-2-yl) methylene]-2H-indol-2-one (FFF)**

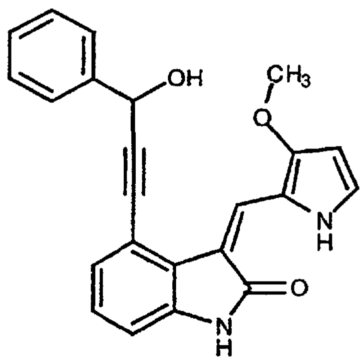

Using Method D above, 3-hydroxy-3-phenyl-1-propyne (0.1 g, 0.78 mmol) (prepared by the addition of ethynylmagnesium chloride (Aldrich) to benzaldehyde (Aldrich) through Method A above) was coupled to (Z)-4-bromo-1,3-dihydro-3-[(3-methoxy-1H-pyrrol-2-yl)methylene]-2H-indol-2-one (0.1 g, 0.31 mmol) (Starting Material 1) using $DPPFPdCl_2$ (12.6 mg) (Aldrich) and CuI (3 mg) (Aldrich) as catalyst in DMF (5 mL) and $Et_3N$ (5 mL) as solvent at 85 °C for 18 h, yielding *rac*-(Z)-1,3-dihydro-4-(3-hydroxy-3-phenyl-1-propynyl)-3-[(3-methoxy-1H-pyrrol-2-yl)methylene]-2H-indol-2-one. (Yield 42 mg, 38%).

**<u>Example 68</u>: Synthesis of (Z)-1,3-Dihydro-3-[(3-methoxy-1H-pyrrol-2-yl)methylene]-5-nitro-4-[(3-pyridinyl) ethynyl]-2H-indol-2-one (GGG)**

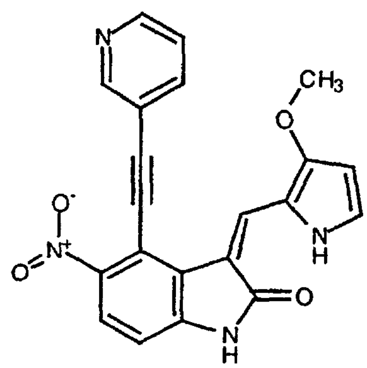

Using Method D above, 3-ethynyl pyridine (0.14 g, 1.38 mmol) (*see* Example 64) was coupled with (Z)-4-bromo-1,3-dihydro-3-[(3-methoxy-1H-pyrrol-2-yl)methylene]-5-nitro-2H-indol-2-one (0.2 g, 0.55 mmol) (Starting Material 3) using $(Ph_3P)_4Pd$ (31.8 mg) (Aldrich) and CuI (5.3 mg) (Aldrich) as catalyst in DMF (6 mL) and $Et_3N$ (6 mL) as solvent and at 85 °C for 18 h, yielding (Z)-1,3-dihydro-3-[(3-methoxy-1H-pyrrol-2-yl)methylene]-5-nitro-4-[(3-pyridinyl)ethynyl]-2H-indol-2-one. (Yield 0.16 g, 71%).

**Example 69: Synthesis of (Z)-5-Amino-1,3-dihydro-3-[(3-methoxy-1H-pyrrol-2-yl)methylene]-4-[(3-pyridinyl) ethynyl]-2H-indol-2-one (HHH)**

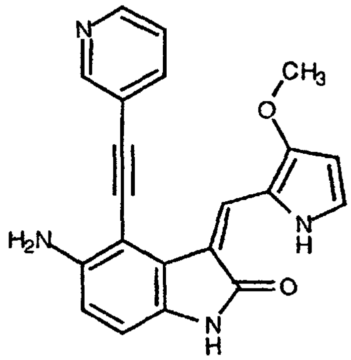

Using Method L above, (Z)-1,3-dihydro-3-[(3-methoxy-1H-pyrrol-2-yl) methylene]-5-nitro-4-[(3-pyridinyl)ethynyl]-2H-indol-2-one (0.1 g, 0.26 mmol) (from Example 68 above) was reduced with Zn (0.15 g, 2.33 mmol) and $NH_4Cl$ (30.6 mg, 0.57 mmol) in 10% water in methanol (10 mL) with a trace of DMF and heating at 90 °C for 5 h, to yield (Z)-5-amino-1,3-dihydro-3-[(3-methoxy-1H-pyrrol-2-yl)methylene]-4-[(3-pyridinyl)ethynyl]-2H-indol-2-one. (Yield 28 mg, 30%).

**Example 70: Synthesis of (Z)-N-[2,3-Dihydro-3-[(3-methoxy-1H-pyrrol-2-yl)methylene]-2-oxo-4-[(3-pyridinyl) ethynyl]-1H-indol-5-yl]-2-thiopheneacetamide (III)**

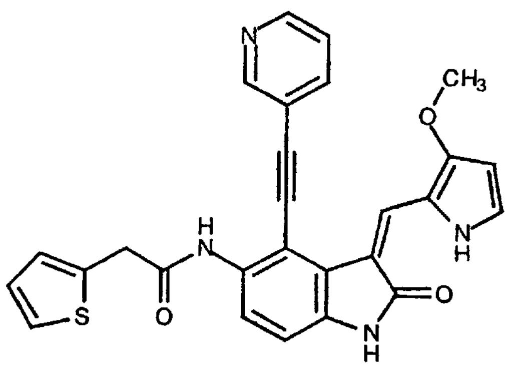

Using Method M above, (Z)-5-amino-1,3-dihydro-3-[(3-methoxy-1H-pyrrol-2-yl)methylene]-4-[(3-pyridinyl) ethynyl]-2H-indol-2-one (18 mg, 0.051 mmol) (from Example 69) was coupled with 2-thiopheneacetyl chloride (16.2 mg, 0.10 mmol) (Aldrich) in THF (2 mL) and saturated aqueous sodium bicarbonate (1 mL) at room temperature for 1 h, to yield (Z)-N-[2,3-dihydro-3-[(3-methoxy-1H-pyrrol-2-yl)methylene]-2-oxo-4-[(3-pyridinyl)ethynyl]-1H-indol-5-yl]-2-thiopheneacetamide. (Yield 7.7 mg, 32%).

**Example 71: Synthesis of (Z)-1,3-Dihydro-4-iodo-3-[(1H-pyrrol-2-yl)methylene]-2H-indol-2-one (JJJ)**

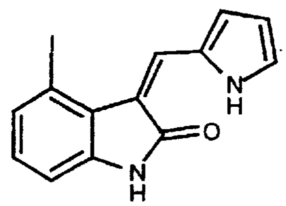

A mixture of 4-iodooxindole (404.1 mg, 1.56 mmol) *(see* Fukuyama, *supra)* and pyrrole-2-carboxaldehyde (163.2 mg, 1.72 mmol) (Aldrich) in 2-propanol (6.2 mL) was treated with 2 drops of piperidne. The reaction mixture was heated at reflux for 24 h and then allowed to cool to 23°C, at which time, the reaction mixture was filtered. The solid was washed several times with cold distilled water and then allowed to air dry to provide pure (Z)-1,3-dihydro-4-iodo-3-[(1H-pyrrol-2-yl)methylene]-2H-indol-2-one (341.8 mg, 65%) as a yellow solid which was used without further purification:

**Example 72: Synthesis of 4-[(E)-2-(2-Chlorophenyl)-ethenyl]-1,3-dihydro-(Z)-3-[(1H-pyrrol-2-yl)methylene]-2H-indol-2-one (KKK)**

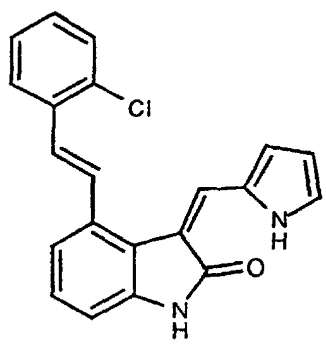

A solution of (Z)-1,3-dihydro-4-iodo-3-[(1H-pyrrol-2-yl)methylene]-2H-indol-2-one (39 mg, 0.116 mmol) (from Example 71 above), triethylamine (65 µL, 0.464 mmol) , tri-o-tolylphosphine (7 mg, 0.023 mmol) (Aldrich), palladium (II) acetate (2 mg, 0.009 mmol) (Aldrich), and 2-chlorostyrene (24 mg, 0.173 mmol) (Aldrich) in 3 mL of dry N,N-dimethylformamide was heated at 85 °C under a nitrogen atmosphere for 20 h. The reaction mixture was allowed to cool to room temperature and then directly purified by flash chromatography (Merck Silica gel 60, 230-400 mesh, 5% ethyl acetate-benzene elution) to yield pure 4-[(E)-2-(2-chlorophenyl)-ethenyl]-1,3-dihydro-(Z)-3-[(1H-pyrrol-2-yl)methylene]-2H-indol-2-one as a yellow solid. (Yield 27 mg, 67%; mp=257-258°C).

**Example 73: Synthesis of 1,3-Dihydro-(Z)-3-[(1H-pyrrol-2yl)methylene]-[(E)-2-phenylethenyl]-2H-indol-2-one (LLL)**

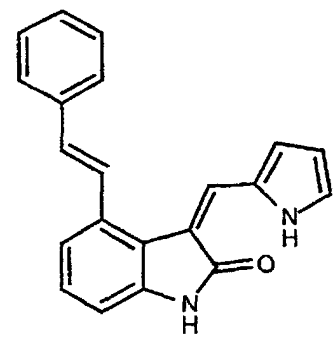

To a stirred solution of (Z)-1,3-dihydro-4-iodo-3-[(1H-pyrrol-2-yl)methylene]-2H-indol-2-one (from Example 71 above) (500 mg, 1.49 mmol) in DMF (8 mL) and TEA (3 mL) was added styrene (0.34 mL, 2.98 mmol) (Aldrich), tri-*o*-tolylphosphine (361 mg, 1.19 mmol) (Aldrich) and $Pd(OAc)_2$ (67 mg, 0.30 mmol) (Aldrich). The reaction mixture was stirred at 85 °C overnight in a pressure tube. The solvent was removed *in vacuo*, and the residue was purified by silica gel chromatography (Hex:EtOAc 5:1) to 1,3-dihydro-(Z)-3-[(1H-pyrrol-2yl)methylene]-[(E)-2-phenylethenyl]-2H-indol-2-one. (Yield 371 mg, 80%).

**Example 74: Synthesis of 1,3-Dihydro-(Z)-3-[(3-methoxy-1H-pyrrol-2yl)methylene]-[(E)-2-phenylethenyl]-2H-indol-2-one (MMM)**

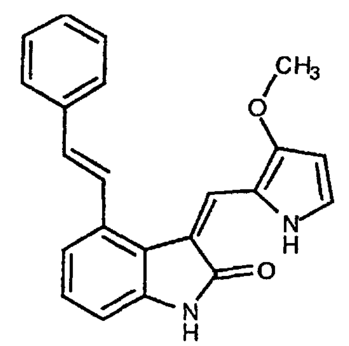

To a stirred solution of (Z)-1,3-dihydro-4-iodo-3-[(3-methoxy-1H-pyrrol-2-yl)methylene]-2H-indol-2-one (500 mg, 1.49 mmol) (Starting Material 2) in DMF (8 mL) and TEA (3 mL) was added styrene (0.33 mL, 2.92 mmol) (Aldrich), tri-*o*-tolylphosphine (361 mg, 1.19 mmol) (Aldrich) and $Pd(OAc)_2$ (67 mg, 0.30 mmol) (Aldrich). The reaction mixture was stirred at 85 °C overnight in a pressure tube. The solvent was removed *in vacuo,* and the residue was purified by silica gel chromatography (Hex:EtOAc = 5:1) to provide 1,3-dihydro-(Z)-3-[(3-methoxy-1H-pyrrol-2yl)methylene]-[(E)-2-phenylethenyl]-2H-indol-2-one as a yellow solid. (Yield 407 mg, 80%).

**Example 75: Synthesis of 1,3-Dihydro-4-[(E)-2-(4-methoxyphenyl)-ethenyl]-(Z)-3-[(1H-pyrrol-2-yl)methlene]-2H-indol-2-one (NNN)**

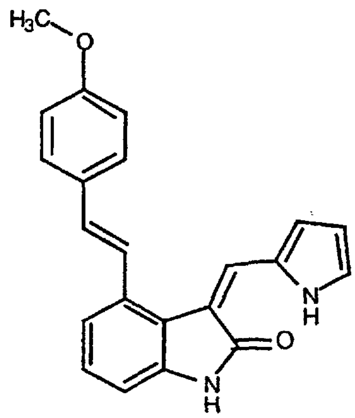

**A. *p*-Methoxy-styrene**

To a stirred solution of *n*-butyllithium (4 mL, 2,5 M solution in Hexane, 10 mmol) (Aldrich) in ether (30 mL) was added methyltriphenylphosphonium bromide (3.57 g, 10 mmol) (Aldrich) over a period of 5 min. The reaction mixture was stirred for 4 h at room temperature. To the resulting orange solution was added 4-methoxybenzaldehyde (1.34 mL, 10 mmol) (Aldrich) dropwise. The solution became colorless, and a white precipitate separated. The mixture was then heated to reflux and immediately allowed to cool to room temperature. The precipitate was removed by filtration. The precipitate was washed with ether and the combined ethereal filtrates were washed with water until neutral and then dried over anhydrous $MgSO_4$. The solvent was removed, and the residue was used in the next reaction without further purification.

B. **1,3-Dihydro-4-[(E)-2-(4-methoxyphenyl)-ethenyl]-(Z)-3-[(1H-pyrrol-2-yl)methlene]-2H-indol-2-one (NNN)**

To a stirred solution of (Z)-1,3-dihydro-4-iodo-3-[(1H-pyrrol-2-yl)methylene]-2H-indol-2-one (from Example 71 above) (100 mg, 0.29 mmol) in DMF (3 mL) and TEA (2 mL) was added *p*-methoxy-styrene (79 mg, 0.58 mmol) (from Step A above), tri-*o*-tolylphosphine (107 mg, 0.35 mmol) (Aldrich) and $Pd(OAc)_2$ (20 mg, 0.089 mmol) (Aldrich). The reaction mixture was stirred at 85°C overnight in a pressure tube. The solvent was removed *in vacuo*, and the residue was purified by silica gel chromatography (Hex:EtOAc = 5:1) to provide 1,3-dihydro-4-[(E)-2-(4-methoxyphenyl)-ethenyl]-(Z)-3-[(1H-pyrrol-2-yl)methlene]-2H-indol-2-one as a yellow solid. (Yield 72 mg, 73%).

**Example 76: Synthesis of 1,3-Dihydro-(Z)-3-[(3-methoxy-1H-pyrrol-2-yl)methylene]-4-[(E)-2-(4-methoxyphenyl)-ethenyl]-2H-indol-2-one (OOO)**

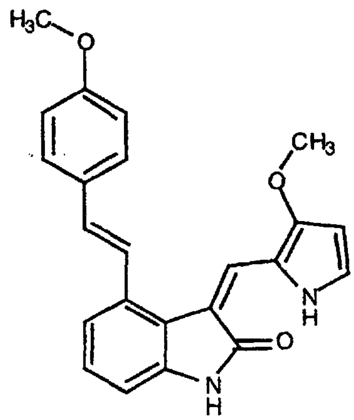

To a stirred solution of (Z)-1,3-dihydro-4-iodo-3-[(3-methoxy-1H-pyrrol-2-yl)methylene]-2H-indol-2-one (100 mg, 0.29 mmol) (Starting Material 2) in DMF (3 mL) and TEA (2 mL) was added *p*-methoxy-styrene (79 mg, 0.58 mmol) (from Example 75A, above), tri-*o*-tolylphosphine (107 mg, 0.35 mmol) (Aldrich) and $Pd(OAc)_2$ (20 mg, 0.089 mmol) (Aldrich). The reaction mixture was stirred at 85 °C overnight in a pressure tube. The solvent was removed *in vacuo*, and the residue was purified by silica gel chromatography (Hex:EtOAc = 5:1) to provide 1,3-dihydro-(Z)-3-[(3-methoxy-1H-pyrrol-2-yl)methylene]-4-[(E)-2-(4-methoxyphenyl)-ethenyl]-2H-indol-2-one as a yellow solid. (Yield 78 mg, 74%).

**Example 77: Synthesis of 4-Ethenyl-benzoic acid methyl ester**

To a stirred solution of *n*-butyllithium (4 mL, 2,5 M solution in hexane, 10 mmol) (Aldrich) in ether (30 mL) was added methyltriphenylphosphonium bromide (3.57 g, 10 mmol) (Aldrich) over a period of 5 min. The reaction mixture was stirred for 4 h at room temperature. To the resulting orange solution was added methyl 4-formylbenzoate (1.54 mL, 10 mmol) dropwise. The solution became colorless, and a white precipitate separated. The mixture was then heated to reflux and immediately allowed to cool to room temperature. The precipitate was removed by filtration. The resulting precipitate was washed with ether, and the combined ethereal filtrates were washed with water until neutral and then dried over anhydrous $MgSO_4$. The solvent was removed and the residue, 4-ethenyl-benzoic acid methyl ester, was used in the next reaction without further purification.

**Example 78: Synthesis of 4-[(E)-2-[2,3-Dihydro-(Z)-3-[(3-methoxy-1H-pyrrol-2-yl)methylene]-2-oxo-1H-indol-4-yl]ethenyl]benzoic acid methyl ester (PPP)**

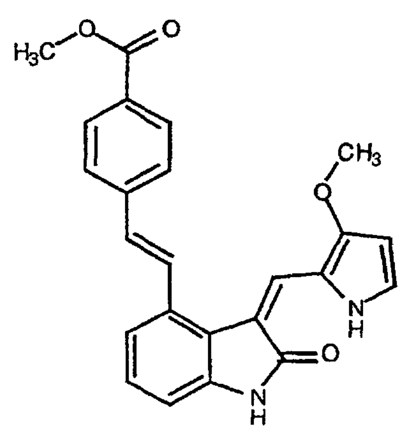

To a stirred solution of (Z)-1,3-dihydro-4-iodo-3-[(3-methoxy-1H-pyrrol-2-yl)methylene]-2H-indol-2-one(100 mg, 0.29 mmol) (Starting Material 2) in DMF (3 mL) and TEA (2 mL) was added 4-ethenyl -benzoic acid methyl ester (0.11 mL, 0.58 mmol)(from Example 77 above), tri-*o*-tolylphosphine (107 mg, 0.35 mmol) (Aldrich) and $Pd(OAc)_2$ (20 mg, 0.089 mmol) (Aldrich). The reaction mixture was stirred at 85 °C overnight in a pressure tube. The solvent was removed *in vacuo*, and the residue was purified by silica gel chromatography (Hex:EtOAc = 5:1) to provide 4-[(E)-2-[2,3-dihydro-(Z)-3-[(3-methoxy-1H-pyrrol-2-yl)methylene]-2-oxo-1H-indol-4-yl]ethenyl]benzoic acid methyl ester as a yellow solid. (Yield 77 mg, 66%)

**Example 79: Synthesis of 1,2-Dimethoxy-4-ethenyl-benzene**

To a stirred solution of *n*-butyllithium (4 mL, 2,5 M solution in hexane, 10 mmol) (Aldrich) in ether (30 mL) was added methyltriphenylphosphonium bromide (3.57 g, 10 mmol) (Aldrich) over a period of 5 min. The reaction mixture was stirred for 4 h at room temperature. To the resulting orange solution was added 3,4-dimethoxybenzaldehyde (1.82g, 11 mmol) (Aldrich) dropwise. The solution became colorless, and a white precipitate separated. The mixture was then heated to reflux and immediately allowed to cool to room temperature. The precipitate was removed by filtration. The precipitate was washed with ether and the combined ethereal filtrates were washed with water until neutral and then dried over anhydrous $MgSO_4$. The solvent was removed and the residue, 1,2-dimethoxy-4-ethenyl -benzene, was used in the next reaction without further purification.

**Example 80: Synthesis of 1,3-Dihydro-4-[(E)-2-(3,4-dimethoxyphenyl)-ethenyl]-(Z)-3-[(3-methoxy-1H-pyrrol-2-yl)methylene]-2H-indol-2-one (QQQ)**

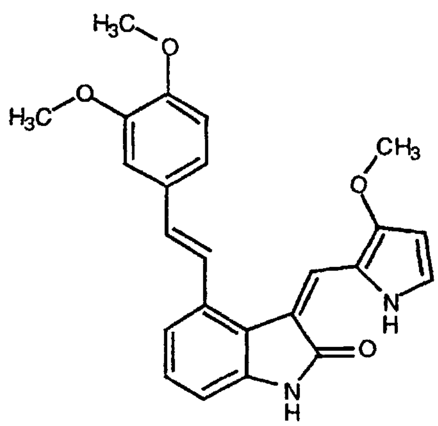

To a stirred solution of (Z)-1,3-dihydro-4-iodo-3-[(3-methoxy-1H-pyrrol-2-yl)methylene]-2H-indol-2-one (100 mg, 0.29 mmol) (Starting Material 2) in DMF (3 mL) and TEA (2 mL) was added 1,2-dimethoxy-4-vinyl-benzene (0.089 mg, 0.58 mmol) (from Example 79), tri-*o*-tolylphosphine (107 mg, 0.35 mmol) (Aldrich) and $Pd(OAc)_2$ (20 mg, 0.089 mmol) (Aldrich). The reaction mixture was stirred at 85°C overnight in a pressure tube. The solvent was removed *in vacuo,* and the residue was purified by silica gel chromatography (Hex:EtOAc = 5:1) to provide 1,3-dihydro-4-[(E)-2-(3,4-dimethoxyphenyl)-ethenyl]-(Z)-3-[(3-methoxy-1H-pyrrol-2-yl)methylene]-2H-indol-2-one as a yellow solid. (Yield 78 mg, 67%).

**Example 81: Synthesis of (Z)-1,3-Dihydro-3-[(1H-pyrrol-2-yl)methylene]-5-(trimethylsilyl)ethynyl-2H-indol-2-one (RRR)**

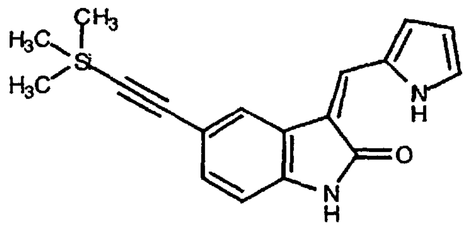

(Trimethylsilyl)acetylene (1.36g, 13.8 mmol) (Aldrich) and (Z)-5-bromo-1,3-dihydro-3-[(1H-pyrrol-2-yl)methylene]-2H-indol-2-one (2.0 g, 6.9 mmol) (Starting Material 7) were dissolved in 30 mL DMF and 40 mL triethylamine. The solution was degassed for 30 minutes by bubbling argon through the solution. At this time CuI (130 mg, 0.68 mmol) (Aldrich) and $(Ph_3P)_2PdCl_2$ (400 mg, 0.57 mmol) (Aldrich) were added, and the reaction was heated, under argon, at 70 °C for 22 hours. Water (20 mL) was then added and the precipitate was filtered off and dried. The product was purified via flash column chromatography ($SiO_2$, 230-400 mesh) with 1% $MeOH/CHCl_3$ to give (Z)-1,3-dihydro-3-[(1H-pyrrol-2-yl)methylene]-5-(trimethylsilyl)ethynyl-2H-indol-2-one as a yellow powder. (Yield 1.07 g, 51%).

**Example 82: Synthesis of (Z)-1,3-Dihydro-5-ethynyl-3-[(1H-pyrrol-2-yl)methylene]-2H-indol-2-one (SSS)**

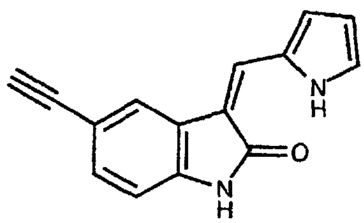

To a solution of (Z)-1,3-dihydro-3-[(1H-pyrrol-2-yl)methylene]-5-(trimethylsilyl)ethynyl-2H-indol-2-one (940 mg, 3.1 mmol) (from Example 81 above) in 50 mL ethanol and 15 mL tetrahydrofuran (Fisher Scientific) was added dropwise a solution of silver nitrate (1.17 g, 6.89 mmol) in 5 mL water and 15 mL ethanol, during which time a precipitate formed. The mixture was stirred at room temperature for 45 minutes, after which a solution of potassium cyanide (2.18 g, 33.47 mmol) in 8 mL of water was added and the precipitated dissolved. To the solution was then added 50 mL of a saturated aqueous sodium bicarbonate solution followed by 500 mL of water. The product was then filtered off and dried to give (Z)-1,3-dihydro-5-ethynyl-3-[(1H-pyrrol-2-yl)methylene]-2H-indol-2-one (yield 700 mg, 96%), which was recrystallized from EtOAc/Hex to yield 540 mg of the product as yellow crystals.

**Example 83: Synthesis of (Z)-1,3-Dihydro-5-(4-hydroxyphenyl)ethynyl-3-[(1H-pyrrol-2-yl)methylene]-2H-indol-2-one (TTT)**

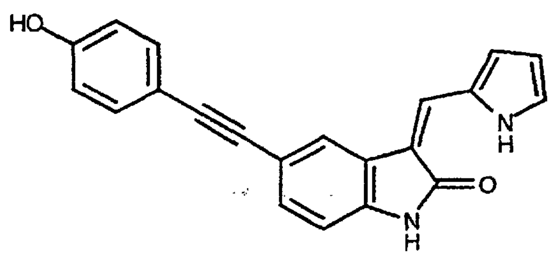

A solution of (Z)-1,3-dihydro-5-ethynyl-3-[(1H-pyrrol-2-yl)methylene]-2H-indol-2-one (100 mg, 0.43 mmol) (from Example 82) and 4-iodophenol (104 mg, 0.47 mmol) (Aldrich) in N,N-dimethylformamide (2 mL) and triethylamine (2 mL) was degassed by bubbling argon through the solution for 15 minutes. Copper(I) iodide (8 mg, 0.042 mmol) (Aldrich) and $(Ph_3P)_2PdCl_2$ (25 mg, 0.021 mmol) (Aldrich) were added, and the reaction was heated at 70 °C for 16 hours. Water (15 mL) was then added and the precipitate was filtered off and dried. The product was purified via flash column chromatography ($SiO_2$, 230-400 mesh) with ethyl acetate/hexane to give (Z)-1,3-dihydro-5-(4-hydroxyphenyl) ethynyl-3-[(1H-pyrrol-2-yl)methylene]-2H-indol-2-one as a yellow powder. (Yield 121 mg, 86%).

**Example 84: Synthesis of (Z)-1,3-Dihydro-5-(3-nitrophenyl)ethynyl-3-[(1H-pyrrol-2-yl)methylene]-2H-indol-2-one (UUU)**

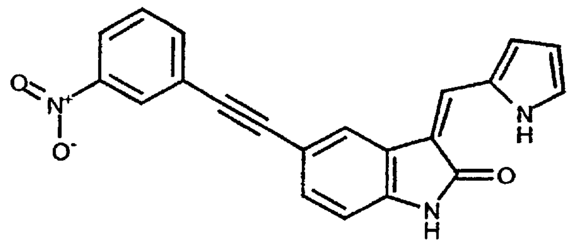

This compound was prepared in a manner analogous to the preparation of (Z)-1,3-dihydro-5-(4-hydroxyphenyl)ethynyl-3-[(1H-pyrrol-2-yl)methylene]-2H-indol-2-one (Compound TTT in Example 83 above). In this example, (Z)-1,3-dihydro-5-ethynyl-3-[(1H-pyrrol-2-yl)methylene]-2H-indol-2-one (150 mg, 0.64 mmol) (from Example 82) was coupled with 1-iodo-3-nitrobenzene (175 mg, 0.70 mmol) (Aldrich) using CuI (13 mg, 0.068 mmol) and $(Ph_3P)_2PdCl_2$ (22 mg, 0.031 mmol) (Aldrich) as catalyst in 3 mL DMF and 3 mL triethylamine at 70 °C for 13 hours to give (Z)-1,3-dihydro-5-(3-nitrophenyl)ethynyl-3-[(1H-pyrrol-2-yl)methylene]-2H-indol-2-one as a red powder. (Yield 148 mg, 65%)

**Example 85: Synthesis of (Z)-1,3-Dihydro-5-phenylethynyl-3-[(1H-pyrrol-2-yl)methylene]-2H-indol-2-one (VVV)**

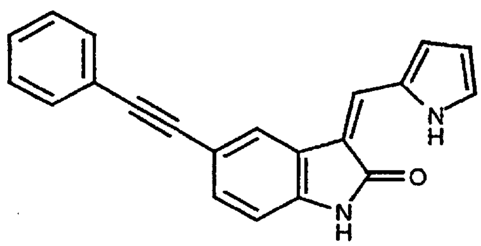

This compound was prepared in a manner analogous to the preparation of (Z)-1,3-dihydro-5-(4-hydroxyphenyl)ethynyl-3-[(1H-pyrrol-2-yl)methylene]-2H-indol-2-one in Example 83 above. In this example, (Z)-1,3-dihydro-5-ethynyl-3-[(1H-pyrrol-2-yl)methylene]-2H-indol-2-one (90 mg, 0.38 mmol) (from Example 82) was coupled with iodobenzene (219 mg, 1.07 mmol) (Aldrich) using CuI (8 mg, 0.042 mmol) (Aldrich) and $(Ph_3P)_2PdCl_2$ (14 mg, 0.020 mmol) (Aldrich) as catalyst in 1 mL DMF and 2 mL triethylamine at 70 °C for 15 hours to give (Z)-1,3-dihydro-5-phenylethynyl-3-[(1H-pyrrol-2-yl)methylene]-2H-indol-2-one as a yellow powder. (Yield 92 mg, 78%)

**Example 86: Synthesis of (Z)-1,3-Dihydro-5-(3-hydroxyphenyl)ethynyl-3-[(1H-pyrrol-2-yl)methylene]-2H-indol-2-one (WWW)**

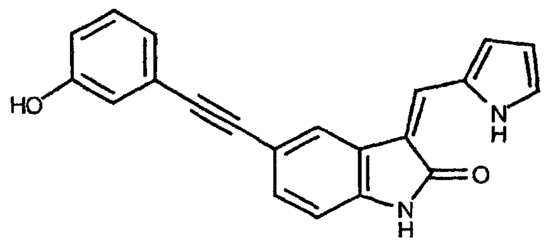

This compound was also prepared in a manner to analogous to the preparation of (Z)-1,3-dihydro-5-(4-hydroxyphenyl)ethynyl-3-[(1H-pyrrol-2-yl)methylene]-2H-indol-2-one in Example 83 above. In this example, (Z)-1,3-dihydro-5-ethynyl-3-[(1H-pyrrol-2-yl)methylene]-2H-indol-2-one (100 mg, 0.43 mmol) (from Example 82) was coupled with 3-iodophenol (110 mg, 0.50 mmol) (Aldrich) using CuI (8 mg, 0.042 mmol) (Aldrich) and $(Ph_3P)_2PdCl_2$ (15 mg, 0.021 mmol) (Aldrich) as catalyst in 1 mL DMF and 3 mL triethylamine at 70 °C for 13 hours to give (Z)-1,3-dihydro-5-(3-hydroxyphenyl)ethynyl-3-[(1H-pyrrol-2-yl) methylene]-2H-indol-2-one as a yellow powder. (Yield 100 mg, 71%).

**Example 87: Synthesis of (Z)-1,3-Dihydro-5-(2-nitrophenyl)ethynyl-3-[(1H-pyrrol-2-yl)methylene]-2H-indol-2-one (XXX)**

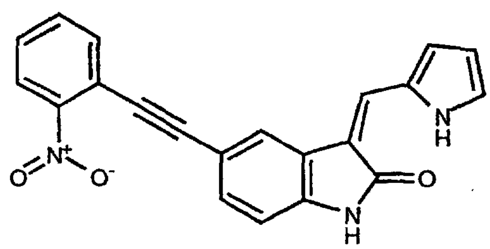

This compound was also prepared in a manner analogous to the preparation of (Z)-1,3-dihydro-5-(4-hydroxyphenyl)ethynyl-3-[(1H-pyrrol-2-yl)methylene]-2H-indol-2-one in Exampe 83 above. In this example, (Z)-1,3-dihydro-5-ethynyl-3-[(1H-pyrrol-2-yl)methylene]-2H-indol-2-one (150 mg, 0.64 mmol) (from Example 82) was coupled with 1-bromo-2-nitrobenzene (150 mg, 0.74 mmol) (Aldrich) using CuI (13 mg, 0.068 mmol) (Aldrich) and $(Ph_3P)_2PdCl_2$ (22 mg, 0.031 mmol) (Aldrich) as catalyst in 2 mL DMF and 4 mL triethylamine at 70 °C for 15 hours to give (Z)-1,3-dihydro-5-(2-nitrophenyl)ethynyl-3-[(1H-pyrrol-2-yl)methylene]-2H-indol-2-one as a red powder. (Yield 115 mg, 51%).

**<u>Example 88:</u> Synthesis (Z)-5-[3-[2,3-dihydro-2-oxo-3-(1H-pyrrol-2-ylmethylene)-1H-indol-5-yl]-2-propynyl]-6 (5H)-phenanthridinone (YYY)**

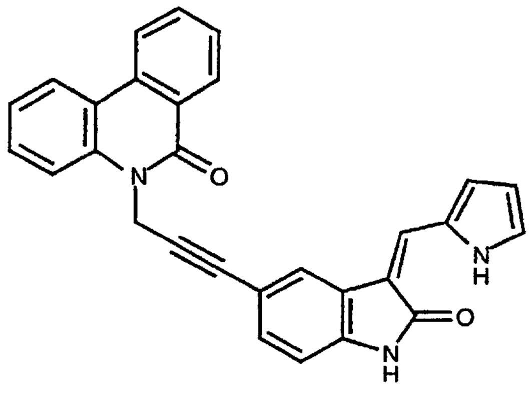

A solution of 5-(2-propynyl)-6(5H)-phenanthridinone (42 mg, 0.18 mmol) (prepared according to Walser et al., *J. Med. Chem.,* 34(3), 1209-1221 (1991)) and (Z)-1,3-dihydro-5-iodo-3-[(1H-pyrrol-2-yl)methylene]-2H-indol-2-one (2.0 g, 0.12 mmol) (Starting Material 8) were dissolved in 3 mL DMF and 0.04 mL triethylamine. The solution was degassed for 30 minutes by bubbling argon through the solution. At this time, CuI (1 mg) (Aldrich), triphenylphosphine (5 mg) (Aldrich), and paltadium(II) acetate (2 mg) (Aldrich) were added, and the reaction was stirred, under argon, at 27 °C for 37 hours. Water (20 mL) was then added and the precipitate was filtered off and dried. The product was purified via flash column chromatography ($SiO_2$, 230-400 mesh) with 5% MeOH in $CHCl_3$ to give (Z)-5-[3-[2,3-dihydro-2-oxo-3-(1H-pyrrol-2-ylmethylene)-1H-indol-5-yl]-2-propynyl]-6(5H)-phenanthridinone as a yellow powder. (Yield 13 mg, 25%)

**<u>Example 89:</u> Synthesis of (Z)-1,3-Dihydro-5-(4-nitrophenyl)ethynyl-3-[(1H-pyrrol-2-yl)methylene]-2H-indol-2-one (ZZZ)**

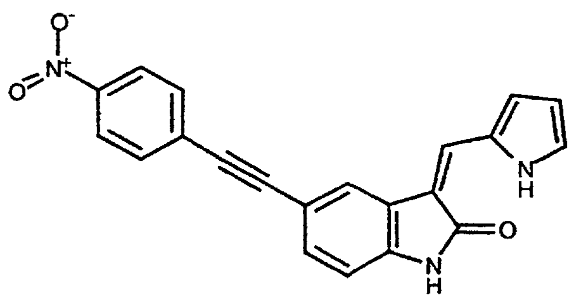

A solution of (Z)-1,3-dihydro-5-ethynyl-3-[(1H-pyrrol-2-yl)methylene]-2H-indol-2-one (150 mg, 0.64 mmol) (from Example 82) and 1-iodo-4-nitrobenzene (175 mg, 0.70 mmol) (Aldrich) in N,N-dimethylformamide (3 mL) (Fisher Scientific) and triethylamine (3 mL) was degassed by bubbling argon through the solution for 15 minutes. Copper(I) iodide (13 mg, 0.068 mmol) (Aldrich) and $(Ph_3P)_2PdCl_2$ (22 mg, 0.031 mmol) (Aldrich) were added, and the reaction mixture was heated at 70 °C for 13 hours. Water (15 mL) was then added and the precipitate was filtered off and dried. The product was purified via flash column chromatography ($SiO_2$, 230-400 mesh) with ethyl acetate/hexane to give (Z)-1,3-dihydro-5-(4-nitrophenyl)ethynyl-3-[(1H-pyrrol-2-yl)methylene]-2H-indol-2-one as a red powder. (Yield 111 mg, 49%).

**Example 90: Synthesis of (Z)-5-(4-Aminophenyl)ethynyl-1,3-dihydro-3-[(1H-pyrrol-2-yl)methylene]-2H-indol-2-one (AAAA)**

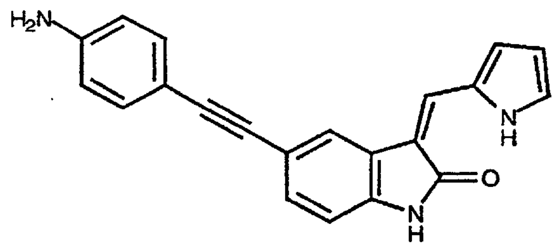

To a solution of (Z)-1,3-dihydro-5-(4-nitrophenyl)ethynyl-3-[(1H-pyrrol-2-yl)methylene]-2H-indol-2-one (45 mg, 0.13 mmol) (from Example 89) in 1 mL of a 10% water in methanol solution and 0.5 mL THF was added zinc dust (145 mg, 2.21 0mmol) followed by ammonium chloride (25 mg, 0.47 mmol). The reaction was heated at gentle reflux for 4 hours, at which time the reaction mixture was filtered through a pad of Celite® (Fisher Scientific) and rinsed thoroughly with ethyl acetate. The resulting solution was diluted with 5 mL water and the product was extracted with ethyl acetate. The combined organic extracts were dried over magnesium sulfate and concentrated. The resulting powder was recrystallized from EtOAc/hex to give (Z)-5-(4-aminophenyl)ethynyl-1,3-dihydro-3-[(1H-pyrrol-2-yl)methylene]-2H-indol-2-one as a red powder. (Yield 24 mg, 56%).

**Example 91: Synthesis of (Z)-5-Bromo-1,3-dihydro-3-[(3-methoxy-1H-pyrrol-2-yl)methylene]-2H-indol-2-one (BBBB)**

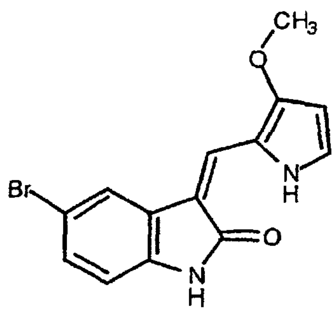

A mixture of 5-bromo-1,3-dihydro-2H-indol-2-one (0.94 g, 4.4 mmol) (Starting Material 6) and 3-methoxy-2-pyrrole-carboxyaldehyde (0.5 g, 4.0 mmol) *(see* Bellany et al., *supra*) in 1% piperidine in 2-propanol (10 mL) was heated at 65 °C for 16 h. Hot water (10 mL) was added. On cooling, the crystallized product was filtered off, washed with aqueous 2-propanol and dried. (Yield 1.13 g, 89%).

**Example 92: Synthesis of (Z)-1,3-Dihydro-3-[(3-methoxy-1H-pyrrol-2-yl)methylene]-5-(trimethylsilyl)ethynyl-2H-indol-2-one (CCCC)**

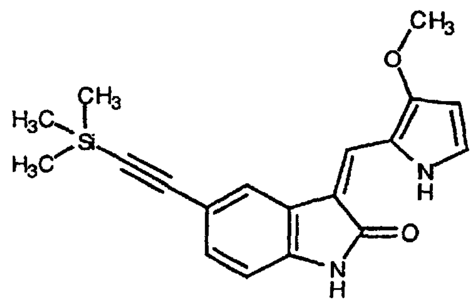

This compound was prepared in a manner analogous to the preparation of (Z)-1,3-dihydro-3-[(1H-pyrrol-2-yl) methylene]-5-(trimethylsilyl)-ethynyl-2H-indol-2-one in Example 81 above. In this example, (Z)-5-bromo-1,3-dihydro-3-[(3-methoxy-1H-pyrrol-2-yl)methylene]-2H-indol-2-one (740 mg, 2.32 mmol) (from Example 91) was coupled with (trimethylsilyl)acetylene (640 mg, 6.52 mmol) (Aldrich) using CuI (40 mg, 0.21 mmol) (Aldrich) and $(Ph_3P)_2PdCl_2$ (90 mg, 0.13 mmol) (Aldrich) as catalyst in 10 mL DMF and 10 mL triethylamine at 70 °C for 22 hours to give (Z)-1,3-dihydro-3-[(3-methoxy-1H-pyrrol-2-yl)methylene]-5-(trimethylsilyl)ethynyl-2H-indol-2-one. (Yield 410 mg, 52%).

**Example 93: Synthesis of (Z)-1,3-Dihydro-5-ethynyl-3-[(3-methoxy-1H-pyrrol-2-yl)methylene]-2H-indol-2-one (DDDD)**

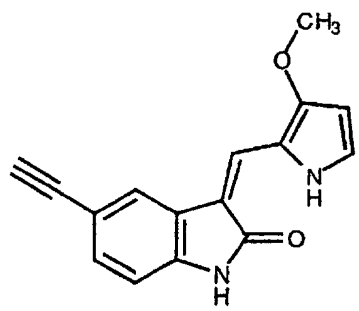

This compound was also prepared in a manner analogous to the preparation of (Z)-1,3-dihydro-5-ethynyl-3-[(1H-pyrrol-2-yl)methylene]-2H-indol-2-one in Example 82 above. In this example, (Z)-1,3-dihydro-3-[(3-methoxy-1H-pyrrol-2-yl)methylene]-5-(trimethylsilyl)ethynyl-2H-indol-2-one (100 mg, 0.3 mmol) (Example 92) in 5 mL ethanol and 1.5 mL tetrahydrofuran was treated with silver nitrate (0.112 g, 0.6 mmol) in 1.5 mL ethanol and 0.5 mL water, followed by potassium cyanide (218 mg, 3.35 mmol) in 1 mL water to give 70 mg (89%) of (Z)-1,3-dihydro-5-ethynyl-3-[(3-methoxy-1H-pyrrol-2-yl)methylene]-2H-indol-2-one (61 mg after recrystallization).

**Example 94: Synthesis of (Z)-1,3-Dihydro-5-(3-pyridinyl)ethynyl-3-[(1H-pyrrol-2-yl)methylene]-2H-indol-2-one (EEEE)**

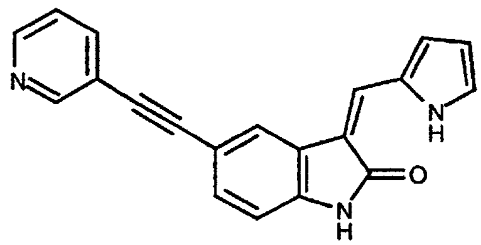

This compound was also prepared in a manner analogous to the preparation of (Z)-1,3-dihydro-5-(4-hydroxyphenyl)ethynyl-3-[(1H-pyrrol-2-yl)methylene]-2H-indol-2-one in Example 83 above. In this example, (Z)-1,3-dihydro-5-ethynyl-3-[(1H-pyrrol-2-yl)methylene]-2H-indol-2-one (47 mg, 0.21 mmol) (Example 82) was coupled with 3-bromopyridine (33 mg, 0.30 mmol) (Aldrich) using CuI (8 mg, 0.042 mmol) (Aldrich) and $(Ph_3P)_2PdCl_2$ (15 mg, 0.021 mmol) (Aldrich) as catalyst in 1 mL DMF and 1 mL triethylamine at 70 °C for 15 hours to give (Z)-1,3-dihydro-5-(3-pyridinyl)ethynyl-3-[(1H-pyrrol-2-yl)methylene]-2H-indol-2-one as a red powder. (Yield 30 mg, 47%).

**Example 95: Synthesis of (Z)-1,3-Dihydro-5-(2-pyridinyl)ethynyl-3-[(1H-pyrrol-2-yl)methylene]-2H-indol-2-one (FFFF)**

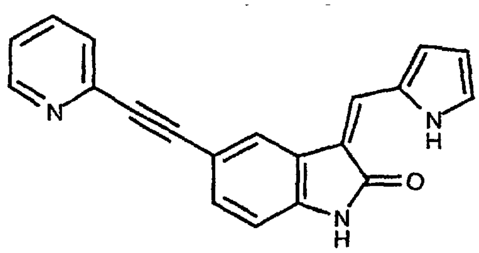

This compound was also prepared in a manner analogous to the preparation of (Z)-1,3-dihydro-5-(4-hydroxyphenyl)ethynyl-3-[(1H-pyrrol-2-yl)methylene]-2H-indol-2-one in Example 83 above. In this example, (Z)-1,3-dihydro-5-ethynyl-3-[(1H-pyrrol-2-yl)methylene]-2H-indol-2-one (40 mg, 0.17 mmol) (Example 82) was coupled with 2-bromopyridine (25 mg, 0.17 mmol) (Aldrich) using CuI (8 mg, 0.042 mmol) (Aldrich) and $(Ph_3P)_2PdCl_2$ (14 mg, 0.020 mmol) (Aldrich) as catalyst in 1 mL DMF and 1 mL triethylamine at 70 °C for 14 hours to give (Z)-1,3-dihydro-5-(2-pyridinyl)ethynyl-3-[(1H-pyrrol-2-yl)methylene]-2H-indol-2-one as a red powder. (Yield 42 mg, 80%).

**Example 96: Synthesis of (Z)-1,3-Dihydro-5-(4-hydroxyphenyl)ethynyl-3-[(3-methoxy-1H-pyrrol-2-yl)-methylene]-2H-indol-2-one (GGGG)**

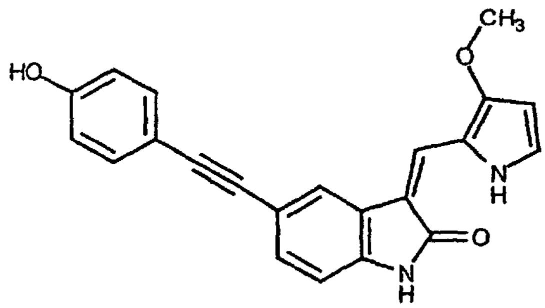

This compound was also prepared in a manner analogous to the preparation of (Z)-1,3-dihydro-5-(4-hydroxyphenyl)ethynyl-3-[(1H-pyrrol-2-yl)methylene]-2H-indol-2-one in Example 83 above. In this example, (Z)-1,3-dihydro-5-ethynyl-3-[(3-methoxy-1H-pyrrol-2-yl)methylene]-2H-indol-2-one (75 mg, 0.28 mmol) (from Example 93) was coupled with 4-iodophenol (75 mg, 0.34 mmol) (Aldrich) using CuI (8 mg, 0.042 mmol) (Aldrich) and $(Ph_3P)_2PdCl_2$ (14 mg, 0.020 mmol) (Aldrich) as catalyst in 1 mL DMF and 1 mL triethylamine at 70 °C for 14 hours to give (Z)-1,3-dihydro-5-(4-hydroxyphenyl) ethynyl-3-[(3-methoxy-1H-pyrrol-2-yl)-methylene]-2H-indol-2-one as a yellow powder. (Yield 62 mg, 62%).

**Example 97: Synthesis of (Z)-1,3-Dihydro-5-(4-methoxyphenyl)ethynyl-3-[(1H-pyrrol-2-yl)-methylene]-2H-indol-2-one (HHHH)**

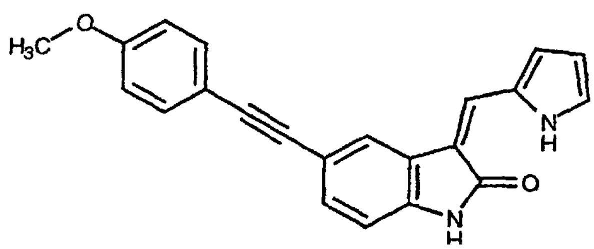

This compound was also prepared in a manner analogous to the preparation of (Z)-1,3-dihydro-5-(4-hydroxyphenyl)ethynyl-3-[(1H-pyrrol-2-yl)methylene]-2H-indol-2-one in Example 83 above. In this example, (Z)-1,3-dihydro-5-ethynyl-3-[(1H-pyrrol-2-yl)methylene]-2H-indol-2-one (60 mg, 0.26 mmol) (Example 82) was coupled with 4-iodoanisole (30 mg, 0.34 mmol) (Aldrich) using CuI (6 mg, 0.031 mmol) (Aldrich) and $(Ph_3P)_2PdCl_2$ (14 mg, 0.020 mmol) (Aldrich) as catalyst in 1 mL DMF and 2 mL triethylamine at 70 °C for 16 hours to give (Z)-1,3-dihydro-5-(4-methoxyphenyl)ethynyl-3-[(1H-pyrrol-2-yl)-methylene]-2H-indol-2-one as a yellow powder. (Yield 63 mg, 71%).

**<u>Example 98</u>: Synthesis of (Z)-1,3-Dihydro-3-[(1H-pyrrol-2-yl)-methylene]-5-(2-thiophenyl)ethynyl-2H-indol-2-one (IIII)**

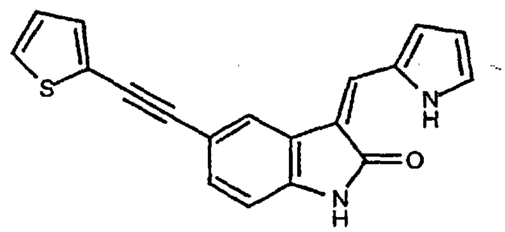

This compound was also prepared in a manner analogous to the preparation of (Z)-1,3-dihydro-5-(4-hydroxyphenyl)ethynyl-3-[(1H-pyrrol-2-yl)methylene]-2H-indol-2-one in Example 83 above. In this example, (Z)-1,3-dihydro-5-ethynyl-3-[(1H-pyrrol-2-yl)methylene]-2H-indol-2-one (42 mg, 0.18 mmol) (from Example 82) was coupled with 2-bromothiophene (30 mg, 0.18 mmol) (Aldrich) using CuI (8 mg, 0.042 mmol) (Aldrich) and $(Ph_3P)_2PdCl_2$ (14 mg, 0.020 mmol) (Aldrich) as catalyst in 1 mL DMF and 1 mL triethylamine at 70 °C for 14 hours to give (Z)-1,3-dihydro-3-[(1H-pyrrol-2-yl)-methylene]-5-(2-thiophenyl)ethynyl-2H-indol-2-one as a yellow powder. (Yield 25 mg, 44%). **<u>Example 99</u>: Synthesis of (Z)-5-Bromo-1,3-dihydro-3-[(4-methyl-1H-imidazol-5-yl)methylene]-2H-indol-2-one (JJJJ)**

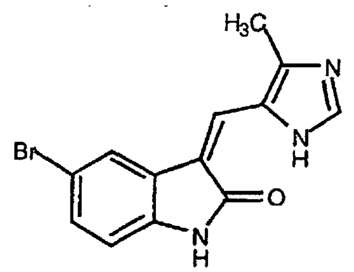

A mixture of 5-bromo-1,3-dihydro-2H-indol-2-one (0.3 g, 1.41 mmol) (Starting Material 6), and excess 4-methyl-5-imidazolecarboxaldehyde (0.25 g, 2.27 mmol) (Aldrich) in 1% piperidine in 2-propanol (6 mL) was heated at 90 °C for 4 h. Hot water (6 mL) was added. On cooling, the crystallized product was filtered off, washed with aqueous 2-propanol and dried. (Yield 0.44 g, 100%)

**<u>Example 100</u>: Synthesis of (Z)-1,3-Dihydro-3-[(4-methyl-1H-imidazol-5-yl)methylene]-5-(trimethylsilyl)ethynyl-2H-indol-2-one (KKKK)**

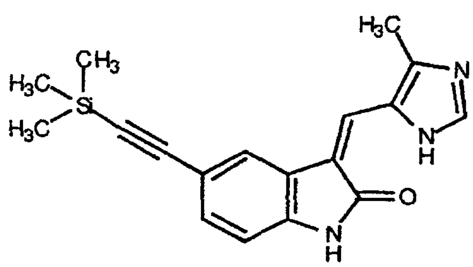

(Z)-5-Bromo-1,3-dihydro-3-[(4-methyl-1H-imidazol-5-yl)methylene]-2H-indol-2-one (0.17 g, 0.56 mmol) (from Example 99) was dissolved in 3 mL DMF and 3 mL triethylamine. The solution was degassed for 30 minutes by bubbling argon through the solution. At this time (trimethylsilyl)acetylene (0.3 mL, 2.1 mmol) (Aldrich), CuI (34 mg) (Aldrich) and $(Ph_3P)_2PdCl_2$ (34 mg) (Aldrich) were added and the reaction flask sealed. The reaction was heated, under argon, at 90 °C for 18 hours. After cooling, the mixture was filtered through Celite® (Fisher Scientific) and residue washed extensively with hot EtOAc and $CH_3CN$. Combined filtrate and washing was concentrated under reduced pressure and

the product was purified via flash column chromatography ($SiO_2$, 230-400 mesh) with 5% MeOH in $CH_2Cl_2$. (Yield 0.1 g, 56%).

**<u>Example 101</u>: Synthesis of (Z)-1,3-Dihydro-5-ethynyl-3-[(4-methyl-1H-imidazol-5-yl)methylene]-2H-indol-2-one, trifluoroacetate salt (LLLL)**

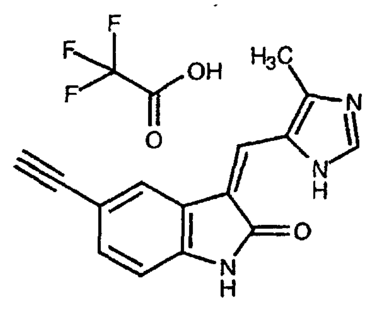

To a solution of (Z)-1,3-dihydro-3-[(4-methyl-1H-imidazol-5-yl)methylene]-5-(trimethylsilyl)ethynyl-2H-indol-2-one (0.1 g, 0.31 mmol) (from Example 100) in 8 mL ethanol was added dropwise a solution of silver nitrate (0.12 g, 0.68 mmol) in 1.5 mL water and 0.5 mL ethanol during which time a precipitate formed. The mixture was stirred at room temperature for 1 h, after which a solution of potassium cyanide (0.22 g, 3.37 mmol) in 1 mL of water was added and the precipitated dissolved. After stirring for an additional 20 min, 30 mL of water was added and the mixture extracted with EtOAc (3X30 mL). The product was purified by reverse phase HPLC using water/acetonitrile/trifluoroacetic acid as solvent. (Yield 20 mg, 18%).

**<u>Example 102</u>: SAPK Inhibitory Activity**

The SAPK inhibitory activity of the compounds of the invention is demonstrated below. These effects indicate that the compounds of the present invention are useful in treating inflammatory diseases such as, for example, rheumatoid arthritis.

**<u>SAPK FlashPlate Assay</u>**

Human JNK is highly homologous to rat SAPK. To measure the inhibitory activity of test compounds, the compounds were tested in the rat SAPK assay.

For the SAPK assay, purified GST-cJun (a chimeric protein containing cJun, a natural substrate of JNK) was coated on 96 well FlashPlates (New England Nuclear, Boston, MA). Purified rat SAPK (isoform β, Kyriakis et al. *supra*) was preincubated with preparations containing MEKK-1 and MKK4 for 30 minutes at 37ºC in assay buffer containing 25 mM HEPES, pH 7.5, 150 mM NaCl, 20 mM $MgCl_2$, 2 mM DTT, 0.001% Tween 20, 1 μM ATP freshly added. In the preincubation step, MEKK-1 phosphorylates and activates MKK-4, which in tum phosphorylates and activates SAPK. The activated SAPK was then added to the cJun coated FlashPlates along with $^{33}$P-ATP (0.32 μCi per reaction) and test compounds. The plates were incubated for 30 minutes at 37ºC, then washed with PBS, 0.01% Tween 20, and counted in the Topcount scintillation counter (Packard Instrument Co., Downers Grove, IL). Dilutions of compounds were tested in duplicate in each assay. The percent inhibition of cJun phosphorylation (a measure of inhibition of SAPK activity) was determined by the following formula:

$$100 \times \left[ 1 - \frac{\text{test compound - nonspecific}}{\text{total - nonspecific}} \right]$$

where "test compound" refers to the average counts per minute of the test duplicates, "nonspecific" refers to the average counts per minute when no SAPK was added, and "total" refers to the average counts per minute when no compound was added.

The results of the SAPK assay with various test compounds is summarized below in Table I A and I B.

Table I A

| | $IC_{50}$ (μM) |
|---|---|
| Compound | SAPK |
| D | <1.0 |
| G | <1.0 |
| H | <1.0 |
| M | <1.0 |
| N | <1.0 |
| Q | <1.0 |
| R | <1.0 |
| T | 0.253 |
| V | 0.034 |
| Y | <1.0 |
| BB | <1.0 |
| DD | <1.0 |
| EE | <1.0 |
| QQQ | <1.0 |
| FF | <1.0 |
| HH | <1.0 |
| MM | <1.0 |
| NN | <1.0 |
| OO | <1.0 |
| PP | <1.0 |
| RR | <1.0 |
| SS | <1.0 |
| TT | <1.0 |
| UU | <1.0 |
| VV<br>XX | <1.0<br><1.0 |
| YY | <1.0 |
| BBB | <1.0 |
| CCC | <1.0 |
| DDD | <1.0 |
| EEE | <1.0 |
| FFF | <1.0 |
| GGG | <1.0 |
| HHH | <1.0 |

Table I B

| | SAPK | |
|---|---|---|
| Compound | % Inhibition | Concentration (μM) |
| O | >50 | <1.0 |
| AA | >50 | <1.0 |
| III | <50 | <0.10 |

**MG-63 Cell-Based Assay**

The MG63 cell line, a human osteosarcoma cell line, was purchased from American Type Culture Collection (ATCC; Rockville, MD) and grown in the medium recommended by ATCC. When stimulated with human IL-1β , MG63 cells release matrix metalloproteinase 3 (MMP-3), an AP-1 dependent inflammatory mediator and IL-6, a NF-κB-dependent mediator. In this assay the ability of a test compound to block MMP-3 expression and not block IL-6 expression is an indication that the compound is a selective inhibitor of the AP-1 transcription pathway.

On day 1 the cells were plated at $2.5X10^4$ cells/well in 96 well plates. After 24 hours, dexamethasone (the assay control) (Sigma, St. Louis, MO) and the test compounds were diluted to appropriate concentrations and added to the MG63 cells. The cells were incubated with the compounds for 24 hours after which the supernatants were removed and analyzed by ELISA.

In the ELISA, 96 well plates were coated with antibody to MMP-3 or IL-6. Supernatants were added to the coated plates and any antigen (MMP-3 or IL-6) in the supernatant bound to the antibody coated on the plates. The plates were then washed with PBS containing 0.05% Tween 20 (Sigma, St. Louis, MO) and the biotinylated secondary antibody was added. This secondary antibody binds to the already bound antigen creating a "sandwich effect". Plates were washed as described above and horseradish peroxidase (HRP)-streptavidin conjugate (Sigma, St. Louis, MO) was added to the plates. HRP-streptavidin bound to the biotin-antibody conjugate. The plates were washed and TMB substrate (Kirkegaard and Perry Labs, Gaithersburg, MD) was added to the wells. This substrate changes color in the presence of HRP-streptavidin. The intensity of the color (measured at 450 nm) is proportional to the amount of MMP-3 or IL-6 produced by the MG63 cells upon exposure to IL-1β and the test compounds. Optical density values were converted to concentration (pg/ml or Units/ml) based on a standard curve included in the assay. $IC_{50}$ values for each test compound were determined from the linear regression of a plot of the logarithm of the concentration of compound versus amount of MMP-3 or IL-6 secreted. (The MMP-3 antibodies were prepared in-house using standard hybridoma technology and the IL-6 antibodies were obtained from either Genzyme, Cambridge, MA or Pharmingen, San Diego, CA.).

The results of this assay on various test compounds is summarized below in Table II.

Table II

| | $IC_{50}$ (μM) in MG63 Cells | |
|---|---|---|
| Compound | MMP3 | IL6 |
| D | <12.5 | 20.0 |
| G | <12.5 | <20 |
| H | <12.5 | <20 |
| N | <12.5 | <20 |
| O | <12.5 | <20 |
| P | <12.5 | <20 |
| R | <12.5 | <20 |
| Y | <12.5 | <20 |
| Z | <12.5 | <20 |
| BB | <12.5 | <20 |
| CC | <12.5 | <20 |

Table II (continued)

| | IC$_{50}$ (μM) in MG63 Cells | |
|---|---|---|
| Compound | MMP3 | IL6 |
| EE | <12.5 | <20 |
| QQQ | <12.5 | 20.0 |
| GG | <12.5 | <20 |
| CCC | <12.5 | >20.0 |
| FFF | <12.5 | <20 |
| III | <12.5 | 20.0 |

## U937 Cell-Based Assay

The U937 cells, a human monocyte/macrophage cell line, was obtained from the ATTC and grown in the recommended medium. These cells when stimulated with lipopolysaccharide (LPS) release TNF, another inflammatory mediator implicated in the JNK pathway (Swantek et al., *supra)* and IL-6. In this assay the ability of a test compound to block TNF expression is evaluated.

The assay is very similar to the MG63 cell based assay except for the following modifications. The U937 cells were suspension cells but when stimulated with phorbol myristate acetate (PMA) (Sigma, St. Louis, MO) they become adherent. After PMA stimulation the cells were washed in cell culture medium and plated at $1X10^5$ cells/well in 96 well plates. The following day the test compounds and dexamethasone control (Sigma, St. Louis, MO) were added to the cells for 1 hour of preincubation. Then the cells were stimulated with LPS (Sigma, St. Louis, MO). After an additional 24 hours of incubation the supematants were removed and assayed for TNF-α and IL-6 by ELISA. The IL-6 ELISA was run as described previously for the MG63 assay. The TNF ELISA was run using a kit supplied by Genzyme (Cambridge, MA).

The results of this assay with various test compounds is summarized below in Table III.

Table III

| | IC$_{50}$ (μM) in U937 Cells | |
|---|---|---|
| Compound | TNF | IL6 |
| N | <8 | <20 |
| O | <8 | <20 |
| R | <8 | 20.0 |
| BB | <8 | <20 |

## Example 103: Tablet Formulation

| Item | Ingredients | mg/Tablet | | | | | |
|---|---|---|---|---|---|---|---|
| 1 | Compound 1* | 5 | 25 | 100 | 250 | 500 | 750 |
| 2 | Anhydrous Lactose | 103 | 83 | 35 | 19 | 38 | 57 |
| 3 | Croscarmellose Sodium | 6 | 6 | 8 | 16 | 32 | 48 |
| 4 | Povidone K30 | 5 | 5 | 6 | 12 | 24 | 36 |
| 5 | Magnesium Stearate | 1 | 1 | 1 | 3 | 6 | 9 |
| | **Total Weight** | 120 | 120 | 150 | 300 | 600 | 900 |

*Compound 1 represents a compound of the invention.

**Manufacturing Procedure:**

1. Mix Items 1, 2 and 3 in a suitable mixer for 15 minutes.
2. Granulate the powder mix from Step 1 with 20% Povidone K30 Solution (Item 4).
3. Dry the granulation from Step 2 at 50°C.
4. Pass the granulation from Step 3 through a suitable milling equipment.
5. Add the Item 5 to the milled granulation Step 4 and mix for 3 minutes.
6. Compress the granulation from Step 5 on a suitable press.

**Example 104: Capsule Formulation**

| Item | Ingredients | mg/Capsule | | | | |
|---|---|---|---|---|---|---|
| 1 | Compound 1 * | 5 | 25 | 100 | 250 | 500 |
| 2 | Anhydrous Lactose | 159 | 123 | 148 | -- | -- |
| 3 | Corn Starch | 25 | 35 | 40 | 35 | 70 |
| 4 | Talc | 10 | 15 | 10 | 12 | 24 |
| 5 | Magnesium Stearate | 1 | 2 | 2 | 3 | 6 |
| | **Total Fill Weight** | 200 | 200 | 300 | 300 | 600 |

* Compound 1 represents a compound of the invention.

**Manufacturing Procedure:**

1. Mix Items 1, 2 and 3 in a suitable mixer for 15 minutes.
2. Add items 4 & 5 and mix for 3 minutes.
3. Fill into a suitable capsule.

**Example 105: Injection Solution/Emulsion Preparation**

| Item | Ingredient | mg/mL |
|---|---|---|
| 1 | Compound 1* | 1 mg |
| 2 | PEG 400 | 10-50 mg |
| 3 | Lecithin | 20-50 mg |
| 4 | Soy Oil | 1-5 mg |
| 5 | Glycerol | 8-12 mg |
| 6 | Water q.s. | 1 mL |

* Compound 1 represents a compound of the invention.

**Manufacturing Procedure:**

1. Dissolve item 1 in item 2

2. Add items 3, 4 and 5 to item 6 and mix until dispersed, then homogenize.

3. Add the solution from step 1 to the mixture from step 2 and homogenize until the dispersion is translucent.

4. Sterile filter through a 0.2 um filter and fill into vials.

**Example106: Injection Solution/Emulsion Preparation**

| Item | Ingredient | mg/mL |
|---|---|---|
| 1 | Compound 1* | 1 mg |
| 2 | Glycofurol | 10-50 mg |
| 3 | Lecithin | 20-50 mg |
| 4 | Soy Oil | 1-5 mg |
| 5 | Glycerol | 8-12 mg |
| 6 | Water | q.s. 1 mL |

* Compound 1 represents a compound of the invention.

**Manufacturing Procedure:**

1. Dissolve item 1 in item 2

2. Add items 3, 4 and 5 to item 6 and mix until dispersed, then homogenize.

3. Add the solution from step 1 to the mixture from step 2 and homogenize until the dispersion is translucent.

4. Sterile filter through a 0.2 um filter and fill into vials.

While the invention has been illustrated by reference to specific and preferred embodiments, those skilled in the art will understand that variations and modifications may be made through routine experimentation and practice of the invention. Thus, the invention is intended not to be limited by the foregoing description, but to be defined by the appended claims and their equivalents.

## Claims

**1.** A compound having the formula

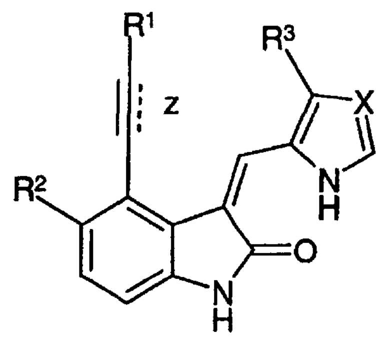

I

and the pharmaceutically acceptable salts thereof,
wherein:

$R^1$ is $C_{1-6}$ alkyl that is substituted by aryl, aryloxy, heteroaryl, heteroaryloxy, substituted aryl, substituted aryloxy, substituted heteroaryl, and/or substituted heteroaryloxy, and optionally also may be substituted by $R^{13}$, perfluoroalkyl, $C_{3-8}$ cycloalkyl (or $C_{3-8}$ cycloalkyl substituted by $C_{1-6}$ alkyl and/or $R^{13}$), or heterocycle (or heterocycle substituted by $C_{1-6}$ alkyl and/or $R^{13}$),
and wherein the substitutents on the substituted aryl, substituted aryloxy, substituted heteroaryl, and substituted heteroaryloxy are one or more of

$R^{13}$, $C_{1-6}$ alkyl (optionally substituted by $R^{13}$), $C_{3-8}$ cycloalkyl (optionally substituted by $R^{13}$), heterocycle (optionally substituted by $R^{13}$); aryl (optionally substituted by $R^{13}$, perfluoroalkyl, $C_{1-6}$ alkyl, $C_{1-6}$ alkyl substituted by $R^{13}$, $C_{3-8}$ cycloalkyl, $C_{3-8}$ cycloalkyl substituted by $R^{13}$, heterocycle (optionally substituted by $R^{13}$); or heteroaryl (optionally substituted by $R^{13}$, perfluoroalkyl, $C_{1-6}$ alkyl, $C_{1-6}$ alkyl substituted by $R^{13}$, $C_{3-8}$ cycloalkyl, $C_{3-8}$ cycloalkyl substituted by $R^{13}$, or heterocycle or heterocycle substituted by $R^{13}$); or
aryl (optionally substituted by halogen, $-OR^4$, $-COR^4$, $-COOR^4$, $-CONR^6R^7$, $C_{1-6}$ alkyl, $C_{3-8}$ cycloalkyl, heterocycle, $C_{1-6}$ alkyl which is substituted by $-OR^4$ , $-NR^6R^7$, $COOR^4$, $CONR^6R^7$, $C_{3-8}$ cycloalkyl which is substituted by $-OR^4$, $COOR^4$, $CONR^6R^7$, and/or $-NR^6R^7$, and heterocycle which is substituted by $-OR^4$, $COOR^4$, $CONR^6R^7$, and/or $-NR^6R^7$), or
heteroaryl (optionally substituted by halogen, $-OR^4$, $-COR^4$, $-COOR^4$, $-CONR^6R^7$, $C_{1-6}$ alkyl, $C_{3-8}$ cycloalkyl, heterocycle, $C_{1-6}$ alkyl which is substituted by $-OR^4$, $COOR^4$, $CONR^6R^7$, and/or $-NR^6R^7$, $C_{3-8}$ cycloalkyl which is substituted by $-OR^4$, $COOR^4$, $CONR^6R^7$, and/or $-NR^6R^7$, and/or heterocycle which is substituted by $-OR^4$, $COOR^4$, $CONR^6R^7$, and/or $-NR^6R^7$);

$R^2$ is hydrogen, $-OR^4$, $-OCOR^4$, $-COR^4$, $-COOR^4$, $-CONR^6R^7$, $-NR^6R^7$, halogen, $-NO_2$, -CN, $-SO_2R^4$, $-SO_2NR^6R^7$, perfluoroalkyl, $C_{1-6}$ alkyl or $C_{1-6}$ alkyl substituted by $-OR^8$ or $-NR^6R^7$;

$R^3$ is hydrogen, $-OR^4$, $-COR^4$, $-COOR^4$, $-CONR^6R^7$, halogen, -CN, $-NR^6R^7$, perfluoroalkyl, $C_{1-6}$ alkyl or $C_{1-6}$ alkyl substituted by $-OR^8$ or $-NR^6R^7$;

$R^4$ is hydrogen, $C_{1-6}$ alkyl (optionally substituted by (a), $C_{3-8}$ cycloalkyl and /or heterocycle), $C_{3-8}$ cycloalkyl (optionally substituted by (a), $C_{1-6}$ alkyl and/or heterocycle), heterocycle (optionally substituted by (a), $C_{1-6}$ alkyl and/or $C_{3-8}$ cycloalkyl), aryl (optionally substituted by (a), $C_{3-8}$ cycloalkyl, heterocycle and/or halogen), heteroaryl (optionally substituted by (a), $C_{3-8}$ cycloalkyl, heterocycle, and/or halogen,
where (a) is $-OR^5$, $-COOR^8$, $-COR^8$, $-CONR^8R^9$, $-NR^6R^7$, -CN, $-NO_2$, $-SO_2R^8$, and/or $-SO_2NR^8R^9$;

$R^5$ is hydrogen, $-COR^8$, $-CONR^8R^9$ or $C_{1-6}$ alkyl (optionally substituted by $-OR^9$, $-NR^9R^{10}$, $-N(COR^9)R^{10}$, $-COR^9$, $-CONR^9R^{10}$, $-SR^9$ and/or $-COOR^9$;

$R^6$ and $R^7$ are each hydrogen, $-COR^8$, $-COOR^8$, $-CONR^8R^9$, $-SO_2R^8$ $-SO_2NR^8R^9$, $C_{1-6}$ alkyl, $C_{1-6}$ alkyl substituted by (b), $C_{3-8}$ cycloalkyl (optionally substituted by (b), $C_{1-6}$ alkyl, and/or heterocycle), heterocycle, heterocycle substituted by (b), $C_{1-6}$ alkyl and/or $C_{3-8}$ cycloalkyl), aryl, aryl substituted by (b), $C_{1-6}$ alkyl, $C_{3-8}$ cycloalkyl and/or heterocycle), heteroaryl, heteroaryl substituted by (b), $C_{1-6}$ alkyl, $C_{3-8}$ cycloalkyl and/or heterocycle);
or $R^6$ and $R^7$ are each

$C_{3-8}$ cycloalkyl (optionally substituted by (b), $C_{1-6}$ alkyl and/or heterocycle; heterocycle (optionally substituted by (b), $C_{1-6}$ alkyl and/or $C_{3-8}$ cycloalkyl; aryl (optionally substituted by (b), $C_{1-6}$ alkyl, $C_{3-8}$ cycloalkyl and/or heterocycle; or heteroaryl (optionally substituted by (b), $C_{1-6}$ alkyl, $C_{3-8}$ cycloalkyl and/or heterocycle; where (b) is $OR^5$, $-NR^8R^9$, $-COOR^8$, $-COR^8$, $-CONR^8R^9$, -CN, $-NO_2$, $-SO_2R^8$, $-SO_2NR^8R^9$; altematively, $-NR^6R^7$ can form a ring having 3 to 7 atoms, said ring optionally including one or more additional hetero atoms and being optionally substituted by one or more of $C_{1-6}$ alkyl, $-OR^5$, $-COR^8$, $-COOR^8$, $-CONR^8R^9$, and $-NR^5R^9$;

$R^8$ is hydrogen, $C_{1-6}$ alkyl (optionally substituted by $C_{3-8}$ cycloalkyl, heterocycle, aryl, heteroaryl, $-OR^9$, $-NR^9R^{10}$, and/or $-N(COR^9)R^{10}$), aryl (optionally substituted by (c), $C_{1-6}$ alkyl, $C_{3-8}$ cycloalkyl and/or heterocycle),heteroaryl (optionally substituted by (c), $C_{1-6}$ alkyl, $C_{3-8}$ cycloalkyl and/or heterocycle), $C_{3-8}$ cycloalkyl (optionally substituted by (c), $C_{1-6}$ alkyl and/or heterocycle), heterocycle (optionally substituted by (c), $C_{1-6}$ alkyl and/or $C_{3-8}$ cycloalkyl); where (c) is $-OR^9$, $-COOR^9$, $-COR^9$, $-CONR^{10}R^9$, $-NR^{10}R^9$ -CN, $-NO_2$, $-SO_2R^9$, $-SO_2NR^{10}R^9$;

$R^9$ and $R^{10}$ are each independently hydrogen or $C_{1-6}$ alkyl;

$R^{13}$ is halogen, $-OR^4$, $-OCOR^4$, $-COR^4$ $-COOR^4$, $-CONR^6R^7$, $-NO_2$, $-NR^6R^7$, -CN, $-SO_2R^4$, or $-SO_2NR^6R^7$;

X is =N- or -CH-; and

the dotted bond represented by z is optional; wherein "aryl" refers to an aromatic group having 5 to 10 atoms and consisting of 1 or 2 rings; "aryloxy" refers to an aryl radical that includes at least one oxygen and which is attached to rest of molecule via the oxygen atom; "heteroaryl" refers to an aromatic group having 5 to 10 atoms, one or 2 rings, and containing one or more hetero atoms; "heteroaryloxy" refers to a heteroaryl radical that includes at least one oxygen and which is attached to rest of molecule via the oxygen atom; "hetero atom" refers to an atom selected from N, O and S; "heterocycle" refers to a 3- to 10-membered non-aromatic, partially or completely saturated hydrocarbon group which contains one or two rings and at least one hetero atom.

**2.** A compound of claim 1, wherein $R^1$ is $C_{1-6}$ alkyl that is substituted by aryl or substituted aryl, and optionally also substituted by halogen, $-OR^4$, $-COR^4$, $-COOR^4$, $-CONR^6R^7$, $C_{3-8}$ cycloalkyl, heterocycle, $-COOR^4$, $CONR^6R^7$, $C_{3-8}$ cycloalkyl which is substituted by $OR^4$, $-NR^6R^7$, $COOR^4$, $CONR^6R^7$, and/or heterocycle which is substituted by $OR^4$ and $-NR^6R^7$, $COOR^4$, $CONR^6R^7$; and wherein the substituents on the substituted aryl are selected from halogen, $-OR^4$, $-COR^4$, $-COOR^4$, $-CONR^6R^7$, $-NO_2$, $NR^6R^7$, $-SO_2R^4$, $-SO_2NR^6R^7$, -CN, perfluoroalkyl, $C_{1-6}$ alkyl, $C_{3-8}$ cycloalkyl, heterocycle, $C_{1-6}$ alkyl which is substituted by $-OR^4$ and $-NR^6R^7$, $COOR^4$, $CONR^6R^7$, $C_{3-8}$ cycloalkyl which is substituted by $OR^4$ and $-NR^6R^7$, $COOR^4$, $CONR^6R^7$, or heterocycle which is substituted by $OR^4$ and $-NR^6R^7$, $COOR^4$, $CONR^6R^7$; $C_{1-6}$ alkyl that is substituted by heteroaryl or substituted heteroaryl, and optionally also substituted by halogen, $-OR^4$, $-COR^4$, $-COOR^4$, $-CONR^6R^7$, $C_{3-8}$ cycloalkyl, heterocycle, $C_{3-8}$ cycloalkyl which is substituted by $OR^4$, $COOR^4$, $CONR^6R^7$, and/or $-NR^6R^7$, and/or heterocycle which is substituted by $-OR^4$, $COOR^4$, $CONR^6R^7$, and/or $-NR^6R^7$; and wherein the substituents on the substituted heteroaryl are selected from halogen, $-OR^4$, $-COR^4$, $-COOR^4$, $NR^6R^7$, $-SO_2R^4$, $-SO_2NR^6R^7$, $-NO_2$, -CN, $-CONR^6R^7$, $C_{1-6}$ alkyl, $C_{3-8}$ cycloalkyl, heterocycle, $C_{1-6}$ alkyl which is substituted by $-OR^4$, $-NR^6R^7$, $COOR^4$, $CONR^6R^7$, $C_{3-8}$ cycloalkyl which is substituted by $-OR^4$ , $-NR^6R^7$, $COOR^4$, $CONR^6R^7$, and/or heterocycle which is substituted by $-OR^4$, $-NR^6R^7$, $COOR^4$ and/or $CONR^6R^7$), aryl (optionally substituted by halogen, $-OR^4$, $-COR^4$, $-COOR^4$, $-CONR^6R^7$, $C_{1-6}$ alkyl, $C_{3-8}$ cycloalkyl, heterocycle, $C_{1-6}$ alkyl which is substituted by $-OR^4$ , $-NR^6R^7$, $COOR^4$, $CONR^6R^7$, $C_{3-8}$ cycloalkyl which is substituted by $-OR^4$, $COOR^4$, $CONR^6R^7$, and/or $-NR^6R^7$, and heterocycle which is substituted by $-OR^4$, $COOR^4$, $CONR^6R^7$, and/or $-NR^6R^7$), or heteroaryl (optionally substituted by halogen, $-OR^4$, $-COR^4$, $-COOR^4$, $-CONR^6R^7$, $C_{1-6}$ alkyl, $C_{3-8}$ cycloalkyl, heterocycle, $C_{1-6}$ alkyl which is substituted by $-OR^4$, $COOR^4$, $CONR^6R^7$, and/or $-NR^6R^7$, $C_{3-8}$ cycloalkyl which is substituted by $-OR^4$, $COOR^4$, $CONR^6R^7$, and/or $-NR^6R^7$, and/or heterocycle which is substituted by $-OR^4$, $COOR^4$, $CONR^6R^7$, and/or $-NR^6R^7$).

**3.** A compound of any one of claims 1 or 2, wherein X is CH and $R^3$ is $C_{1-6}$ alkoxy.

**4.** A compound of any one of claims 1-3 wherein $R^1$ is $C_{1-6}$ alkyl substituted by phenyl which is substituted by one to three substituents from the group hydroxy, $C_{1-6}$ alkoxy, di-( $C_{1-6}$ alkyl)-amino, di-( $C_{1-6}$ alkyl)amino- $C_{1-6}$ alkoxy, morpholino- $C_{1-6}$ alkyl, carboxy- $C_{1-6}$ alkoxy and $C_{1-6}$ alkanoylamino; or $R^1$ is $C_{1-6}$ alkyl substituted as before and additionally by hydroxy.

**5.** A compound of any of claims 1-4, wherein $R^1$ is $C_{1-6}$ alkyl substituted by pyridyl, pyrrolyl, N- $C_{1-6}$ alkyl-pyyrolyl, thienyl, $C_{1-6}$-alkoxy substitituted thienyl, furyl, 1,3-benzodioxolyl, or $C_{1-6}$-alkoxy substituted 1,3-benzodioxolyl; or $R^1$ is $C_{1-6}$ alkyl substituted as before and additionally by hydroxy.

**6.** A compound of any one of claims 1-3 wherein $R^1$ is pyridyl.

**7.** A compound of claim 1 or 2 wherein the optional bond z is present.

**8.** A compound of claim 4 which is

*rac*-(Z)-1,3-Dihydro-4-[3-hydroxy-3-(4-methoxyphenyl)-1-propynyl]-3-[(3-methoxy-1H-pyrrol-2-yl)methylene]-2H-indol-2-one (H),
*rac*-(Z)-1,3-Dihydro-4-[3-hydroxy-3-(3-hydroxyphenyl)-1-propynyl]-3-[(3-methoxy-1H-pyrrol-2-yl)methylene]-2H-indol-2-one (I),
*rac*-(Z)-1,3-Dihydro-4-[3-hydroxy-3-(3-methoxyphenyl)-1-propynyl]-3-[(3-methoxy-1H-pyrrol-2-yl)methylene]-2H-indol-2-one (J),
*rac*-(Z)-4-[3-[2,3-Dihydro-3-[(3-methoxy-1H-pyrrol-2-yl)methylene]-2-oxo-1H-indol-4-yl]-1-hydroxy-2-propynyl]benzoic acid methyl ester (K),
*rac*-(Z)-4-[3-[2,3-Dihydro-3-[(3-methoxy-1H-pyrrol-2-yl)methylene]-2-oxo-1H-indol-4-yl]-1-hydroxy-2-propynyl]benzoic acid (L),
*rac*-(Z)-1,3-Dihydro-4-[3-hydroxy-3-(2-methoxyphenyl)-1-propynyl]-3-[(3-methoxy-1H-pyrrol-2-yl)methylene]-2H-indol-2-one (M),
*rac*-(Z)-4-[3-(1,3-benzodioxol-5-yl)-3-hydroxy-1-propynyl]-1,3-dihydro-3-[(3-methoxy-1H-pyrrol-2-yl)methylene]-2H-indol-2-one (N),
*rac*-(Z)-1,3-Dihydro-4-[3-hydroxy-3-(4-hydroxy-3-methoxyphenyl)-1-propynyl]-3-[(3-methoxy-1H-pyrrol-2-yl) methylene]-2H-indol-2-one (O),
*rac*-(Z)-1,3-Dihydro-4-[3-hydroxy-3-(4-hydroxyphenyl)-1-propynyl]-3-[(3-methoxy-1H-pyrrol-2-yl)methylene]-2H-indol-2-one (Q),
*rac*-(Z)-1,3-Dihydro-4-[3-(4-dimethylaminophenyl)-3-hydroxy-1-propynyl]-3-[(3-methoxy-1H-pyrrol-2-yl)methylene]-2H-indol-2-one (R),
*rac*-(Z)-1,3-Dihydro-4-[3-hydroxy-3-(4-phenoxyphenyl)-1-propynyl]-3-[(3-methoxy-1H-pyrrol-2-yl)methylene]-2H-indol-2-one (S),
*rac*-(Z)-1,3-Dihydro-4-[3-hydroxy-3-phenyl-1-butynyl]-3-[(3-methoxy-1H-pyrrol-2-yl)methylene]-2H-indol-2-one (T),
*rac*-(Z)-1,3-Dihydro-4-[3-[4-(3-dimethylaminopropoxy)-phenyl]-3-hydroxy-1-propynyl]-3-[(3-methoxy-1H-pyrrol-2-yl)methylene]-2H-indol-2-one (V),
*rac*-(Z)-1,3-Dihydro-4-[3-(2,3-dimethoxyphenyl)-3-hydroxy-1-propynyl]-3-[(3-methoxy-1H-pyrrol-2-yl)methylene]-2H-indol-2-one (EE),
*rac*-(Z)-1,3-Dihydro-4-[3-(3,4-dimethoxyphenyl)-3-hydroxy-1-propynyl]-3-[(3-methoxy-1H-pyrrol-2-yl)methylene]-2H-indol-2-one (FF),
*rac*-(Z)-1,3-Dihydro-4-[3-hydroxy-3-(3-hydroxy-4-methoxyphenyl)-1-propynyl]-3-[(3-methoxy-1H-pyrrol-2-yl) methylene]-2H-indol-2-one (HH),
*rac*-(Z)-1,3-Dihydro-4-[3-hydroxy-3-[3-methoxy-4-[2-(4-morpholinyl)-ethoxy]-phenyl]-1-propynyl]-3-[(3-methoxy-1H-pyrrol-2-yl)methylene]-2H-indol-2-one (MM).
*rac*-(Z)-1,3-Dihydro-4-[3-hydroxy-3-[3-methoxy-4-[2-(4-morpholinyl)-ethoxy]-phenyl]-1-propynyl]-3-[(3-methoxy-1H-pyrrol-2-yl)methylene]-2H-indol-2-one hydrochloride salt (NN),
*rac*-(Z)-1,3-Dihydro-4-[3-hydroxy-3-(2,4,5-trimethoxyphenyl)-1-propynyl]-3-[(3-methoxy-1H-pyrrol-2-yl)methylene]-2H-indol-2-one (PP),
*rac*-(Z)-[4-[3-[2,3-Dihydro-3-[(3-methoxy-1H-pyrrol-2-yl)methylene]-2-oxo-1H-indol-4-yl]-1-hydroxy-2-propynyl]-2-methoxyphenoxy]acetic acid methyl ester (QQ),
*rac*-(Z)-[4-[3-[2,3-Dihydro-3-[(3-methoxy-1H-pyrrol-2-yl)methylene]-2-oxo-1H-indol-4-yl]-1-hydroxy-2-propynyl]-2-methoxyphenoxy]acetic acid (RR),
*rac*-(Z)-4-[3-hydroxy-3-(4-methoxy-1,3-benzodioxol-6-yl)-1-propynyl]-1,3-dihydro-3-[(3-methoxy-1H-pyrrol-2-yl)methylene]-2H-indol-2-one (SS),
*rac*-(Z)-1,3-Dihydro-4-[3-hydroxy-3-[4-[2-(4-morpholinyl)-ethoxy]-phenyl]-1-propynyl]-3-[(3-methoxy-1H-pyrrol-2-yl)methylene]-2H-indol-2-one (TT),
*rac*-(Z)-4-[3-(4-Chloro-2-methylsulfanylmethoxy-phenyl)-3-hydroxy-1-propynyl]-1,3-dihydro-3-[(3-methoxy-1H-pyrrol-2-yl)methylene]-2H-indol-2-one (UU),
*rac*-(Z)-4-[3-(3-Chlorophenyl)-3-hydroxy-1-propynyl]-1,3-dihydro-3-[(3-methoxy-1H-pyrrol-2-yl)methylene]-2H-indol-2-one (WW),
*rac*-(Z)-[4-[3-[2,3-dihydro-3-[(3-methoxy-1H-pyrrol-2-yl)methylene]-2-oxo-1H-indol-4-yl]-1-hydroxy-2-propynyl]phenoxy]acetic acid 1,1-dimethylethyl ester (XX),
*rac*-(Z)-[4-[3-[2,3-dihydro-3-[(3-methoxy-1H-pyrrol-2-yl)methylene]-2-oxo-1H-indol-4-yl]-1-hydroxy-2-propynyl]phenoxy]acetic acid(YY),
*rac*-(Z)-1,3-Dihydro-4-[3-hydroxy-3-(3-nitrophenyl)-1-propynyl]-3-[(3-methoxy-1H-pyrrol-2-yl)methylene]-2H-indol-2-one (ZZ),
*rac*-(Z)-4-[3-(3-Aminophenyl)-3-hydroxy-1-propynyl]-1,3-dihydro-3-[(3-methoxy-1H-pyrrol-2-yl)methylene]-2H-indol-2-one (AAA),
*rac*-(Z)-4-[3-(4-Acetamidophenyl)-3-hydroxy-1-propynyl]-1,3-dihydro-3-[(3-methoxy-1H-pyrrol-2-yl)methylene]-2H-indol-2-one (BBB), or
*rac*-(Z)-1,3-Dihydro-4-(3-hydroxy-3-phenyl-1-propynyl)-3-[(3-methoxy-1H-pyrrol-2-yl)methylene]-2H-indol-2-one (FFF).

**9.** A compound of claim 5 which is

*rac*-(Z)-1,3-Dihydro-4-[3-hydroxy-3-(3-pyridinyl)-1-propynyl]-3-[(3-methoxy-1H-pyrrol-2-yl)methylene]-2H-indol-2-one (X),
Synthesis of *rac*-(Z)-1,3-Dihydro-4-[3-hydroxy-3-(1-methyl-pyrrol-2-yl)-1-propynyl]-3-[(3-methoxy-1H-pyrrot-2-yl)methylene]-2H-indol-2-one (AA),
*rac*-(Z)-1,3-Dihydro-4-[3-hydroxy-3-(thiophen-3-yl)-1-propynyl]-3-[(3-methoxy-1H-pyrrol-2-yl)methylene]-2H-indol-2-one (BB),
*rac*-(Z)-1,3-Dihydro-4-[3-hydroxy-3-(1H-pyrrol-2-yl)-1-propynyl]-3-[(3-methoxy-1H-pyrrol-2-yl)methylene]-2H-indol-2-one (DD),*rac*-(Z)-1,3-Dihydro-4-[3-hydroxy-3-(2-pyridinyl)-1-propynyl]-3-[(3-methoxy-1H-pyrrol-2-yl)methylene]-2H-indol-2-one (JJ),
*rac*-(Z)-1,3-Dihydro-4-[3-hydroxy-3-(2-thiophenyl)-1-propynyl]-3-[(3-methoxy-1H-pyrrol-2-yl)methylene]-2H-indol-2-one (KK),
*rac*-(Z)-1,3-Dihydro-4-[3-hydroxy-3-(3-methoxy-2-thiophenyl)-1-propynyl]-3-[(3-methoxy-1H-pyrrol-2-yl) methylene]-2H-indol-2-one (OO), or
*rac*-(Z)-1,3-Dihydro-4-[3-(2-furanyl)-3-hydroxy-1-propynyl]-3-[(3-methoxy-1H-pyrrol-2-yl)methylene]-2H-indol-2-one (VV).

**10.** A compound of claim 6 which is

(Z)-1,3-Dihydro-3-[(3-methoxy-1H-pyrrol-2-yl)methylene]-4-[(3-pyridinyl)ethynyl]-2H-indol-2-one(CCC),
(Z)-1,3-Dihydro-3-[(3-methoxy-1H-pyrrol-2-yl)methylene]-4-[(2-pyridinyl)ethynyl]-2H-indol-2-one (DDD),
(Z)-1,3-Dihydro-3-[(3-methoxy-1H-pyrrol-2-yl)methylene]-4-[(4-pyridinyl)ethynyl]-2H-indol-2-one (EEE),
(Z)-1,3-Dihydro-3-[(3-methoxy-1H-pyrrol-2-yl)methylene]-5-nitro-4-[(3-pyridinyl)ethynyl]-2H-indol-2-one (GGG),
(Z)-5-Amino-1,3-dihydro-3-[(3-methoxy-1H-pyrrol-2-yl)methylene]-4-[(3-pyridinyl)ethynyl]-2H-indol-2-one (HHH), or
(Z)-N-[2,3-Dihydro-3-[(3-methoxy-1H-pyrrol-2-yl)methylene]-2-oxo-4-[(3-pyridinyl)ethynyl]-1H-indol-5-yl]-2-thiopheneacetamide (III).

**11.** A compound of claim 1 which is

4-[(E)-2-(2-Chlorophenyl)-ethenyl]-1,3-dihydro-(Z)-3-[(1H-pyrrol-2-yl)methylene]-2H-indol-2-one (KKK),
1,3-Dihydro-(Z)-3-[(1H-pyrrol-2yl)methylene]-[(E)-2-phenylethenyl]-2H-indol-2-one (LLL),
1,3-Dihydro-(Z)-3-[(3-methoxy-1H-pyrrol-2yl)methylene]-[(E)-2-phenylethenyl]-2H-indol-2-one (MMM),
1,3-Dihydro-4-[(E)-2-(4-methoxyphenyl)-ethenyl]-(Z)-3-[(1H-pyrrol-2-yl)methlene]-2H-indol-2-one (NNN),

1,3-Dihydro-(Z)-3-[(3-methoxy-1H-pyrrol-2-yl)methylene]-4-[(E)-2-(4-methoxy-phenyl)-ethenyl]-2H-indol-2-one (OOO),
4-[(E)-2-[2,3-Dihydro-(Z)-3-[(3-methoxy-1H-pyrrol-2-yl)methylene]-2-oxo-1H-indol-4-yl]ethenyl]benzoic acid methyl ester (PPP), or
1,3-Dihydro-4-[(E)-2-(3,4-dimethoxyphenyl)-ethenyl]-(Z)-3-[(3-methoxy-1H-pyrrol-2-yl)methylene]-2H-indol-2-one (QQQ).

**12.** Acompound of claim 1, which is

(Z)-1,3-Dihydro-4-(phenylethynyl)-3-[(3-methoxy-1H-pyrrol-2-yl)methylene]-2H-indol-2-one (D),
(Z)-1,3-Dihydro-4-[(4-methoxyphenyl)ethynyl]-3-[(3-methoxy-1H-pyrrol-2-yl)methylene]-2H-indol-2-one (G) or
(Z)-1,3-Dihydro-3-[(3-methoxy-1H-pyrrol-2-yl)methylene]-4-(3-phenoxy-1-propynyl)-2H-indol-2-one (Y).

**13.** A compound having the formula:

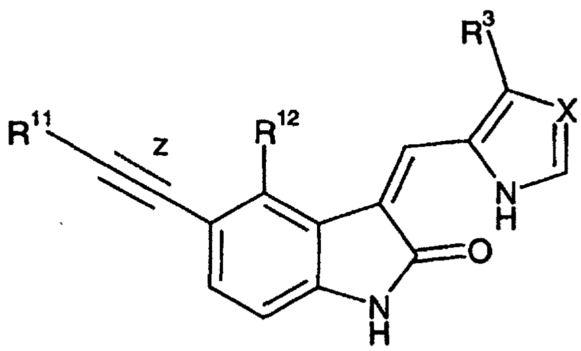

II

and the pharmaceutically acceptable salts thereof,
wherein:

$R^{11}$ is hydrogen, $-COR^4$, $-COOR^4$, $-CONR^6R^7$,
$C_{1-6}$ alkyl (optionally substituted by $-OR^5$, $-NR^6R^7$, halogen, $-NO_2$, $-SO_2R^4$, $-SO_2NR^6R^7$, -CN, $-COR^4$, $-COOR^4$, $-CONR^6R^7$, $C_{3-8}$ cycloalkyl, heterocycle, aryl, and/or heteroaryl),
$C_{3-8}$ cycloalkyl (optionally substituted by $-OR^5$, $-NR^6R^7$, halogen, $-NO_2$, $-SO_2R^4$, $-SO_2NR^6R^7$, -CN, $-COR^4$,
$-COOR^4$, $-CONR^6R^7$, $C_{1-6}$ alkyl, heterocycle, aryl, and/or heteroaryl)
heterocycle (optionally substituted by $-OR^5$, $-NR^6R^7$, halogen, $-NO_2$, $-SO_2R^4$, $-SO_2NR^6R^7$, -CN, $-COR^4$, $-COOR^4$, $-CONR^6R^7$, $C_{1-6}$ alkyl, $C_{3-8}$ cycloalkyl, aryl, and/or heteroaryl),
aryl (optionally substituted by the group consisting of $-OR^5$, $-NR^6R^7$, halogen, $-NO_2$, $-SO_2R^4$, $-SO_2NR^6R^7$, -CN, $-COR^4$, $-COOR^4$, $-CONR^6R^7$, $C_{1-6}$ alkyl, and/or perfluoroalkyl) or
heteroaryl (optionally substituted by $-OR^5$, $-NR^6R^7$, halogen, $-NO_2$, $-SO_2R^4$, $-SO_2NR^6R^7$, -CN, $-COR^4$, $-COOR^4$,
$-CONR^6R^7$, $C_{1-6}$ alkyl, and/or perfluoroalkyl);

$R^{12}$ is hydrogen, $-OR^4$, $-OCOR^4$, $-COR^4$, $-COOR^4$, $-CONR^6R^7$, - $NR^6R^7$, halogen, $-NO_2$, -CN, $-SO_2R^4$, $-SO_2NR^6R7$, perfluoroalkyl,
$C_{1-6}$ alkyl (optionally substituted by $OR^4$,$-NR^6R^7$, $C_{3-8}$ cycloalkyl, heterocycle, $-COR^4$, $-COOR^4$, $-CONR^6R^7$, -CN, $-NO_2$, $-SO_2R^4$, $-SO_2NR^6R^7$ and/or halogen),
$C_{3-8}$ cycloalkyl (optionally substituted by $-OR^4$, $-NR^6R^7$, $C_{1-6}$ alkyl, heterocycle, $-COR^4$, $-COOR^4$, $-CONR^6R^7$, -CN, $-NO_2$, $-SO_2R^4$, $-SO_2NR^6R^7$ and/or halogen), or
heterocycle (optionally substituted by $-OR^4$, $-NR^6R^7$, $C_{1-6}$ alkyl, $C_{3-8}$ cycloalkyl, $-COR^4$, $-COOR^4$, $-CONR^6R^7$, -CN, $-NO_2$, $-SO_2R^4$, $-SO_2NR^6R^7$ and/or halogen), and

$R^3$ through $R^7$, X, z aryl, heteroaryl, and heterocycle are as defined for formula I in claim 1.

**14.** A compound of claim 1 or 13, wherein

$R^4$ is hydrogen, $C_{1-6}$ alkyl (optionally substituted by (a), $C_{3-8}$ cycloalkyl and /or heterocycle), $C_{3-8}$ cycloalkyl (optionally substituted by (a), $C_{1-6}$ alkyl and/or heterocycle), or heterocycle (optionally substituted by (a), $C_{1-6}$ alkyl and/or $C_{3-8}$ cycloalkyl), where (a) is $-OR^5$, $-COOR^8$, $-COR^8$, $-CONR^8R^9$, $-NR^6R^7$, -CN, $-NO_2$, $-SO_2R^8$, and/or $-SO_2NR^8R^9$; and $R^5$ is hydrogen, $-COR^8$, $-CONR^8R^9$ or $C_{1-6}$ alkyl (optionally substituted by $-OR^9$, $-NR^9R^{10}$, $-N(COR^9)R^{10}$, $-COR^9$, $-CONR^9R^{10}$ and/or $-COOR^9$); and $R^1$, $R^2$, $R^3$, $R^8$, $R^9$, $R^{10}$, X and z are as in claim 1.

**15.** A compound of claim 13 wherein

$R^3$ is hydrogen, $-OR^4$, $-NR^6R^7$, and/or $C_{1-6}$ alkyl (optionally substituted by $-OR^8$ and/or $-NR^6R^7$);

$R^4$ is hydrogen, $C_{1-6}$ alkyl (optionally substituted by one or more $-OR^5$, $-COOR^8$, $-COR^8$, $-CONR^8R^9$), $C_{3-8}$ cycloalkyl (optionally substituted by one or more $-OR^5$, $-COOR^8$, $-COR^8$ and $-CONR^8R^9$), or heterocycle (optionally substituted by one or more $-OR^5$, $-COOR^8$, $-COR^8$ and $-CONR^8R^9$);

$R^5$ is hydrogen, $-COR^8$, $-CONR^8R^9$, or $C_{1-6}$ alkyl;

$R^6$ and $R^7$ are each independently hydrogen, $-COR^8$, $-COOR^8$, $-CONR^8R^9$, or $C_{1-6}$ alkyl (optionally substituted by one or more of $-OR^9$ , $-NR^8R^9$, $COOR^8$, and $CONR^8R^9$), or alternatively, $-NR^6R^7$ optionally form a ring having 3 to 7 atoms, said ring optionally including one or more additional hetero atoms and being optionally substituted by one or more of $C_{1-6}$ alkyl, $-OR^5$, $-COR^8$, $-COOR^8$, $-CONR^8R^9$, and $-NR^5R^9$;

$R^8$ is hydrogen or $C_{1-6}$ alkyl (optionally substituted by one or more of aryl, heteroaryl, $-OR^9$, $COOR^9$, $CONR^9R^{10}$, and $-NR^9R^{10}$);

$R^{11}$ is aryl (optionally substituted by $-OR^5$ and/or $-NR^6R^7$);

$R^{12}$ is hydrogen, $-COR^4$, $-COOR^4$, $-CONR^6R^7$,
$C_{1-6}$ alkyl (optionally substituted by one or more of $-OR^4$,$-NR^6R^7$, $C_{3-8}$ cycloalkyl, heterocycle, $-COR^4$, $-COOR^4$, $-CONR^6R^7$, -CN, $-NO_2$, $-SO_2R^4$, $-SO_2$ $NR^6R^7$ and halogen),
$C_{3-8}$ cycloalkyl (optionally substituted by one or more of $-OR^4$, $-NR^6R^7$, $C_{1-6}$ alkyl, heterocycle, $-COR^4$, $-COOR^4$, $-CONR^6R^7$, -CN, $-NO_2$, $-SO_2R^4$, $-SO_2$ $NR^6R^7$ and halogen), or
heterocycle (optionally substituted by one or more of $-OR^4$, $-NR^6R^7$, $C_{1-6}$ alkyl, $C_{3-8}$ cycloalkyl, $-COR^4$, $-COOR^4$, $-CONR^6R^7$, -CN, $-NO_2$, $-SO_2R^4$, $-SO_2$ $NR^6R^7$ and halogen);

and the optional bond z is present.

**16.** A compound of claim 13 which is

(Z)-1,3-Dihydro-5-ethynyl-3-[(1H-pyrrol-2-yl)methylene]-2H-indol-2-one (SSS),
(Z)-1,3-Dihydro-5-(4-hydroxyphenyl)ethynyl-3-[(1H-pyrrol-2-yl)methylene]-2H-indol-2-one (TTT),
(Z)-1,3-Dihydro-5-(3-nitrophenyl)ethynyl-3-[(1H-pyrrol-2-yl)methylene]-2H-indol-2-one (UUU),
(Z)-1,3-Dihydro-5-phenylethynyl-3-[(1H-pyrrol-2-yl)methylene]-2H-indol-2-one (VVV),
(Z)-1,3-Dihydro-5-(3-hydroxyphenyl)ethynyl-3-[(1H-pyrrol-2-yl)methylene]-2H-indol-2-one (WWW),
(Z)-1,3-Dihydro-5-(2-nitrophenyl)ethynyl-3-[(1H-pyrrol-2-yl)methylene]-2H-indol-2-one (XXX),
(Z)-1,3-Dihydro-5-(4-nitrophenyl)ethynyl-3-[(1H-pyrrol-2-yl)methylene]-2H-indol-2-one (ZZZ),
(Z)-5-(4-Aminophenyl)ethynyl-1,3-dihydro-3-[(1H-pyrrol-2-yl)methylene]-2H-indot-2-one (AAAA),
(Z)-1,3-Dihydro-5-ethynyl-3-[(3-methoxy-1H-pyrrol-2-yl)methylene]-2H-indol-2-one (DDDD),
(Z)-1,3-Dihydro-5-(3-pyridinyl)ethynyl-3-[(1H-pyrrol-2-yl)methylene]-2H-indol-2-one (EEEE),
(Z)-1,3-Dihydro-5-(2-pyridinyl)ethynyl-3-[(1H-pyrrol-2-yl)methylene]-2H-indol-2-one (FFFF),
(Z)-1,3-Dihydro-5-(4-hydroxyphenyl)ethynyl-3-[(3-methoxy-1H-pyrrol-2-yl)-methylene]-2H-indol-2-one (GGGG),
(Z)-1,3-Dihydro-5-(4-methoxyphenyl)ethynyl-3-[(1H-pyrrol-2-yl)-methylene]-2H-indol-2-one (HHHH),
(Z)-1,3-Dihydro-3-[(1H-pyrrol-2-yl)-methylene]-5-(2-thiophenyl)ethynyl-2H-indol-2-one (IIII), or
(Z)-1,3-Dihydro-5-ethynyl-3-[(4-methyl-1H-imidazol-5-yl)methylene]-2H-indol-2-one, trifluoroacetate salt (LLLL).

**17.** The compounds

(Z)-1,3-Dihydro-3-[(1H-pyrrol-2-yl)methylene]-5-(trimethylsilyl)ethynyl-2H-indol-2-one,
(Z)-5-Bromo-1,3-dihydro-3-[(3-methoxy-1H-pyrrol-2-yl)methylene]-2H-indol-2-one,
(Z)-1,3-Dihydro-3-[(3-methoxy-1H-pyrrol-2-yl)methylene]-5-(trimethylsilyl)ethynyl-2H-indol-2-one,
(Z)-5-Bromo-1,3-dihydro-3-[(4-methyl-1H-imidazol-5-yl)methylene]-2H-indol-2-one,
(Z)-1,3-Dihydro-3-[(4-methyl-1H-imidazol-5-yl)methylene]-5-(trimethylsilyl)ethynyl-2H-indol-2-one.

**18.** A pharmaceutical composition comprising as an active ingredient a compound of claim 1 or 13 and a pharmaceutically acceptable carrier or excipient.

**19.** The compounds of claim 1 and 13 for use as medicaments.

**20.** The use of a compound of claim 1 or 13 or prodrugs and pharmaceutically active metabolites of such compound in the preparation of a medicament for the treatment or control of inflammatory diseases, particularly rheumatoid arthritis.

**Patentansprüche**

**1.** Verbindung der Formel

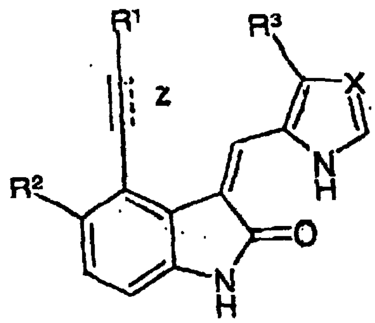

I

und die pharmazeutisch verträglichen Salze davon,
wobei

$R^1$ ein $C_{1-6}$-Alkylrest ist, welcher substituiert ist mit einem Aryl-, einem Aryloxy-, einem Heteroaryl-, einem Heteroaryloxyrest, einem substituierten Aryl-, einem substituierten Aryloxy-, einem substituierten Heteroarylrest und/oder einem substituierten Heteroaryloxyrest, und welcher gegebenenfalls auch substituiert sein kann mit $R^{13}$, einem Perfluoralkyl-, einem $C_{3-8}$-Cycloalkylrest (oder einem mit einem $C_{1-6}$-Alkylrest und/oder $R^{13}$ substituierten $C_{3-8}$-Cycloalkylrest), oder einem Heterocyclus (oder einem mit einem $C_{1-6}$-Alkylrest und/oder $R^{13}$ substituierten Heterocyclus),
und wobei die Substituenten am substatuierten Aryl-, substituierten Aryloxy-, substituierten Heteroaryl- und substituierten Heteroaryloxyrest ein oder mehrere sind aus
$R^{13}$, einem $C_{1-6}$-Alkylrest (gegebenenfalls substituiert mit $R^{13}$), einem $C_{3-8}$-Cycloalkylrest (gegebenenfalls substituiert mit $R^{13}$), einem Heterocyclus (gegebenenfalls substituiert mit $R^{13}$), einem Arylrest (gegebenenfalls substituiert mit $R^{13}$), einem Perfluoralkylrest, einem $C_{1-6}$-Alkylrest, einem mit $R^{13}$ substituierten $C_{1-6}$-Alkylrest, einem $C_{3-8}$-Cycloalkylrest, einem mit $R^{13}$ substituierten $C_{3-8}$-Cycloalkylrest, einem Heterocyclus (gegebenenfalls substituiert mit $R^{13}$) oder einem Heteroarylrest (gegebenenfalls substituiert mit $R^{13}$, einem Perfluoralkylrest, einem $C_{1-6}$-Alkylrest, einem mit $R^{13}$ substituierten $C_{1-6}$-Alkylrest, einem $C_{3-8}$-Cycloalkylrest, einem mit $R^{13}$ substituierten $C_{3-8}$-Cycloalkylrest, oder einem Heterocyclus oder einem mit $R^{13}$ substituierten Heterocyclus), oder
einem Arylrest (gegebenenfalls substituiert mit einem Halogenatom, $-OR^4$, $-COR^4$, $-COOR^4$, $-CONR^6R^7$, einem $C_{1-6}$-Alkylrest, einem $C_{3-8}$-Cycloalkylrest, einem Heterocyclus, einem $C_{1-6}$-Alkylrest, welcher substituiert ist mit $-OR^4$, $-NR^6R^7$, $COOR^4$, $CONR^6R^7$, einem $C_{3-8}$-Cycloalkylrest, welcher substituiert ist mit $-OR^4$, $COOR^4$, $CONR^6R^7$ und/oder $-NR^6R^7$ und einem Heterocyclus, welcher substituiert ist mit $-OR^4$, $-COOR^4$, $-CONR^6R^7$

und/oder $-NR^6R^7$), oder
einem Heteroarylrest (gegebenenfalls substituiert mit einem Halogenatom, $-OR^4$, $-COR^4$, $-COOR^4$, $-CONR^6R^7$, einem $C_{1-6}$-Alkylrest, einem $C_{3-8}$-Cycloalkylrest, einem Heterocyclus, einem $C_{1-6}$-Alkylrest, welcher substituiert ist mit $-OR^4$, $-COOR^4$, $-CONR^6R^7$ und/oder $-NR^6R^7$, einem $C_{3-8}$-Cycloalkylrest, welcher substituiert ist mit $-OR^4$, $COOR^4$, $CONR^6R^7$ und/oder $-NR^6R^7$ und/oder einem Heterocyclus, welcher substituiert ist mit $-OR^4$, $COOR^4$, $CONR^6R^7$ und/oder $-NR^6R^7$),

$R^2$ ein Wasserstoffatom, $-OR^4$, $-OCOR^4$, $-COR^4$, $-COOR^4$, $-CONR^6R^7$, $-NR^6R^7$, ein Halogenatom, $-NO_2$, -CN, $-SO_2R^4$, $-SO_2NR^6R^7$, ein Perfluoralkylrest, ein $C_{1-6}$-Alkylrest oder ein mit $-OR^8$ oder $-NR^6R^7$ substituierter $C_{1-6}$-Alkylrest, ist,

$R^3$ ein Wasserstoffatom, $-OR^4$, $-COR^4$, $-COOR^4$, $-CONR^6R^7$, ein Halogenatom, -CN, $-NR^6R^7$, ein Perfluoralkylrest, ein $C_{1-6}$-Alkylrest oder ein mit $-OR^8$ oder $-NR^6R^7$ substituierter $C_{1-6}$-Alkylrest, ist,

$R^4$ ein Wasserstoffatom, ein $C_{1-6}$-Alkylrest (gegebenenfalls substituiert mit (a), einem $C_{3-8}$-Cycloalkylrest und/oder einem Heterocyclus), ein $C_{3-8}$-Cycloalkylrest (gegebenenfalls substituiert mit (a), einem $C_{1-6}$-Alkylrest und/oder einem Heterocyclus), ein Heterocyclus (gegebenenfalls substituiert mit (a), einem $C_{1-6}$-Alkylrest und/oder einem $C_{3-8}$-Cycloalkylrest), ein Arylrest (gegebenenfalls substituiert mit (a), einem $C_{3-8}$-Cycloalkylrest, einem Heterocyclus und/oder einem Halogenatom), ein Heteroarylrest (gegebenenfalls substituiert mit (a), einem $C_{3-8}$-Cycloalkylrest, einem Heterocyclus und/oder einem Halogenatom) ist,
wobei (a) $-OR^5$, $-COOR^8$, $-COR^8$, $-CONR^8R^9$, $-NR^6R^7$, -CN, $-NO_2$, $-SO_2R^8$, und/oder $-SO_2NR^8R^9$ ist,

$R^5$ ein Wasserstoffatom, $-COR^8$, $-CONR^8R^9$ oder ein $C_{1-6}$-Alkylrest (gegebenenfalls substituiert mit $-OR^9$, $-NR^9R^{10}$, $-N(COR^9)R^{10}$, $-COR^9$, $-CONR^9R^{10}$, $-SR^9$ und/oder $-COOR^9$) ist,

$R^6$ und $R^7$ jeweils ein Wasserstoffatom, $-COR^8$, $-COOR^8$, $-CONR^8R^9$, $-SO_2R^8$, $-SO_2NR^8R^9$, ein $C_{1-6}$-Alkylrest, ein mit (b) substituierter $C_{1-6}$-Alkylrest, ein $C_{3-8}$-Cycloalkylrest (gegebenenfalls substituiert, mit (b), einem $C_{1-6}$-Alkylrest und/oder einem Heterocyclus), ein Heterocyclus, ein mit (b), einem $C_{1-6}$-Alkylrest und/oder einem $C_{3-8}$-Cycloalkylrest substituierter Heterocyclus, ein Arylrest, ein mit (b), einein $C_{1-6}$-Alkylrest, einem $C_{3-8}$-Cycloalkylrest und/oder einem Heterocyclus substituierter Arylrest, ein Heteroarylrest, ein mit (b), einiem $C_{1-6}$-Alkylrest, einem $C_{3-8}$-Cycloalkylrest und/oder einem Heterocyclus substituierter Heteroarylrest, sind,
oder $R^6$ und $R^7$ jeweils ein $C_{3-8}$-Cycloalkylrest (gegebenenfalls substituiert mit (b), einem $C_{1-6}$-Alkylrest und/oder einem Heterocyclus), ein Heterocyclus (gegebenenfalls substituiert mit (b), einem $C_{1-6}$-Alkylrest und/oder einem $C_{3-8}$-Cycloalkylrest), ein Arylrest (gegebenenfalls substituiert mit (b), einem $C_{1-6}$-Alkylrest, einem $C_{3-8}$-Cycloalkylrest und/oder einem Heterocyclus), oder ein Heterocyclus (gegebenenfalls substituiert mit (b), einem $C_{1-6}$-Alkylrest, einem $C_{3-8}$-Cycloalkylrest und/oder einem Heterocyclus), sind,
wobei (b) $-OR^5$, $-NR^8R^9$, $-COOR^8$, $-COR^8$, $-CONR^8R^9$, -CN, $-NO_2$, $-SO_2R^8$, $-SO_2NR^8R^9$ ist,
alternativ $-NR^6R^7$ einen Ring mit 3 bis 7 Atomen bilden kann, wobei der Ring gegebenenfalls ein oder mehrere zusätzliche Heteroatome einschließt und gegebenenfalls substituiert ist mit einem oder mehreren $C_{1-6}$-Alkylresten, $-OR^5$, $-COR^8$, $-COOR^8$, $-CONR^8R^9$ und $-NR^5R^9$,

$R^8$ ein Wasserstoffatom, ein $C_{1-6}$-Alkylrest (gegebenenfalls substituiert mit einem $C_{3-8-}$ Cycloalkylrest, einem Heterocyclus, einem Arylrest, einem Heteroarylrest, $-OR^9$, $-NR^9R^{10}$ und/oder $-N(COR^9)R^{10}$), ein Arylrest (gegebenenfalls substituiert mit (c), einem $C_{1-6}$-Alkylrest, einem $C_{3-8}$-Cycloalkylrest und/oder einem Heterocyclus), ein Heteroarylrest (gegebenenfalls substituiert mit (c), einem $C_{1-6}$-Alkylrest, einem $C_{3-8}$-Cycloalkylrest und/oder einem Heterocyclus), ein $C_{3-8}$-Cycloalkylrest (gegebenenfalls substituiert mit (c), einem $C_{1-6}$-Alkylrest und/oder einem Heterocyclus), ein Heterocyclus (gegebenenfalls substituiert mit (c), einem $C_{1-6}$-Alkylrest und/oder einem $C_{3-8}$-Cycloalkylrest)
wobei (c) $-OR^9$, $-COOR^9$, $-COR^9$, $-CONR^{10}R^9$, $-NR^{10}R^9$, -CN, $-NO_2$, $-SO_2R^9$, $-SO_2NR^{10}R^9$ ist,

$R^9$ und $R^{10}$ jeweils unabhängig ein Wasserstoffatom oder ein $C_{1-6}$-Alkylrest sind,

$R^{13}$ ein Wasserstoffatom, $-OR^4$, $-OCOR^4$, $-COR^4$, $-COOR^4$, $-CONR^6R^7$, $-NO_2$, $-NR^6R^7$, -CN, $-SO_2R^4$ oder $-SO_2NR^6R^7$ ist,

X gleich =N- oder -CH- ist, und

die gestrichelte Bindung, dargestellt durch z, optional ist,
wobei
"Arylrest" sich auf einen aromatischen Rest mit 5 bis 10 Atomen bezieht und aus 1 oder 2 Ringen besteht,
"Aryloxyrest" sich auf ein Arylradikal bezieht, welches mindestens ein Sauerstoffatom einschließt und welches durch das Sauerstoffatom an den Rest des Moleküls gebunden wird,
"Heteroarylrest" sich auf einen aromatischen Rest mit 5 bis 10 Atomen und 1 oder 2 Ringe bezieht und welcher ein oder mehrere Heteroatome enthält,
"Heteroaryloxyrest" sich auf ein Heteroarylradikal bezieht, welches mindestens ein Sauerstoffatom einschließt und welches durch das Sauerstoffatom an den Rest des Moleküls gebunden wird,
"Heteroatom" sich auf ein Atom bezieht, welches ausgewählt ist aus N, O und S,
"Heterocyclus" sich auf 3- bis 10-gliedrigen, nicht aromatischen, teilweise oder vollständig gesättigten Kohlenwasserstoffrest bezieht, welcher ein oder mehrere Ringe und mindestens ein Heteroatom enthält.

**2.** Verbindung nach Anspruch 1, wobei $R^1$

ein $C_{1-6}$-Alkylrest ist, der mit einem Arylrest oder einem substituierten Arylrest substituiert ist und gegebenenfalls auch substituiert ist mit einem Halogenatom, $-OR^4$, $-COR^4$, $-COOR^4$, $-CONR^6R^7$, einem $C_{3-8}$-Cycloalkylrest, einem Heterocyclus, $-COOR^4$, $-CONR^6R^7$, einem $C_{3-8}$-Cycloalkylrest, welcher substituiert ist mit $-OR^4$, $-NR^6R^7$, $COOR^4$, $CONR^6R^7$, und/oder einem Heterocyclus, welcher substituiert ist mit $-OR^4$ und $-NR^6R^7$, $-COOR^4$, $-CONR^6R^7$, und wobei die Substituenten am substituierten Arylrest ausgewählt sind aus einem Halogenatom, $-OR^4$, $-COR^4$, $-COOR^4$, $-CONR^6R^7$, $-NO_2$, $-NR^6R^7$, $-SO_2R^4$, $-SO_2NR^6R^7$, -CN, einem Perfluoralkylrest, einem $C_{1-6}$-Alkylrest, einem $C_{3-8}$-Cycloalkylrest, einem Heterocyclus, einem $C_{1-6}$-Alkylrest, welcher substituiert ist mit $-OR^4$ und $-NR^6R^7$, $COOR^4$, $CONR^6R^7$, einem $C_{3-8}$-Cycloalkylrest, welcher substituiert ist mit $-OR^4$ und $-NR^6R^7$, $-COOR^4$, $-CONR^6R^7$, oder einem Heterocyclus, welcher substituiert ist mit $-OR^4$ und $-NR^6R^7$, $-COOR^4$, $-CONR^6R^7$,

ein $C_{1-6}$-Alkylrest ist, der mit einem Heteroarylrest oder einem substituierten Heteroarykest substituiert ist und gegebenenfalls auch substituiert ist mit einem Halogenatom, $-OR^4$, $-COR^4$, $-COOR^4$, $-CONR^6R^7$, einem $C_{3-8}$-Cycloalkylrest, einem Heterocyclus, einem $C_{3-8}$-Cycloalkylrest, welcher substituiert ist mit $-OR^4$, $-COOR^4$, $-CONR^6R^7$ und/oder $-NR^6R^7$, und/oder einem Heterocyclus, welcher substituiert ist mit $-OR^4$, $-COOR^4$, $-CONR^6R^7$ und/oder $-NR^6R^7$ und wobei die Substituenten am substituierten Heteroarylrest ausgewählt sind aus einem Halogenatom, $-OR^4$, $-COR^4$, $-COOR^4$, $-NR^6R^7$, $-SO_2R^4$, $-SO_2NR^6R^7$, $-NO_2$, -CN, $-CONR^6R^7$, einem $C_{1-6}$-Alkylrest, einem $C_{3-8}$-Cycloalkylrest, einem Heterocyclus, einem $C_{1-6}$-Alkylrest, welcher substituiert ist mit $-OR^4$, $-NR^6R^7$, $-COOR^4$, $-CONR^6R^7$, einem $C_{3-8}$-Cycloalkylrest, welcher substituiert ist mit $-OR^4$, $-NR^6R^7$, $-COOR^4$, $-CONR^6R^7$ und/oder einem Heterocyclus, welcher substituiert ist mit $-OR^4$, $-NR^6R^7$, $-COOR^4$ und/oder $-CONR^6R^7$,

ein Arylrest ist (gegebenenfalls substituiert mit einem Halogenatom, $-OR^4$, $-COR^4$, $-COOR^4$, $-CONR^6R^7$, einem $C_{1-6}$-Alkylrest, einem $C_{3-8}$-Cycloalkylrest, einem Heterocyclus, einem $C_{1-6}$-Alkylrest, welcher substituiert ist mit $-OR^4$, $-NR^6R^7$, $COOR^4$, $CONR^6R^7$, einem $C_{3-8}$-Cycloalkylrest, welcher substituiert ist mit $-OR^4$, $COOR^4$, $CONR^6R^7$ und/oder $-NR^6R^7$ und einem Heterocyclus, welcher substituiert ist mit $-OR^4$, $COOR^4$, $CONR^6R^7$ und/oder $-NR^6R^7$) oder

ein Heteroarylrest ist (gegebenenfalls substituiert mit einem Halogenatom, $-OR^4$, $-COR^4$, $-COOR^4$, $-CONR^6R^7$, einem $C_{1-6}$-Alkylrest, einem $C_{3-8}$-Cycloalkylrest, einem Heterocyclus, einem $C_{1-6}$-Alkylrest, welcher substituiert ist mit $-OR^4$, $-COOR^4$, $-CONR^6R^7$ und/oder $-NR^6R^7$, einem $C_{3-8}$-Cycloalkylrest, welcher substituiert ist mit $-OR^4$, $-COOR^4$, $-CONR^6R^7$ und/oder $-NR^6R^7$ und/oder einem Heterocyclus, welcher substituiert ist mit $-OR^4$, $-COOR^4$, $-CONR^6R^7$ und/oder $-NR^6R^7$).

**3.** Verbindung nach einem der Ansprüche 1 oder 2, wobei X gleich CH und $R^3$ gleich ein $C_{1-6}$-Alkoxyrest ist.

**4.** Verbindung nach einem der Ansprüche 1 bis 3, wobei $R^1$ ein mit einem Phenylrest substituierter $C_{1-6}$-Alkylrest ist, welcher substituiert ist mit einem bis drei Substituenten aus einer Hydroxy-, einer $C_{1-6}$-Alkoxy-, einer Di-($C_{1-6}$-Alkyl)amino, einer Di-($C_{1-6}$-Alkyl)-amino-$C_{1-6}$-alkoxygruppe, einem Mopholino-$C_{1-6}$-alkylrest, einer Carboxy-$C_{1-6}$-alkoxy- und einer $C_{1-6}$-Alkanoylaminogruppe, oder $R^1$ ein $C_{1-6}$-Alkylrest ist, welcher wie vorstehend und zusätzlich mit einer Hydroxygruppe substituiert ist.

**5.** Verbindung nach einem der Ansprüche 1 bis 4, wobei $R^1$ ein $C_{1-6}$-Alkylrest ist, welcher substituiert ist mit einem

Pyridyl-, einem Pyrrolyl-, einem N-$C_{1-6}$-Alkylpyrrolyl-, einem Thienylrest, einem mit einer $C_{1-6}$-Alkoxygruppe substituierten Thienylrest, einem Furyl-, einem 1,3-Benzodioxolyl- oder einem mit einer $C_{1-6}$-Alkoxygruppe substituierten 1,3-Benzodioxolylrest, oder $R^1$ ein $C_{1-6}$-Alkylrest ist, welcher wie vorstehend und zusätzlich mit einer Hydroxygruppe substituiert ist.

**6.** Verbindung nach einem der Ansprüche 1 bis 3, wobei $R^1$ ein Pyridylrest ist.

**7.** Verbindung nach Anspruch 1 oder 2, wobei die optionale Bindung z anwesend ist.

**8.** Verbindung nach Anspruch 4, nämlich:

*rac*-(Z)-1,3-Dihydro-4-[3-hydroxy-3-(4-methoxyphenyl)-1-propinyl]-3-[(3-methoxy-1H-pyrrol-2-yl)methylen]-2H-indol-2-on (H),
*rac*-(Z)-1,3-Dihydro-4-[3-hydroxy-3-(3-hydroxyphenyl)-1-propinyl]-3-[(3-methoxy-1H-pyrrol-2-yl)methylen]-2H-indol-2-on (I).
*rac*-(Z)-1,3-Dihydro-4-[3-hydroxy-3-(3-methoxyphenyl)-1-propinyl]-3-[(3-methoxy-1H-pyrrol-2-yl)methylen]-2H-indol-2-on (J),
*rac*-(Z)-4-[3-[2,3-Dihydro-3-[(3-methoxy-1H-pyrrol-2-yl)methylen]-2-oxo-1H-indol-4-yl]-1-hydroxy-2-propinyl] benzoesäuremethylester (K),
*rac*-(Z)-4-[3-[2,3-Dihydro-3-[(3-methoxy-1H-pyrrol-2-yl)methylen]-2-oxo-1H-indol-4-yl]-1-hydroxy-2-propinyl] benzoesäure (L),
*rac*-(Z)-1,3-Dihydro-4-[3-hydroxy-3-(2-methoxyphenyl)-1-propinyl]-3-[(3-methoxy-1H-pyrrol-2-yl)methylen]-2H-indol-2-on (M),
*rac*-(Z)-4-[3-[1,3-Benzodioxol-5-yl)-3-hydroxy-1-propinyl]-1,3-dihydro-3-[(3-methoxy-1H-pyrrol-2-yl)methylen]-2H-indol-2-on (N),
*rac*-(Z)-1,3-Dihydro-4-[3-hydroxy-3-(4-hydroxy-3-methoxyphenyl)-1-propinyl]-3-[(3-methoxy-1H-pyrrol-2-yl) methylen]-2H-indol-2-on (O),
*rac*-(Z)-1,3-Dihydro-4-[3-hydroxy-3-(4-hydroxyphenyl)-1-propinyl]-3-[(3-methoxy-1H-pyrrol-2-yl)methylen]-2H-indol-2-on (Q),
*rac*-(Z)-1,3-Dihydro-4-[3-(4-dimethylaminophenyl)-3-hydroxy-1-propinyl]-3-[(3-methoxy-1H-pyrrol-2-yl)methylen]-2H-indol-2-on (R),
*rac*-(Z)-1,3-Dihydro-4-[3-hydroxy-3-(4-phenoxyphenyl)-1-propinyl]-3-[(3-methoxy-1H-pyrrol-2-yl)methylen]-2H-indol-2-on (S).
*rac*-(Z)-1,3-Dihydro-4-[3-hydroxy-3-phenyl-1-butiny]-3-[(3-methoxy-1H-pyrrol-2-yl)methylen]-2H-indol-2-on (T),
*rac*-(Z)-1,3-Dihydro-4-[3-[4-(3-dimethylaminopropoxy)-phenyl]-3-hydroxy-1-propinyl)-3-[(3-methoxy-1H-pyrrol-2-yl)methylen]-2H-indol-2-on (V)
*rac*-(Z)-1,3-Dihydro-4-[3-(2,3-dimethoxyphenyl)-3-hydroxy-1-propinyl]-3-[(3-methaxy-1H-pyrrol-2-yl)methylen]-2H-indol-2-on (EE),
*rac*-(Z)-1,3-Dihydro-4-[3-(3,4-dimethoxyphenyl)-3-hydroxy-1-propinyl]-3-[(3-methoxy-1H-pyrrol-2-yl)methylen]-2H-indol-2-on (FF),
*rac*-(Z)-1,3-Dihydro-4-[3-hydroxy-3-(3-hydroxy-4-methoxyphenyl)-1-propinyl]-3-[(3-methoxy-1H-pyrrol-2-yl) methylen]-2H-indol-2-on (HH),
*rac*-(Z)-1,3-Dihydro-4-[3-hydroxy-3-[3-methoxy-4-[2-(4-morpholinyl)-ethoxy]-phenyl]-1-propinyl]-3-[(3-methoxy-1H-pyrrol-2-yl)methylen]-2H-indol-2-on (MM),
*rac*-(Z)-1,3-Dihydro-4-[3-hydroxy-3-[3-methoxy-4-[2-(4-morpholinyl)-ethoxy]-phenyl]-1-propinyl]-3-[(3-methoxy-1H-pyrrol-2-yl)methylen]-2H-indol-2-onhydrochloridsalz (NN),
*rac*-(Z)-1,3-Dihydro-4-[3-hydroxy-3-(2,4,5-trimethoxyphenyl)-1-propinyl]-3-[(3-methoxy-1H-pyrrol-2-yl)methylen]-2H-indol-2-on (PP),
*rac*-(Z)-[4-[3-[2,3-Dihydro-3-[(3-methoxy-1H-pyrrol-2-yl)methylen]-2-oxo-1H-indol-4-yl]-1-hydroxy-2-propinyl]-2-methoxyphenoxy]essigsäuremethylester (QQ),
*rac*-(Z)-[4-[3-[2,3-Dihydro-3-[(3-methoxy-1H-pyrrol-2-yl)methylen]-2-oxo-1H-indol-4-yl]-1-hydroxy-2-propinyl]-2-methoxyphenoxy]essigsäure (RR),
*rac*-(Z)-4-[3-hydroxy-3-(4-methoxy-1,3-benzodioxol-6-yl)-1-propinyl]-1,3-dihydro-3-[(3-methoxy-1H-pyrrol-2-yl)methylen]-2H-indol-2-on (SS),
*rac*-(Z)-1,3-Dihydro-4-[3-hydroxy-3-[4-[2-(4-morpholinyl)-ethoxy]-phenyl]-1-propinyl]-3-[(3-methoxy-1H-pyrrol-2-yl)methylen]-2H-indol-2-on (TT),
*rac*-(Z)-4-[3-(4-Chlor-2-methylsulfanylmethoxy-phenyl)-3-hydroxy-1-propinyl]-1,3-dihydro-3-[(3-methoxy-1H-pyrrol-2-yl)methylen]-2H-indol-2-on (UU),
*rac*-(Z)-4-[3-(3-Chlorphenyl)-3-hydroxy-1-propinyl]-1,3-dihydro-3-[(3-methoxy-1Hpyrrol-2-yl)methylen]-2H-indol-2-on (WW),
*rac*-(Z)-[4-[3-[2,3-Dihydro-3-[(3-methoxy-1H-pyrrol-2-yl)methylen]-2-oxo-1H-indol-4-yl]-1-hydroxy-2-propinyl] phenoxy]essigsaure-1,1-dimethylethylester (XX),
*rac*-(Z)-[4-[3-[2,3-Dihydro-3-[(3-methoxy-1H-pyrrol-2-yl)methylen]-2-oxo-1H-indol-4-yl]-1-hydroxy-2-propinyl] phenoxy]essigsäure (YY),
*rac*-(Z)-1,3-Dihydro-4-[3-hydroxy-3-(3-nitrophenyl)-1-propinyl]-3-[(3-methoxy-1Hpyrrol-2-yl)methylen]-2H-indol-2-on (ZZ),
*rac*-(Z)-4-[3-(3-Aminophenyl)-3-hydroxy-1-propinyl]-1,3-dihydro-3-[(3-methoxy-1Hpyrrol-2-yl)methylen]-2H-indol-2-on (AAA)
*rac*-(Z)-4-[3-(4-Acetamidophenyl)-3-hydroxy-1-propinyl]-1,3-dihydro-3-[(3-methoxy-1H-pyrrol-2-yl)methylen]-2H-indol-2-on (BBB), oder
*rac*-(Z)-1,3-Dihydro-4-[3-hydroxy-3-phenyl-1-propinyl)-3-[(3-methoxy-1H-pyrrol-2-yl)methylen]-2H-indol-2-on (FFF).

**9.** Verbindung nach Anspruch 5, nämlich

*rac*-(Z)-1,3-Dihydro-4-[3-hydroxy-3-(3-pyridinyl)-1-propinyl]-3-[(3-methoxy-1Hpyrrol-2-yl)methylen]-2H-indol-2-on (X),
Synthese von *rac*-(Z)-1,3-Dihydro-4-[3-hydroxy-3-(1-methyl-pyrrol-2-yl)-1-propinyl]-3-[(3-methoxy-1H-pyrrol-2-yl)methylen]-2H-indol-2-on (AA),
*rac*-(Z)-1,3-Dihydro-4-[3-hydroxy-3-(thiophen-3-yl)-1-propinyl]-3-[(3-methoxy-1Hpyrrol-2-yl)methylen]-2H-indol-2-on (BB),
*rac*-(Z)-1,3-Dihydro-4-[3-hydroxy-3-(1H-pyrrol-2-yl)-1-propinyl]-3-[(3-methoxy-1Hpyrrol-2-yl)methylen]-2H-indol-2-on (DD),
*rac*-(Z)-1,3-Dihydro-4-[3-hydroxy-3-(2-pyridinyl)-1-propinyl]-3-[(3-methoxy-1Hpyrrol-2-yl)methylen]-2H-indol-2-on (JJ),
*rac*-(Z)-1,3-Dihydro-4-[3-hydroxy-3-(2-thiophenyl)-1-propinyl]-3-[(3-methoxy-1Hpyrrol-2-yl)methylen]-2H-indol-2-on (KK),
*rac*-(Z)-1,3-Dihydro-4-[3-hydroxy-3-(3-methoxy-2-thiophenyl)-1-propinyl]-3-[(3-methoxy-1H-pyrrol-2-yl)methylen]-2H-indol-2-on (OO), oder
*rac*-(Z)-1,3-Dihydro-4-[3-(2-furanyl)-3-hydroxy-1-propinyl]-3-[(3-methoxy-1Hpyrrol-2-yl)methylen]-2H-indol-2-on (VV).

**10.** Verbindung nach Anspruch 6, nämlich

(Z)-1,3-Dihydro-3-[(3-methoxy-1H-pyrrol-2-yl)methylen]-4-[(3-pyridinyl)ethinyl]-2H-indol-2-on (CCC),
(Z)-1,3-Dihydro-3-[(3-methoxy-1H-pyrrol-2-yl)methylen]-4-[(2-pyridinyl)ethinyl]-2H-indol-2-on (DDD),
(Z)-1,3-Dihydro-3-[(3-methoxy-1H-pyrrol-2-yl)methylen]-4-[(4-pyridinyl)ethinyl]-2H-indol-2-on (EEE),
(Z)-1,3-Dihydro-3-[(3-methoxy-1H-pyrrol-2-yl)methylen]-5-nitro-4-[(3-pyridinyl)ethinyl]-2H-indol-2-on (GGG),
(Z)-5-Amino-1,3-Dihydro-3-[(3-methoxy-1H-pyrrol-2-yl)methylen]-4-[(3-pyridinyl)ethinyl]-2H-indol-2-on (HHH), oder
(Z)-N-[2,3-Dihydro-3-[(3-methoxy-1H-pyrrol-2-yl)methylen]-2-oxo-4-[(3-pyridinyl)ethinyl]-1H-indol-5-yl]-2-thiophenacetamid (III).

**11.** Verbindung nach Anspruch 1, nämlich

4-[(E)-2-(2-Chlorphenyl)-ethenyl]-1,3-dihydro-(Z)-3-[(1H-pyrrol-2-yl)methylen]-2Hindol-2-on (KKK),
1,3-Dihydro-(2)-3-[(1H-pyrrol-2-yl)methylen]-[(E)-2-phenylethenyl)-2H-indol-2-on (LLL),
1,3-Dihydro-(Z)-3-[(3-methoxy-1H-pyrrol-2-yl)methylen]-[(E)-2-phenylethenyl)-2Hindol-2-on (MMM),
1,3-Dihydro-4-[(E)-2-(4-methoxyphenyl)-ethenyl]-(Z)-3-[(1H-pyrrol-2-yl)methylen]-2H-indol-2-on (NNN),
1,3-Dihydro-(Z)-3-[(3-methoxy-1H-pyrrol-2-yl)methylen]-4-[(E)-2-(4-methoxyphenyl)-ethenyl]-2H-indol-2-on (OOO),
4-[(E)-2-[2,3-Dihydro-(Z)-3-[(3-methoxy-1H-pyrrol-2-yl)methylen]-2-oxo-1H-indol-4-yl]ethenyl]benzoesäuremethylester (PPP), oder
1,3-Dihydro-4-[(E)-2-(3,4-dimethoxyphenyl)-ethenyl]-(Z)-3-[(3-methoxy-1H-pyrrol-2-yl)methylen]-2H-indol-2-on (QQQ).

**12.** Verbindung nach Anspruch 1, nämlich

(Z)-1,3-Dihydro-4-(phenylethinyl)-3-[(3-methoxy-1H-pyrrol-2-yl)methylen]-2H-indol-2-on (D),
(Z)-1,3-Dihydro-4-[(4-methoxyphenyl)ethinyl]-3-[(3-methoxy-1H-pyrrol-2-yl)methylen]-2H-indol-2-on (G), oder
(Z)-1,3-Dihydro-3-[(3-methoxy-1H-pyrrol-2-yl)methylen]-4-(3-phenoxy-1-propinyl)-2H-indol-2-on (Y).

**13.** Verbindung der Formel

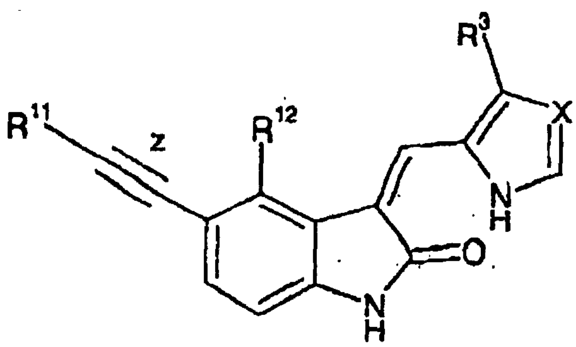

II

und die pharmazeutisch verträglichen Salze davon,
wobei

$R^{11}$ ein Wasserstoffatom, $-COR^4$, $-COOR^4$, $-CONR^6R^7$, ein $C_{1-6}$-Alkylrest (gegebenenfalls substituiert mit $-OR^5$, $-NR^6R^7$, einem Halogenatom, $-NO_2$, $-SO_2R^4$, $-SO_2NR^6R^7$, -CN, $-COR^4$, $-COOR^4$, $-CONR^6R^7$, einem $C_{3-8}$-Cycloalkylrest, einem Heterocyclus, einem Arylrest und/oder einem Heteroarylrest), ein $C_{3-8}$-Cycloalkylrest (gegebenenfalls substituiert mit-$OR^5$, $-NR^6R^7$, einem Halogenatom, $-NO_2$, $-SO_2R^4$, $-SO_2NR^6R^7$, -CN, $-COR^4$, $-COOR^4$, $-CONR^6R^7$, einem $C_{1-6}$-Alkylrest, einem Heterocyclus, einem Arylresc und/oder einem Heteroarylrest), ein Heterocyclus (gegebenenfalls substituiert mit $-OR^5$, $-NR^6R^7$, einem Halogenatom, $-NO_2$, $-SO_2R^4$, $-SO_2NR^6R^7$, -CN, $-COR^4$, $-COOR^4$, $-CONR^6R^7$, einem $C_{1-6}$-Alkylrest, einem $C_{3-8}$-Cycloalkylrest, einem Arylrest und/oder einem Heteroarylrest), ein Arylrest (gegebenenfalls substituiert mit $-OR^5$, $-NR^6R^7$, einem Halogenatom, $-NO_2$, $-SO_2R^4$, $-SO_2NR^6R^7$, -CN, $-COR^4$, $-COOR^4$, $-CONR^6R^7$, einem $C_{1-6}$-Alkylrest und/oder einem Perfluoralkylrest) oder ein Heteroarylrest (gegebenenfalls substituiert mit $-OR^5$, $-NR^6R^7$, einem Halogenatom, $-NO_2$, $-SO_2R^4$, $-SO_2NR^6R^7$, -CN, $-COR^4$, $-COOR^4$, $-CONR^6R^7$, einem $C_{1-6}$-Alkylrest und/oder einem Perfluoralkylrest) ist;

$R^{12}$ ein Wasserstoffatom, $-OR^4$, $-OCOR^4$, $-COR^4$, $-COOR^4$, $-CONR^6R^7$, $-NR^6R^7$, ein Halogenatom, $-NO_2$, -CN, $-SO_2R^4$, $-SO_2NR^6R^7$, ein Perfluoralkylrest, ein $C_{1-6}$-Alkylrest (gegebenenfalls substituiert mit $-OR^4$, $-NR^6R^7$, einem $C_{3-8}$-Cycloalkylrest, einem Heterocyclus, $-COR^4$, $-COOR^4$, $-CONR^6R^7$, -CN, $-NO_2$, $-SO_2R^4$, $-SO_2NR^6R^7$ und/oder einem Halogenatom), ein $C_{3-8}$-Cycloalkylrest (gegebenenfalls substituiert mit $-OR^4$, $-NR^6R^7$, einem $C_{1-6}$-Alkylrest, einem Heterocyclus, $-COR^4$, $-COOR^4$, $-CONR^6R^7$, -CN, $-NO_2$, $-SO_2R^4$, $-SO_2NR^6R^7$ und/oder einem Halogenatom) oder ein Heterocyclus (gegebenenfalls substituiert mit $-OR^4$, $-NR^6R^7$, einem $C_{1-6}$-Alkylrest, einem $C_{3-8}$-Cycloalkylrest, $-COR^4$, $-COOR^4$, $-CONR^6R^7$, -CN, $-NO_2$, $-SO_2R^4$, $-SO_2NR^6R^7$ und/oder einem Halogenatom) ist;

$R^3$ bis $R^7$, X, z, der Arylrest, der Heteroarylrest und der Heterocyclus wie vorstehend für die Formel I in Anspruch 1 definiert sind.

**14.** Verbindung nach Anspruch 1 oder 13, wobei $R^4$ ein Wasserstoffatom, ein $C_{1-6}$-Alkylrest (gegebenenfalls substituiert mit (a), einem $C_{3-8}$-Cycloalkylrest und/oder einem Heterocyclus), ein $C_{3-8}$-Cycloalkylrest (gegebenenfalls substituiert mit (a), einem $C_{1-6}$-Alkylrest und/oder einem Heterocyclus), oder ein Heterocyclus (gegebenenfalls substituiert mit (a), einem $C_{1-6}$-Alkylrest und/oder einem $C_{3-8}$-Cycloalkyl) ist, wobei (a) $-OR^5$, $-COOR^8$, $-COR^8$, $-CONR^8R^9$, $-NR^6R^7$, -CN, $-NO_2$, $-SO_2R^8$, und/oder $-SO_2NR^8R^9$ ist, und $R^5$ ein Wasserstoffatom, $-COR^8$, $-CONR^8R^9$ oder ein $C_{1-6}$-Alkylrest (gegebenenfalls substituiert mit $-OR^9$, $-NR^9R^{10}$, $-N(COR^9)R^{10}$, $-COR^9$, $-CONR^9R^{10}$ ist, und/oder $-COOR^9$) ist, und $R^1$, $R^2$, $R^3$, $R^8$, $R^9$, $R^{10}$, X und z wie in Anspruch 1 definiert sind.

**15.** Verbindung nach Anspruch 13, wobei

$R^3$ ein Wasserstoffatom, $-OR^4$, $-NR^6R^7$ und oder ein $C_{1-6}$-Alkylrest (gegebenenfalls substituiert mit $-OR^8$ und/oder $-NR^6R^7$) ist;
$R^4$ ein Wasserstoffatom, ein $C_{1-6}$-Alkylrest (gegebenenfalls substituiert mit einem oder mehreren $-OR^5$, $-COOR^8$, $-COR^8$, $-CONR^8R^9$), ein $C_{3-8}$-Cycloalkylrest (gegebenenfalls substituiert mit einem oder mehreren $-OR^5$, $-COOR^8$, $-COR^8$, und $-CONR^8R^9$), oder ein Heterocyclus (gegebenenfalls substituiert mit einem oder mehreren $-OR^5$, $-COOR^8$, $-COR^8$, und $-CONR^8R^9$) ist;
$R^5$ ein Wasserstoffatom, $-COR^8$, $-CONR^8R^9$ oder ein $C_{1-6}$-Alkylrest ist;
$R^6$ und $R^7$ jeweils unabhängig ein Wasserstoffatom, $-COR^8$, $-COOR^8$, $-CONR^8R^9$ oder ein $C_{1-6}$-Alkylrest (gegebenenfalls substituiert mit einem oder mehreren $-OR^9$, $-NR^8R^9$, $-COOR^8$ und $-CONR^8R^9$) sind; oder alternativ, $-NR^6R^7$ gegebenenfalls einen Ring mit 3 bis 7 Atomen bildet, wobei der Ring gegebenenfalls ein oder mehrere Heteroatome enthält und gegebenenfalls mit einem oder mehreren $C_{1-6}$-Alkylresten, $-OR^5$, $-COR^8$, $-COOR^8$, $-CONR^8R^9$ und $-NR^5R^9$ substituiert ist;
$R^8$ ein Wasserstoffatom oder ein $C_{1-6}$-Alkylrest ist (gegebenenfalls substituiert mit einem oder mehreren Arylresten, Heteroarylresten, $-OR^9$, $COOR^9$, $CONR^9R^{10}$ und $-NR^9R^{10}$) ist ;
$R^{11}$ ein Arylrest ist (gegebenenfalls substituiert mit $-OR^5$ und/oder $-NR^6R^7$);
$R^{12}$ ein Wasserstoffatom, $-COR^4$, $-COOR^4$, $-CONR^6R^7$, ein $C_{1-6}$-Alkylrest (gegebenenfalls substituiert mit einem oder mehreren $-OR^4$, $-NR^6R^7$, $C_{3-8}$-Cycloalkylresten, Heterocyclen, $-COR^4$, $-COOR^4$, $-CONR^6R^7$, -CN, $-NO_2$, $-SO_2R^4$, $-SO_2NR^6R^7$ und Halogenatomen), ein $C_{3-8}$-Cycloalkylrest (gegebenenfalls substituiert mit einem oder mehreren $-OR^4$, $-NR^6R^7$, $C_{1-6}$-Alkylresten, Heterocyclen, $-COR^4$, $-COOR^4$, $-CONR^6R^7$, -CN, $-NO_2$, $-SO_2R^4$, $-SO_2NR^6R^7$ und Halogenatomen), oder ein Heterocyclus (gegebenenfalls substituiert mit einem oder mehreren $-OR^4$, $-NR^6R^7$, $C_{1-6}$-Alkylresten, $C_{3-8}$-Cycloalkylresten, $-COR^4$, $-COOR^4$, $-CONR^6R^7$, -CN, $-NO_2$, $-SO_2R^4$, $-SO_2NR^6R^7$ und Halogenatomen) ist;

und die optionale Bindung z anwesend ist.

**16.** Verbindung nach Anspruch 13, nämlich

(Z)-1,3-Dihydro-5-ethinyl-3-[(1H-pyrrol-2-yl)methylen]-2H-indol-2-on (SSS),
(Z)-1,3-Dihydro-5-(4-hydroxyphenyl)ethinyl-3-[(1H-pyrrol-2-yl)methylen]-2H-indol-2-on (TTT),
(Z)-1,3-Dihydro-5-(3-nitrophenyl)ethinyl-3-[(1H-pyrrol-2-yl)methylen]-2H-indol-2-on (UUU),
(Z)-1,3-Dihydro-5-phenylethinyl-3-[(1H-pyrrol-2-yl)methylen]-2H-indol-2-on (VVV),
(Z)-1,3-Dihydro-5-(3-hydroxyphenyl)ethinyl-3-[(1H-pyrrol-2-yl)methylen]-2H-indol-2-on (WWW),
(Z)-1,3-Dihydro-5-(2-nitrophenyl)ethinyl-3-[(1H-pyrrol-2-yl)methylen]-2H-indol-2-on (XXX),
(Z)-1,3-Dihydro-5-(4-nitrophenyl)ethinyl-3-[(1H-pyrrol-2-yl)methylen]-2H-indol-2-on (ZZZ),
(Z)-5-(4-Aminophenyl)ethinyl-1,3-Dihydro-3-[(1H-pyrrol-2-yl)methylen]-2H-indol-2-on (AAAA),
(Z)-1,3-Dihydro-5-ethinyl-3-[(3-methoxy-1H-pyrrol-2-yl)methylen]-2H-indol-2-on (DDDD),
(Z)-1,3-Dihydro-5-(3-pyridinyl)ethinyl-3-[(1H-pyrrol-2-yl)methylen]-2H-indol-2-on (EEEE),
(Z)-1,3-Dihydro-5-(2-pyridinyl)ethinyl-3-[(1H-pyrrol-2-yl)methylen]-2H-indol-2-on (FFFF),
(Z)-1,3-Dihydro-5-(4-hydroxyphenyl)ethinyl-3-[(3-methoxy-1H-pyrrol-2-yl)methylen]-2H-indol-2-on (GGGG),
(Z)-1,3-Dihydro-5-(4-methoxyphenyl)ethinyl-3-[(1H-pyrrol-2-yl)methylen]-2H-indol-2-on (HHHH),
(Z)-1,3-Dihydro-3-[(1H-pyrrol-2-yl)methylen]-5-(2-thiophenyl)ethinyl-2H-indol-2-on (IIII), oder
(Z)-1,3-Dihydro-5-ethinyl-3-[(4-methyl-1H-imidazol-5-yl)methylen]-2H-indol-2-ontrifluoracetatsalz (LLLL).

**17.** Verbindungen

(Z)-1,3-Dihydro-3-[(1H-pyrrol-2-yl)methylen]-5-(trimethylsilyl)ethinyl-2H-indol-2-on,
(Z)-5-Brom-1,3-dibydro-3-[(3-methoxy-1H-pyrrol-2-yl)methylen]-2H-indol-2-on,
(Z)-1,3-Dihydro-3-[(3-methoxy-1H-pyrrol-2-yl)methylen]-5-(trimethylsilyl)ethinyl-2H-indol-2-on,
(Z)-5-Brom-1,3-dihydro-3-[(4-methyl-1H-imidazol-5-yl)methylen]-2H-indol-2-on,
(Z)-1,3-Dihydro-3-[(4-methyl-1H-imidazol-5-yl)methylen]-5-(trimethylsilyl)ethinyl-2H-indol-2-on.

**18.** Arzneimittel, welches als Wirkstoff eine Verbindung nach Anspruch 1 oder 13 und einen pharmazeutisch verträglichen Träger oder Exzipienten umfasst.

**19.** Verbindungen nach Anspruch 1 und 13 zur Verwendung als Medikamente.

**20.** Verwendung einer Verbindung nach Anspruch 1 oder 13 oder Prodrugs und pharmazeutisch aktive Stoffwechselprodukte einer solchen Verbindung zur Herstellung eines Medikaments zur Behandlung oder Bekämpfung von Entzündungserkrankungen, insbesondere rheumathoider Arthritis.

## Revendications

**1.** Composé répondant à la formule

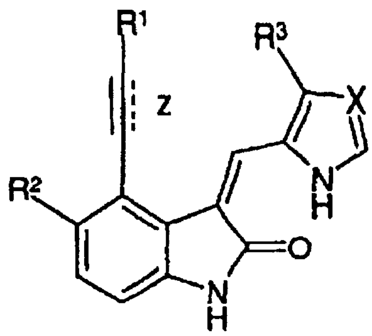

I

et sels pharmaceutiquement acceptables de ce composé, formule dans laquelle:

$R^1$ est un groupe allyle en $C_{1-6}$ qui est substitué par un groupe aryle, aryloxy, hétéroaryle, hétéroaryloxy, aryle substitué, aryloxy substitué, hétéroaryle substitué et/ou hétéroaryloxy substitué, et qui peut être éventuellement aussi substitué par un groupe $R^{13}$, perfluoroalkyle, cycloalkyle en $C_{3-8}$ (ou cycloalkyle en $C_{3-8}$ substitué par un groupe alkyle en $C_{1-6}$ et/ou $R^{13}$) ou un hétérocycle (ou un hétérocycle substitué par un groupe alkyle en $C_{1-6}$ et/ou $R^{13}$),

et dans laquelle les substituants sur les groupes aryle substitué, aryloxy substitué, hétéroaryle substitué et hétéroaryloxy substitué sont un ou plusieurs des groupes

$R^{13}$, alkyle en $C_{1-6}$ (éventuellement substitué par $R^{13}$), cycloalkyle en $C_{3-8}$ (éventuellement substitué par $R^{13}$), un hétérocycle (éventuellement substitué par $R^{13}$); aryle (éventuellement substitué par $R^{13}$ ou un groupe perfluoroalkyle, alkyle en $C_{1-6}$, alkyle en $C_{1-6}$ substitué par $R^{13}$, cycloalkyle en $C_{3-8}$, cycloalkyle en $C_{3-8}$ substitué par $R^{13}$, un hétérocycle (éventuellement substitué par $R^{13}$); ou hétéroaryle (éventuellement substitué par $R^{13}$ ou un groupe perfluoroalkyle, alkyle en $C_{1-6}$, alkyle en $C_{1-6}$ substitué par $R^{13}$, cycloalkyle en $C_{3-8}$, cycloalkyle en $C_{3-8}$ substitué par $R^{13}$, ou un hétérocycle ou un hétérocycle substitué par $R^{13}$); ou

aryle (éventuellement substitué par un groupe halogéno, $-OR^4$, $-COR^4$-, $COOR^4$, $-CONR^6R^7$, alkyle en $C_{1-6}$, cycloalkyle en $C_{3-8}$, un hétérocycle, alkyle en $C_{1-6}$ qui est substitué par $-OR^4$, $-NR^6R^7$, $COOR^4$, $CONR^6R^7$, cycloalkyle en $C_{3-8}$ qui est substitué par $-OR^4$, $COOR^4$, $CONR^6R^7$ et/ou $-NR^6R^7$, et un hétérocycle qui est substitué par $-OR^4$, $COOR^4$, $-CONR^6R^7$ et/ou $-NR^6R^7$), ou

hétéroaryle (éventuellement substitué par un groupe halogéno, $-OR^4$, $-COR^4$, $-COOR^4$, $-CONR^6R^7$, alkyle en $C_{1-6}$, cycloalkyle en $C_{3-8}$, un hétérocycle, alkyle en $C_{1-6}$ qui est substitué par $-OR^4$, $COOR^4$, $CONR^6R^7$ et/ou $-NR^6R^7$, cycloalkyle en $C_{3-8}$ qui est substitué par $-OR^4$, $COOR^4$, $CONR^6R^7$ et/ou $-NR^6R^7$, et/ou un hétérocycle qui est substitué par $-OR^4$, $COOR^4$, $CONR^6R^7$ et/ou $-NR^6R^7$);

$R^2$ est un atome d'hydrogène ou un groupe $-OR^4$, $-OCOR^4$, $-COR^4$, $-COOR^4$, $-CONR^6R^7$, $-NR^6R^7$, halogéno, $-NO_2$, -CN, $-SO_2R^4$, $-SO_2NR^6R^7$, perfluoroalkyle, alkyle en $C_{1-6}$ ou alkyle en $C_{1-6}$ substitué par $-OR^8$, ou $-NR^6R^7$;

$R^3$ est un atome d'hydrogène ou un groupe $-OR^4$, $-COR^4$, $-COOR^4$, $-CONR^6R^7$, halogéno, -CN, $-NR^6R^7$, perfluoroalkyle, alkyle en $C_{1-6}$ ou alkyle en $C_{1-6}$ substitué par $-OR^8$ ou $-NR^6R^7$;

$R^4$ est un atome d'hydrogène ou un groupe alkyle en $C_{1-6}$ (éventuellement substitué par un groupe (a), cycloalkyle en $C_{3-8}$ et/ou un hétérocycle), cycloalkyle en $C_{3-8}$ (éventuellement substitué par un groupe (a), alkyle en $C_{1-6}$ et/ou un hétérocycle), un hétérocycle (éventuellement substitué par un groupe (a), alkyle en $C_{1-6}$ et/ou cycloalkyle en $C_{3-8}$), aryle (éventuellement substitué par un groupe (a), cycloalkyle en $C_{3-8}$, un hétérocycle et/ou un halogène), hétéroaryle (éventuellement substitué par un groupe (a), cycloalkyle en $C_{3-8}$, un hétérocycle et/ou un halogène), où le groupe (a) est $-OR^5$, $-COOR^8$, $-COR^8$, $-CONR^8R^9$, $-NR^6R^7$, -CN, $-NO_2$, $-SO_2R^8$ et/ou $-SO_2NR^8R^9$;

$R^5$ est un atome d'hydrogène ou un groupe $COR^8$, $-CONR^8R^9$ ou alkyle en $C_{1-6}$ (éventuellement substitué par $-OR^9$, $-NR^9R^{10}$, $-N(COR^9)R^{10}$, $-COR^9$, $-CONR^9R^{10}$, $-SR^9$ et/ou $-COOR^9$;

$R^6$ et $R^7$ sont chacun des atomes d'hydrogène ou des groupes $-COR^8$, $-COOR^8$, $-CONR^8R^9$, $-SO_2R^8$, $-SO_2NR^8R^9$, alkyle en $C_{1-6}$, alkyle en $C_{1-6}$ substitué par un groupe (b), cycloalkyle en $C_{3-8}$ (éventuellement substitué par un groupe (b), alkyle en $C_{1-6}$ et/ou un hétérocycle), un hétérocycle, un hétérocycle substitué par un groupe (b), alkyle en $C_{1-6}$ et/ou cycloalkyle en $C_{3-8}$), aryle, aryle substitué par un groupe (b), alkyle en $C_{1-6}$, cycloalkyle en $C_{3-8}$ et/ou un hétérocycle), hétéroaryle, hétéroaryle substitué par un groupe (b), alkyle en $C_{1-6}$, cycloalkyle en $C_{3-8}$ et/ou un hétérocycle);

ou $R^6$ et $R^7$ sont chacun

un groupe cycloalkyle en $C_{3-8}$ (éventuellement substitué par un groupe (b), alkyle en $C_{1-6}$ et/ou un hétérocycle); un hétérocycle (éventuellement substitué par un groupe (b), alkyle en $C_{1-6}$ et/ou cycloalkyle en $C_{3-8}$); aryle (éventuellement substitué par un groupe (b), alkyle en $C_{1-6}$, cycloalkyle en $C_{3-8}$ et/ou un hétérocycle); ou hétéroaryle (éventuellement substitué par un groupe (b), alkyle en $C_{1-6}$, cycloalkyle en $C_{3-8}$ et/ou un hétérocycle);

où le groupe (b) est $OR^5$, $-NR^8R^9$, $-COOR^8$, $-COR^8$, $-CONR^8R^9$, -CN, $-NO_2$, $-SO_2R^8$, $-SO_2NR^8R^9$;

en variante, $-NR^6R^7$ peuvent former un noyau comportant 3 à 7 atomes, ledit noyau contenant éventuellement un ou plusieurs autres hétéroatomes et étant éventuellement substitué par un ou plusieurs groupes alkyle en $C_{1-6}$, $-OR^5$, $-COR^8$, $-COOR^8$, $-CONR^8R^9$ et $-NR^5R^9$;

$R^8$ est un atome d'hydrogène ou un groupe alkyle en $C_{1-6}$ (éventuellement substitué par un groupe cycloalkyle en $C_{3-8}$, un hétérocycle, aryle, hétéroaryle, $-OR^9$, $-NR^9R^{10}$ et/ou $-N(COR^9)R^{10}$), aryle. (éventuellement substitué par un groupe (c), alkyle en $C_{1-6}$, cycloalkyle en $C_{3-8}$ et/ou un hétérocycle), hétéroaryle (éventuellement substitué par un groupe (c), alkyle en $C_{1-6}$, cycloalkyle en $C_{3-8}$ et/ou un hétérocycle), cycloalkyle en $C_{3-8}$ (éventuellement substitué par un groupe (c), alkyle en $C_{1-6}$ et/ou un hétérocycle), un hétérocycle (éventuellement substitué par un groupe (c), alkyle en $C_{1-6}$ et/ou cycloalkyle en $C_{3-8}$);

où le groupe (c) est $-OR^9$, $-COOR^9$, $-COR^9$, $-CONR^{10}R^9$, $-NR^{10}R^9$, -CN, $-NO_2$, $-SO_2R^9$, $-SO_2NR^{10}R^9$;

$R^9$ et $R^{10}$ sont chacun indépendamment des atomes d'hydrogène ou des groupes alkyle en $C_{1-6}$;

$R^{13}$ est un groupe halogéno, $-OR^4$, $-OCOR^4$, $-COR^4$, $-COOR^4$, $-CONR^6R^7$, $-NO_2$, $-NR^6R^7$, -CN, $-SO_2R^4$ ou $-SO_2NR^6R^7$;

X est =N- ou -CH-; et

la liaison en pointillés représentée par z est facultative;

dans ces définitions,

"aryle" désigne un groupe aromatique comportant 5 à 10 atomes et constitué de 1 ou 2 noyaux;

"aryloxy" désigne un radical aryle qui contient au moins un atome d'oxygène et qui est fixé au reste de la molécule par l'intermédiaire de l'atome d'oxygène;

"hétéroaryle" désigne un groupe aromatique comportant 5 à 10 atomes, un ou 2 noyaux, et contenant un ou plusieurs hétéroatomes;

"hétéroaryloxy" désigne un radical hétéroaryle qui contient au moins un atome d'oxygène et qui est fixé au reste de la molécule par l'intermédiaire de l'atome d'oxygène;

"hétéroatome" désigne un atome choisi parmi N, O et S;

"hétérocycle" désigne un groupe hydrocarboné non aromatique, partiellement ou entièrement saturé, à 3 à 10 chaînons, qui contient un ou deux noyaux et au moins un hétéroatome.

**2.** Composé selon la revendication 1, dans lequel $R^1$ est un groupe

alkyle en $C_{1-6}$ qui est substitué par un groupe aryle ou aryle substitué, et éventuellement substitué aussi par un groupe halogéno, $-OR^4$, $-COR^4$, $-COOR^4$, $-CONR^6R^7$, cycloalkyle en $C_{3-8}$, un hétérocycle, $-COOR^4$, $CONR^6R^7$, cycloalkyle en $C_{3-8}$ qui est substitué par $OR^4$, $-NR^6R^7$, $COOR^4$, $CONR^6R^7$, et/ou un hétérocycle qui est substitué par $OR^4$ et $-NR^6R^7$, $COOR^4$, $CONR^6R^7$, et dans lequel les substituants sur le groupe aryle substitué sont choisis parmi les groupes halogéno, $OR^4$, $-COR^4$, $-COOR^4$, $-CONR^6R^7$, $-NO_2$, $NR^6R^7$, $-SO_2R_4$, $-SO_2NR^6R^7$, -CN, perfluoroalkyle, alkyle en $C_{1-6}$, cycloalkyle en $C_{3-8}$, un hétérocycle, alkyle en $C_{1-6}$ qui est substitué par $-OR^4$ et $-NR^6R^7$, $COOR^4$, $CONR^6R^7$, cycloalkyle en $C_{3-8}$ qui est substitué par $OR^4$ et $-NR^6R^7$, $COOR^4$, $CONR^6R^7$, ou un hétérocycle qui est substitué par $OR^4$ et $-NR^6R^7$, $COOR^4$, $CONR^6R^7$;

alkyle en $C_{1-6}$ qui est substitué par un groupe hétéroaryle ou hétéroaryle substitué, et éventuellement substitué aussi par un groupe halogéno, $-OR^4$, $-COR^4$, $-COOR^4$, $-CONR^6R^7$, cycloalkyle en $C_{3-8}$, un hétérocycle, cycloalkyle en $C_{3-8}$ qui est substitué par $-OR^4$, $COOR^4$, $CONR^6R^7$ et/ou $-NR^6R^7$, et/ou un hétérocycle qui est substitué par $-OR^4$, $COOR^4$, $CONR^6R^7$ et/ou $-NR^6R^7$, et dans lequel les substituants sur le groupe hétéroaryle substitué sont choisis parmi les groupes halogéno, $-OR^4$, $-COR^4$, $-COOR^4$, $NR^6R^7$, $-SO_2R^4$, $-SO_2NR^6R^7$,

$-NO_2$, -CN, $-CONR^6R^7$, alkyle en $C_{1-6}$, cycloalkyle en $C_{3-8}$, un hétérocycle, alkyle en $C_{1-6}$ qui est substitué par $-OR^4$, $-NR^6R^7$, $COOR^4$, $CONR^6R^7$, cycloalkyle en $C_{3-8}$ qui est substitué par $-OR^4$, $-NR^6R^7$, $COOR^4$, $CONR^6R^7$ et/ou un hétérocycle qui est substitué par $-OR^4$, $-NR^6R^7$, $COOR^4$ et/ou $CONR^6R^7$,
aryle (éventuellement substitué par un groupe halogéno, $-OR^4$, $-COR^4$, $-COOR^4$, $-CONR^6R^7$, alkyle en $C_{1-6}$, cycloalkyle en $C_{3-8}$, un hétérocycle, alkyle en $C_{1-6}$ qui est substitué par $-OR^4$, $-NR^6R^7$, $COOR^4$, $CONR^6R^7$, cycloalkyle en $C_{3-8}$ qui est substitué par $-OR^4$, $COOR^4$, $CONR^6R^7$ et/ou $-NR^6R^7$, et un hétérocycle qui est substitué par $-OR^4$, $COOR^4$, $CONR^6R^7$ et/ou $-NR^6R^7$), ou
hétéroaryle (éventuellement substitué par un groupe halogéno, $-OR^4$, $-COR^4$, $-COOR^4$, $-CONR^6R^7$, alkyle en $C_{1-6}$, cycloalkyle en $C_{3-8}$, un hétérocycle, alkyle en $C_{1-6}$ qui est substitué par $-OR^4$, $COOR^4$, $CONR^6R^7$ et/ou $-NR^6R^7$, cycloalkyle en $C_{3-8}$ qui est substitué par $-OR^4$, $COOR^4$, $CONR^6R^7$ et/ou $-NR^6R^7$, et/ou un hétérocycle qui est substitué par $-OR^4$, $COOR^4$, $CONR^6R^7$ et/ou $-NR^6R^7$).

**3.** Composé selon l'une quelconque des revendications 1 et 2, dans lequel X est CH et $R^3$ est un groupe alcoxy en $C_{1-6}$.

**4.** Composé selon l'une quelconque des revendications 1-3 dans lequel $R^1$ est un groupe alkyle en $C_{1-6}$ substitué par un groupe phényle qui est substitué par un à trois substituants choisis parmi les groupes hydroxy, alcoxy en $C_{1-6}$, di-alkyl(en $C_{1-6}$)amino, di-alkyl(en $C_{1-6}$)amino-alcoxy en $C_{1-6}$, morpholino-alkyle en $C_{1-6}$, carboxy-alcoxy en $C_{1-6}$ et alcanoylamino en $C_{1-6}$; ou $R^1$ est un groupe alkyle en $C_{1-6}$ substitué comme précédemment et en plus par un groupe hydroxy.

**5.** Composé selon l'une quelconque des revendications 1-4, dans lequel $R^1$ est un groupe alkyle en $C_{1-6}$ substitué par un groupe pyridyle, pyrrolyle, N-alkyl(en $C_{1-6}$)pyrrolyle, thiényle, thiényle substitué par un groupe alcoxy en $C_{1-6}$, furyle, 1,3-benzodioxolyle ou 1,3-benzodioxolyle substitué par un groupe alcoxy en $C_{1-6}$; ou $R^1$ est un groupe alkyle en $C_{1-6}$ substitué comme précédemment et en plus par un groupe hydroxy.

**6.** Composé selon l'une quelconque des revendications 1-3 dans lequel $R^1$ est un groupe pyridyle.

**7.** Composé selon la revendication 1 ou 2 dans lequel la liaison facultative z est présente.

**8.** Composé selon la revendication 4 qui est

la rac-(Z)-1,3-dihydro-4-[3-hydroxy-3-(4-méthoxyphényl)-1-propynyl)-3-[(3-méthoxy-1H-pyrrol-2-yl)méthylène]-2H-indol-2-one (H),
la rac-(Z)-1,3-dihydro-4-[3-hydroxy-3-(3-hydroxyphényl)-1-propynyl]-3-[(3-méthoxy-1H-pyrrol-2-yl)méthylène]-2H-indol-2-one (I),
la rac-(Z)-1,3-dihydro-4-[3-hydroxy-3-(3-méthoxyphényl)-1-propynyl]-3-[(3-méthoxy-1H-pyrrol-2-yl)méthylène]-2H-indol-2-one (J),
l'ester méthylique de l'acide rac-(Z)-4-[3-[2,3-dihydro-3-[(3-méthoxy-1H-pyrrol-2-yl)méthylène]-2-oxo-1H-indol-4-yl]-1-hydroxy-2-propynyl]benzoïque (K),
l'acide rac-(Z)-4-[3-[2,3-dihydro-3-[(3-méthoxy-1H-pyrrol-2-yl)-méthylène]-2-oxo-1H-indol-4-yl]-1-hydroxy-2-propynyl]benzoïque (L),
la rac-(Z)-1,3-dihydro-4-[3-hydroxy-3-(2-méthoxyphényl)-1-propynyl]-3-[(3-méthoxy-1H-pyrrol-2-yl)méthylène]-2H-indol-2-one (M),
la rac-(Z)-4-[3-(1,3-benzodioxol-5-yl)-3-hydroxy-1-propynyl]-1,3-dihydro-3-[(3-méthoxy-1H-pyrrol-2-yl)méthylène]-2H-indol-2-one (N),
la rac-(Z)-1,3-dihydro-4-[3-hydroxy-3-(4-hydroxy-3-méthoxyphényl)-1-propynyl]-3-[(3-méthoxy-1H-pyrrol-2-yl)méthylène]-2H-indol-2-one(O),
la rac-(Z)-1,3 -dihydro-4-[3-hydroxy-3-(4-hydroxyphényl)-1-propynyl]-3-[(3-méthoxy-1H-pyrrol-2-yl)méthylène]-2H-indol-2-one (Q),
la rac-(Z)-1,3-dihydro-4-[3-(4-diméthylaminophényl)-3-hydroxy-1-propynyl]-3-[(3-méthoxy-1H-pyrrol-2-yl)méthylène]-2H-indol-2-one (R),
la rac-(Z)-1,3-dihydro-4-[3-hydroxy-3-(4-phénoxyphényl)-1-propynyl]-3-[(3-méthoxy-1H-pyrrol-2-yl)méthylène]-2H-indol-2-one (S),
la rac-(Z)-1,3-dihydro-4-[3-hydroxy-3-phényl-1-butynyl]-3-[(3-méthoxy-1H-pyrrol-2-yl)méthylène]-2H-indol-2-one (T),
la rac-(Z)-1,3-dihydro-4-[3-[4-(3-diméthylaminopropoxy)phényl]-3-hydroxy-1-propynyl]-3-[(3-méthoxy-1H-pyrrol-2-yl)méthylène]-2H-indol-2-one (V),

la rac-(Z)-1,3-dihydro-4-[3-(2,3-diméthoxyphényl)-3-hydroxy-1-propynyl]-3-[(3-méthoxy-1H-pyrrol-2-yl)méthylène]-2H-indol-2-one (EE),
la rac-(Z)-1,3-Dihydro-4-[3-(3,4-diméthoxyphényl)-3-hydroxy-1-propynyl]-3-[(3-méthoxy-1H-pyrrol-2-yl)méthylène]-2H-indol-2-one (FF),
la rac-(Z)-1,3-dihydro-4-[3-hydroxy-3-(3-hydroxy-4-méthoxyphényl)-1-propynyl]-3-[(3-méthoxy-1H-pyrrol-2-yl)méthylène]-2H-indol-2-one (HH),
la rac-(Z)-1,3-dihydro-4-[3-hydroxy-3-[3-méthoxy-4-[2-(4-morpholinyl)éthoxy]phényl]-1-propynyl]-3-[(3-méthoxy-1H-pyrrol-2-yl)-méthylène]-2H-indol-2-one (MM),
le sel chlorhydrate de rac-(Z)-1,3-dihydro-4-[3-hydroxy-3-[3-méthoxy-4-[2-(4-morpholinyl)éthoxy]phényl]-1-propynyl]-3-[(3-méthoxy-1H-pyrrol-2-yl)méthylène]-2H-indol-2-one (NN),
la rac-(Z)-1,3-dihydro-4-[3-hydroxy-3-(2,4,5-triméthoxyphényl)-1-propynyl]-3-[(3-méthoxy-1H-pyrrol-2-yl)méthylène]-2H-indol-2-one (PP),
l'ester méthylique de l'acide rac-(Z)-[4-[3-[2,3-dihydro-3-[(3-méthoxy-1H-pyrrol-2-yl)méthylène]-2-oxo-1H-indol-4-yl]-1-hydroxy-2-propynyl]-2-méthoxyphénoxy]acétique (QQ),
l'acide rac-(Z)-[4-[3-[2,3-dihydro-3-[(3-méthoxy-1H-pyrrol-2-yl)-méthylène]-2-oxo-1H-indol-4-yl]-1-hydroxy-2-propynyl]-2-méthoxyphénoxy]acétique (RR),
la rac-(Z)-4-[3-hydroxy-3-(4-méthoxy-1,3-benzodioxol-6-yl)-1-propynyl]-1,3-dihydro-3-[(3-méthoxy- 1H-pyrrol-2-yl)méthylène]-2H-indol-2-one (SS),
la rac-(Z)-1,3-dihydro-4-[3-hydroxy-3-[4-[2-(4-morpholinyl)-éthoxy]phényl]-1-propynyl]-3-[(3-méthoxy-1H-pyrrol-2-yl)méthylène]-2H-indol-2-one (TT),
la rac-(Z)-4-[3-(4-chloro-2-méthylsulfanylméthoxyphényl)-3-hydroxy-1-propynyl]-1,3-dihydro-3-[(3-méthoxy-1H-pyrrol-2-yl)-méthylène]-2H-indol-2-one (UU),
la rac-(Z)-4- [3-(3-chlorophényl)-3-hydroxy-1-propynyl]-1,3-dihydro-3-[(3-méthoxy-1H-pyrrol-2-yl)méthylène]-2H-indol-2-one (WW),
l'ester 1,1-diméthyléthylique de l'acide rac-(Z)-[4-[3-[2,3-dihydro-3-[(3-méthoxy-1H-pyrrol-2-yl)méthylène]-2-oxo-1H-indol-4-yl]-1-hydroxy-2-propynyl]phénoxy]acétique (XX),
l'acide rac-(Z)-[4-[3-[2,3-dihydro-3-[(3-méthoxy-1H-pyrrol-2-yl)-méthylène]-2-oxo-1H-indol-4-yl]-1-hydroxy-2-propynyl]phénoxy]acétique (YY),
la rac-(Z)-1,3-dihydro-4-[3-hydroxy-3-(3-nitrophényl)-1-propynyl]-3-[(3-méthoxy-1H-pyrrol-2-yl)méthylène]-2H-indol-2-one (ZZ),
la rac-(Z)-4-[3-(3-aminophényl)-3-hydroxy-1-propynyl]-1,3-dihydro-3-[(3-méthoxy-1H-pyrrol-2-yl)méthylène]-2H-indol-2-one (AAA),
la rac-(Z)-4-[3-(4-acétamidophényl)-3-hydroxy-1-propynyl]-1,3-dihydro-3-[(3-méthoxy-1H-pyrrol-2-yl)méthylène]-2H-indol-2-one (BBB) ou
la rac-(Z)-1,3-dihydro-4-(3-hydroxy-3-phényl-1-propynyl)-3-[(3-méthoxy-1H-pyrrol-2-yl)méthylène]-2H-indol-2-one (FFF).

**9.** Composé selon la revendication 5 qui est

la rac-(Z)-1,3-dihydro-4-[3-hydroxy-3-(3-pyridinyl)-1-propynyl]-3-[(3-méthoxy-1H-pyrrol-2-yl)méthylène]-2H-indol-2-one (X),
la rac-(Z)-1,3-dihydro-4-[3-hydroxy-3-(1-méthylpyrrol-2-yl)-1-propynyl]-3-[(3-méthoxy-1H-pyrrol-2-yl)méthylène]-2H-indol-2-one (AA),
la rac-(Z)-1,3-dihydro-4-[3-hydroxy-3-(thiophén-3-yl)-1-propynyl]-3-[(3-méthoxy-1H-pyrrol-2-yl)méthylène]-2H-indol-2-one (BB),
la rac-(Z)-1,3-dihydro-4-[3-hydroxy-3-(1H-pyrrol-2-yl)-1-propynyl]-3-[(3-méthoxy-1H-pyrrol-2-yl)méthylène]-2H-indol-2-one (DD),
la rac-(Z)-1,3-dihydro-4-[3-hydroxy-3-(2-pyridinyl)-1-propynyl]-3-[(3-méthoxy-1H-pyrrol-2-yl)méthylène]-2H-indol-2-one (JJ),
la rac-(Z)-1,3-dihydro-4-[3-hydroxy-3-(2-thiophényl)-1-propynyl]-3-[(3-méthoxy-1H-pyrrol-2-yl)méthylène]-2H-indol-2-one (KK),
la rac-(Z)-1,3-dihydro-4-[3-hydroxy-3-(3-méthoxy-2-thiophényl)-1-propynyl]-3-[(3-méthoxy-1H-pyrrol-2-yl) méthylène]-2H-indol-2-one (OO), ou
la rac-(Z)-1,3-dihydro-4-[3-(2-furanyl)-3-hydroxy-1-propynyl]-3-[(3-méthoxy-1H-pyrrol-2-yl)méthylène]-2H-indol-2-one (VV).

**10.** Composé selon la revendication 6 qui est

la (Z)-1,3-Dihydro-3-[(3-méthoxy-1H-pyrrol-2-yl)méthylène]-4-[(3-pyridinyl)éthynyl]-2H-indol-2-one (CCC),
la (Z)-1,3-dihydro-3-[(3-méthoxy-1H-pyrrol-2-yl)méthylène]-4-[(2-pyridinyl)éthynyl]-2H-indol-2-one (DDD),
la (Z)-1,3-dihydro-3-[(3-méthoxy-1H-pyrrol-2-yl)méthylène]-4-[(4-pyridinyl)éthynyl]-2H-indol-2-one (EEE),
la (Z)-1,3-dihydro-3-[(3-méthoxy-1H-pyrrol-2-yl)méthylène]-5-nitro-4-[(3-pyridinyl)éthynyl]-2H-indol-2-one (GGG),
la (Z)-5-amino-1,3-dihydro-3-[(3-méthoxy-1H-pyrrol-2-yl)-méthylène]-4-[(3-pyridinyl)éthynyl]-2H-indol-2-one (HHH) ou
le (Z)-N-[2,3-dihydro-3-[(3-méthoxy-1H-pyrrol-2-yl)méthylène]-2-oxo-4-[(3-pyridinyl)éthynyl]-1H-indol-5-yl]-2-thiophèneacétamide (III).

**11.** Composé selon la revendication 1 qui est

la 4-[(E)-2-(2-chlorophényl)éthényl]-1,3-dihydro-(Z)-3-[(1H-pyrrol-2-yl)méthylène]-2H-indol-2-one (KKK),
la 1,3-dihydro-(Z)-3-[(1H-pyrrol-2-yl)méthylène]-[(E)-2-phényléthényl]-2H-indol-2-one (LLL),
la 1,3-dihydro-(Z)-3-[(3-méthoxy-1H-pyrrol-2-yl)méthylène]-[(E)-2-phényléthényl]-2H-indol-2-one (MMM),
la 1,3-dihydro-4-[(E)-2-(4-méthoxyphényl)éthényl]-(Z)-3-[(1H-pyrrol-2-yl)méthylène]-2H-indol-2-one (NNN),
la 1,3-dihydro-(Z)-3-[(3-méthoxy-1H-pyrrol-2-yl)méthylène]-4-[(E)-2-(4-méthoxyphényl)éthényl]-2H-indol-2-one (OOO),
l'ester méthylique de l'acide 4-[(E)-2-[2,3-dihydro-(Z)-3-[(3-méthoxy-1H-pyrrol-2-yl)méthylène]-2-oxo-1H-indol-4-yl]éthényl]-benzoïque (PPP), ou
la 1,3-dihydro-4-[(E)-2-(3,4-diméthoxyphényl)éthényl]-(Z)-3-[(3-méthoxy-1H-pyrrol-2-yl)méthylène]-2H-indol-2-one (QQQ).

**12.** Composé selon la revendication 1, qui est

la (Z)-1,3-dihydro-4-(phényléthynyl)-3-[(3-méthoxy-1H-pyrrol-2-yl)méthylène]-2H-indol-2-one (D),
la (Z)-1,3-dihydro-4-[(4-méthoxyphényl)éthynyl]-3-[(3 -méthoxy-1H-pyrrol-2-yl)méthylène]-2H-indol-2-one (G) ou
la (Z)-1,3-dihydro-3-[(3-méthoxy-1H-pyrrol-2-yl)méthylène]-4-(3-phénoxy-1-propynyl)-2H-indol-2-one (Y).

**13.** Composé répondant à la formule:

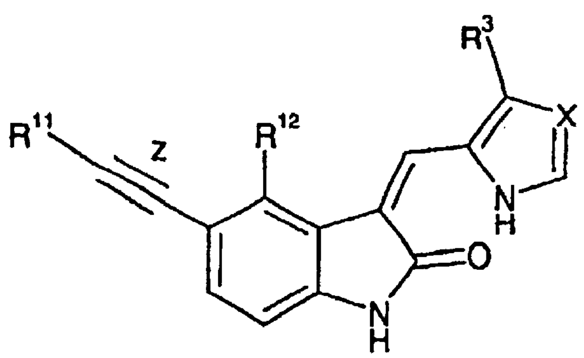

II

et sels pharmaceutiquement acceptable,
formule dans laquelle:

$R^{11}$ est un atome d'hydrogène ou un groupe $-COR^4$, $-COOR^4$, $-CONR^6R^7$,
alkyle en $C_{1-6}$ (éventuellement substitué par $-OR^5$, $-NR^6R^7$, halogéno, $-NO_2$, $-SO_2R^4$, $-SO_2NR^6R^7$, -CN, $-COR^4$, $-COOR^4$, $-CONR^6R^7$, cycloalkyle en $C_{3-8}$, un hétérocycle, aryle et/ou hétéroaryle),
cycloalkyle en $C_{3-8}$ (éventuellement substitué par $-OR^5$, $-NR^6R^7$, halogéno, $-NO_2$, $-SO_2R^4$,$-SO_2NR^6R^7$, -CN, $-COR^4$, $-COOR^4$, $-CONR^6R^7$, alkyle en $C_{1-6}$, un hétérocycle, aryle et/ou hétéroaryle),
un hétérocycle (éventuellement substitué par $-OR^5$, $-NR^6R^7$, halogéno, $-NO_2$, $-SO_2R^4$, $-SO_2NR^6R^7$, -CN, $-COR^4$, $-COOR^4$, $-CONR^6R^7$, alkyle en $C_{1-6}$, cycloalkyle en $C_{3-8}$, aryle et/ou hétéroaryle),
aryle (éventuellement substitué par un des groupes $-OR^5$, $-NR^6R^7$, halogéno, $-NO_2$, $-SO_2R^4$, $-SO_2NR^6R^7$, -CN, $-COR^4$, $-COOR^4$, $-CONR^6R^7$, alkyle en $C_{1-6}$ et/ou perfluoroalkyle) ou

hétéroaryle (éventuellement substitué par $-OR^5$, $-NR^6R^7$, halogéno, $-NO_2$, $-SO_2R^4$, $-SO_2NR^6R^7$, -CN, $-COR^4$, $-COOR^4$, $-CONR^6R^7$, alkyle en $C_{1-6}$ et/ou perfluoroalkyle);
$R^{12}$ est un atome d'hydrogène ou un groupe $-OR^4$, $-OCOR^4$, $-COR^4$, $-COOR^4$, $-CONR^6R^7$, $-NR^6R^7$, halogéno, $-NO_2$, -CN, $-SO_2R^4$, $-SO_2NR^6R^7$, perfluoroalkyle,
alkyle en $C_{1-6}$ (éventuellement substitué par $OR^4$, $-NR^6R^7$, cycloalkyle en $C_{3-8}$, un hétérocycle, $-COR^4$, $-COOR^4$, $-CONR^6R^7$, -CN, $-NO_2$, $-SO_2R^4$, $-SO_2NR^6R^7$ et/ou halogéno),
cycloalkyle en $C_{3-8}$ (éventuellement substitué par $-OR^4$, $-NR^6R^7$, alkyle en $C_{1-6}$, un hétérocycle, $-COR^4$, $-COOR^4$, $-CONR^6R^7$, -CN, $-NO_2$, $-SO_2R^4$, $-SO_2NR^6R^7$ et/ou halogéno) ou
un hétérocycle (éventuellement substitué par $-OR^4$, $-NR^6R^7$, alkyle en $C_{1-6}$, cycloalkyle en $C_{3-8}$, $-COR^4$, $-COOR^4$, $-CONR^6R^7$, -CN, $-NO_2$, $-SO_2R^4$, $-SO_2NR^6R^7$ et/ou halogéno), et
$R^3$ à $R^7$, X, z, aryle, hétéroaryle et hétérocycle sont tels que définis pour la formule I dans la revendication 1.

**14.** Composé selon la revendication 1 ou 13, dans lequel
$R^4$ est un atome d'hydrogène ou un groupe alkyle en $C_{1-6}$ (éventuellement substitué par un groupe (a), cycloalkyle en $C_{3-8}$ et/ou un hétérocycle), cycloalkyle en $C_{3-8}$ (éventuellement substitué par un groupe (a), alkyle en $C_{1-6}$ et/ou un hétérocycle) ou un hétérocycle (éventuellement substitué par un groupe (a), alkyle en $C_{1-6}$ et/ou cycloalkyle en $C_{3-8}$), où (a) est $-OR^5$, $-COOR^8$, $-COR^8$, $-CONR^8R^9$, $-NR^6R^7$, -CN, $-NO_2$, $-SO_2R^8$ et/ou $-SO_2NR^8R^9$; et $R^5$ est un atome d'hydrogène ou un groupe $-COR^8$, $-CONR^8R^9$ ou alkyle en $C_{1-6}$ (éventuellement substitué par $-OR^9$, $-NR^9R^{10}$, $-N(COR^9)R^{10}$, $-COR^9$, $-CONR^9R_{10}$ et/ou $-COOR^9$); et $R^1$, $R^2$, $R^3$, $R^8$, $R^9$, $R^{10}$, X et z sont tels que dans la revendication 1.

**15.** Composé selon la revendication 13 dans lequel

$R^3$ est un atome d'hydrogène ou un groupe $-OR^4$, $-NR^6R^7$ et/ou alkyle en $C_{1-6}$ (éventuellement substitué par $-OR^8$ et/ou $-NR^6R^7$);
$R_4$ est un atome d'hydrogène ou un groupe alkyle en $C_{1-6}$ (éventuellement substitué par un ou plusieurs groupes $-OR^5$, $-COOR^8$, $-COR^8$, $-CONR^8R^9$), cycloalkyle en $C_{3-8}$ (éventuellement substitué par un ou plusieurs $-OR^5$, $-COOR^8$, $-COR_8$ et $-CONR_8R_9$) ou un hétérocycle (éventuellement substitué par un ou plusieurs $-OR^5$, $-COOR^8$, $-COR^8$ et $-CONR^8R^9$);
$R^5$ est un atome d'hydrogène ou un groupe $-COR^8$, $-CONR^8R^9$ ou alkyle en $C_{1-6}$;
$R^6$ et $R^7$ sont chacun indépendamment des atomes d'hydrogène ou des groupes $-COR^8$, $-COOR^8$, $-CONR^8R^9$ ou alkyle en $C_{1-6}$ (éventuellement substitué par un ou plusieurs groupes $-OR^9$, $-NR^8R^9$, $COOR^8$ et $CONR^8R^9$), ou
en variante, $-NR^6R^7$ forme éventuellement un noyau comportant 3 à 7 atomes, ledit noyau contenant éventuellement un ou plusieurs autres hétéroatomes et étant éventuellement substitué par un ou plusieurs groupes alkyle en $C_{1-6}$, $-OR^5$, $-COR^8$, $-COOR^8$, $-CONR^8R^9$ et $-NR^5R^9$;
$R^8$ est un atome d'hydrogène ou un groupe ou alkyle en $C_{1-6}$ (éventuellement substitué par un ou plusieurs groupes aryle, hétéroaryle, $-OR^9$, $COOR^9$, $CONR^9R^{10}$ et $-NR^9R^{10}$);
$R^{11}$ est un groupe aryle (éventuellement substitué par $-OR^5$ et/ou $-NR^6R^7$);
$R^{12}$ est un atome d'hydrogène ou un groupe $-COR^4$, $-COOR^4$, $-CONR^6R^7$,
alkyle en $C_{1-6}$ (éventuellement substitué par un ou plusieurs groupes $-OR^4$, $NR^6R^7$, cycloalkyle en $C_{3-8}$, un hétérocycle, $-COR^4$, $-COOR^4$, $-CONR^6R^7$, -CN, $-NO_2$, $-SO_2R^4$, $-SO_2$, $NR^6R^7$ et halogéno),
cycloalkyle en $C_{3-8}$ (éventuellement substitué par un ou plusieurs groupes $-OR^4$, $-NR^6R^7$, alkyle en $C_{1-6}$, un hétérocycle, $-COR^4$, $-COOR^4$, $-CONR^6R^7$, -CN, $-NO_2$, $-SO_2R^4$, $-SO_2$ $NR^6R^7$ et halogéno) ou
un hétérocycle (éventuellement substitué par un ou plusieurs groupes $-OR^4$, $-NR^6R^7$, alkyle en $C_{1-6}$, cycloalkyle en $C_{3-8}$, $-COR^4$, $-COOR^4$, $-CONR^6R^7$, -CN, $-NO_2$, $-SO_2R^4$, $-SO_2$ $NR^6R^7$ et halogéno);

et la liaison facultative z est présente.

**16.** Composé selon la revendication 13 qui est

la (Z)-1,3-dihydro-5-éthynyl-3-[(1H-pyrrol-2-yl)méthylène]-2H-indol-2-one (SSS),
la (Z)-1,3-dihydro-5-(4-hydroxyphényl)éthynyl-3-[(1H-pyrrol-2-yl)-méthylène]-2H-indol-2-one (TTT),
la (Z)-1,3-dihydro-5-(3-nitrophényl)éthynyl-3-[(1H-pyrrol-2-yl)-méthylène]-2H-indol-2-one (UUU),
la (Z)-1,3-dihydro-5-phényléthynyl-3-[(1H-pyrrol-2-yl)méthylène]-2H-indol-2-one (VVV),
la (Z)-1,3-dihydro-5-(3-hydroxyphényl)éthynyl-3-[(1H-pyrrol-2-yl)-méthylène]-2H-indol-2-one (WWW),
la (Z)-1,3-dihydro-5-(2-nitrophényl)éthynyl-3-[(1H-pyrrol-2-yl)-méthylène]-2H-indol-2-one (XXX),
la (Z)-1,3-dihydro-5-(4-nitrophényl)éthynyl-3-[(1H-pyrrol-2-yl)-méthylène]-2H-indol-2-one (ZZZ),

la (Z)-5-(4-aminophényl)éthynyl-1,3-dihydro-3-[(1H-pyrrol-2-yl)-méthylène]-2H-indol-2-one (AAAA),
la (Z)-1,3-dihydro-5-éthynyl-3-[(3-méthoxy-1H-pyrrol-2-yl)-méthylène]-2H-indol-2-one (DDDD),
la (Z)-1,3-dihydro-5-(3-pyridinyl)éthynyl-3-[(1H-pyrrol-2-yl)-méthylène]-2H-indol-2-one (EEEE),
la (Z)-1,3-dihydro-5-(2-pyridinyl)éthynyl-3-1[(1H-pyrrol-2-yl)-méthylène]-2H-indol-2-one (FFFF),
la (Z)-1,3-dihydro-5-(4-hydroxyphényl)éthynyl-3-[(3-méhoxy-1H-pyrrol-2-yl)méthylène]-2H-indol-2-one (GGGG),
la (Z)-1,3-dihydro-5-(4-méthoxyphényl)éthynyl-3-[(1H-pyrrol-2-yl)-méthylène]-2H-indol-2-one (HHHH),
la (Z)-1,3-dihydro-3-[(1H-pyrrol-2-yl)méthylène]-5-(2-thiophényl)-éthynyl-2H-indol-2-one (IIII) ou
le sel trifluoroacétate de la (Z)-1,3-dihydro-5-éthynyl-3-[(4-méthyl-1H-imidazol-5-yl)méthylène]-2H-indol-2-one (LLLL).

**17.** Composés

(Z)-1,3-dihydro-3-[(1H-pyrrol-2-yl)méthylène]-5-(triméthylsilyl)-éthynyl-2H-indol-2-one,
(Z)-5-bromo-1,3-dihydro-3-[(3-méthoxy-1H-pyrrol-2-yl)méthylène]-2H-indol-2-one,
(Z)-1,3-Dihydro-3-[(3-méthoxy-1H-pyrrol-2-yl)méthylène]-5-(triméthylsilyl)éthynyl-2H-indol-2-one,
(Z)-5-Bromo-1,3-dihydro-3-[(4-méthyl-1H-imidazol-5-yl)-méthylène]-2H-indol-2-one,
(Z)-1,3-dihydro-3-[(4-méthyl-1H-imidazol-5-yl)méthylène]-5-(triméthylsilyl)éthynyl-2H-indol-2-one.

**18.** Composition pharmaceutique comprenant, comme principe actif, un composé selon la revendication 1 ou 13 et un véhicule ou excipient pharmaceutiquement acceptable.

**19.** Composés selon la revendication 1 et 13 à utiliser comme médicaments.

**20.** Utilisation d'un composé selon la revendication 1 ou 13 ou de précurseurs de médicaments et de métabolites pharmaceutiquement actifs de ce composé dans la préparation d'un médicament destiné au traitement ou à la lutte contre les maladies inflammatoires, en particulier la polyarthrite rhumatoïde.